# EUROPÄISCHE PATENTSCHRIFT

(11) **EP 1 725 543 B1**
(45) Veröffentlichungstag und Bekanntmachung des Hinweises auf die Patenterteilung: **28.11.2007**
(21) Anmeldenummer: 05715654.9
(22) Anmeldetag: 02.03.2005
(51) Int. Cl.: C07D 401/14, C07D 403/04, C07D 403/14, C07D 409/14, C07D 413/14, C07D 417/14, C07D 471/04, A61K 31/501, A61K 31/506, A61K 31/5377, A61K 31/553, A61P 3/00, A61P 25/00

(54) **4-BENZIMIDAZOL-2-YL-PYRIDAZIN-3-ON-DERIVATIVE, IHRE HERSTELLUNG UND VERWENDUNG IN ARZNEIMITTELN**
4-BENZIMIDAZOL-2-YL-PYRIDAZINE-3-ONE-DERIVATIVES, PRODUCTION AND USE THEREOF IN MEDICAMENTS
DERIVE DE 4-BENZIMIDAZOL-2-YL-PYRIDAZIN-3-ONE, SA PRODUCTION ET SON UTILISATION DANS DES MEDICAMENTS

(30) Priorität: 02.03.2004 DE 102004010194
(43) Veröffentlichungstag der Anmeldung: 29.11.2006
(73) Patentinhaber: Sanofi-Aventis Deutschland GmbH, 65929 Frankfurt am Main (DE)
(72) Erfinder: SCHOENAFINGER, Karl, 63755 Alzenau (DE); HOELDER, Swen, 78464 Konstanz (DE); WILL, David, William, 65926 Frankfurt am Main (DE); MATTER, Hans, 63505 Langenselbold (DE); MUELLER, Guenther, 65843 Sulzbach (DE); BOSSART, Martin, 60435 Frankfurt (DE)
(86) Internationale Anmeldenummer: PCT/EP2005/002179
(87) Internationale Veröffentlichungsnummer: WO 2005/085230

(56) Entgegenhaltungen:
- EP-A- 1 061 077
- WO-A-01/74771
- WO-A-03/035065
- WO-A-03/066629
- WO-A-20/04046117

## Beschreibung

Die Erfindung betrifft Verbindungen gemäß der allgemeinen Formel (I), wobei die Definitionen der Substituenten A, B, D, E, R¹ und R² im nachfolgenden Text aufgeführt sind, sowie deren physiologisch verträglichen Salze, Verfahren zur Herstellung dieser Verbindungen und deren Verwendung als Arzneimittel.

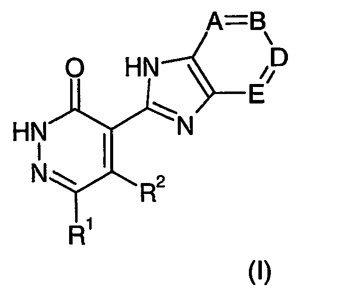

Diese Verbindungen sind Kinaseinhibitoren, insbesondere Inhibitoren der Kinase GSK-3β (Glykogensynthasekinase-3β).

Zur Behandlung von Krankheiten wie Diabetes oder Alzheimer sind bereits zahlreiche Verbindungen beziehungsweise Medikamente bekannt, die an bestimmten (unterschiedlichen) Stellen des biochemischen Prozesses, der mit dem jeweiligen Krankheitsbild verbunden ist, eingreifen können. Für die Behandlung von Stoffwechselerkrankungen sind bis jetzt nur wenige Verbindungen bekannt, die eine Inhibierung von GSK-3β bewirken können, zum Beispiel aus WO 03/066629. Aus WO 03/035065 sind weitere Verbindungen als kinaseinhibitoren bekannt.

So sind in WO 04/046117 Pyridazinonderivate offenbart, die sich zur Inhibierung von GSK-3β eignen. Die dort beschriebenen Pyridazinonderivate unterscheiden sich von den erfindungsgemäßen Verbindungen dahingehend, dass sie in Position 4 des Pyridazinon anstelle eines Benzimidazolrestes (beziehungsweise eines Derivates davon) einen Amidsubstituenten aufweisen, der sowohl über das Amid-Kohlenstoffatom als auch über das Amid-Stickstoffatom mit dem Pyridazinon-Grundkörper verknüpft sein kann.

Weiterhin sind in der Literatur zahlreiche Pyridazinon-Derivate beschrieben, die sich aber von den erfindungsgemäßen Verbindungen durch ein anderes Substitutionsmuster und (teilweise) andere Indikationen unterscheiden. So lassen sich aus der in WO 01/74786 offenbarten allgemeinen Formel unter anderem Pyridazinon-Derivate ableiten, die zwar in Position 4 einen Benzimidazol-Substituenten aufweisen können, andererseits aber in Position 5 im Gegensatz zu den erfindungsgemäßen Verbindungen zwingend eine Sulfonamid-Gruppe aufweisen. Die in WO 01/74786 beschriebenen Verbindungen besitzen eine inhibitorische Wirkung auf die Phosphodiesterase 7 und lassen sich zur Behandlung von Autoimmun-Krankenheiten verwenden.

In JP-A 09 216883 werden Pyridazinon-Derivate offenbart, die zur Behandlung von Herzinsuffizienz oder Bluthochdruck verwendet werden können. Die darin beschriebenen Pyridazinon-Derivate weisen in Position 6 zwingend einen Pyrazolo[1,5-a]pyridin-Substituenten auf, der wiederum in Position 2 mit Aryl, vorzugsweise Phenyl substituiert ist. Der Pyridazinon-Ring selbst ist in Position 2 zusätzlich mit Substituenten wie Wasserstoff, Niederalkyl oder einem Heterocyclus substituiert, während die Position 4 Substituenten wie Wasserstoff, Acyl, Cyano, Heterocyclyl, Amino oder eine geschützte Aminogruppe aufweist. Sofern der Substituent in Position 4 ein Heterocyclus ist, hat dieser vorzugsweise 3 bis 8 Ringglieder und ist gesättigt. Eine heterocyclische Gruppe gehört jedoch nicht zu den bevorzugten Substituenten in Position 4 des Pyridazinonringes der in diesem Dokument offenbarten Verbindungen.

In JP-A 09 216883 explizit offenbarte Verbindungen sind nicht Gegenstand der vorliegenden Erfindung.

In WO 03/059891 werden hingegen Pyridazinon-Derivate offenbart, die zur Behandlung von Krankheiten verwendbar sind, die durch nicht-regulierte p38 MAP Kinase- und/oder TNF-Aktivität verursacht oder verschärft werden. Die dort beschriebenen Verbindungen eignen sich zum Beispiel zur Behandlung von Entzündungen, von Diabetes, der Alzheimer Krankheit oder von Krebs. Sie unterscheiden sich von den erfindungsgemäßen Verbindungen dahingehend, dass der Stickstoff in Position 2 überwiegend mit Alkyl, Aryl oder Heteroaryl substituiert ist und dass ein Heteroarylsubstituent, wie Benzimidazol, für die Position 4 des Pyridazinon nicht definiert ist.

Bicyclische Heterocyclen, die eine aggregationshemmende Wirkung aufweisen, werden in EP-A 0 639 575 beschrieben. Aus der dort aufgeführten allgemeinen Formel (I) lässt sich für den den Substituenten A aufweisenden Bicyclus ein Benzimidazol-Derivat ableiten, das mindestens ein weiteres Ring-Stickstoffatom aufweisen muss. Ferner kann für den Substituenten B theoretisch ein Pyridazinon-Derivat abgeleitet werden, das wiederum zwingend mit einem mehrgliedrigen Substituenten versehen sein muss, der eine 1,4-Cyclohexylen oder 1,4-Cyclohex-3-enylen-Gruppe sowie eine Carbonylgruppe zwingend beinhaltet. Somit ist es ersichtlich, dass die erfindungsgemäßen Verbindungen nicht durch EP-A 0 639 575 offenbart sind. Verbindungen, die durch EP-A 0 639 575 explizit offenbart sind, sind nicht Gegenstand der vorliegenden Erfindung.

Es besteht somit ein großer Bedarf an Verbindungen, die eine inhibitorische Wirkung auf GSK-3β haben. Der vorliegenden Erfindung liegt deshalb die Aufgabe zugrunde, Verbindungen bereitzustellen, die diese Fähigkeiten haben.

Die Aufgabe wird durch 4-Benzimidazol-2-yl-pyridazin-3-on-Derivate gemäß der nachstehenden allgemeinen Formel (I) gelöst, 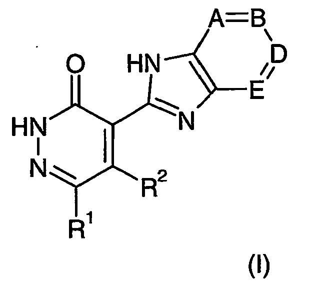 worin bedeuten:
A ist CR³ oder N;
B ist CR⁴ oder N;
D ist CR⁵ oder N;
E ist CR⁶ oder N;
wobei maximal drei der Substituenten A, B, D und E gleichzeitig N sein können;
- R¹: ist Halogen;
unsubstituiertes oder zumindest monosubstituiertes C₁-C₁₀-Alkyl,
wobei die Substituenten ausgewählt sind aus der Gruppe bestehend aus: Halogen, CN, NO₂, -OR⁷, -C(O)R⁷, -C(O)OR⁷, -O-C(O)R⁷, -NR⁷R⁸, -NHC(O)R⁷, -C(O)NR⁷R⁸, -NHC(S)R⁷, -C(S)NR⁷R⁸, -SR⁷, -S(O)R⁷,-SO,R⁷, -NHSO₂R⁷, -SO₂NR⁷R⁸, -O-SO₂R⁷, -SO₂-O-R⁷, Aryl, Heteroaryl, Heterocyclyl, Trifluormethyl und Trifluormethoxy,
und Heterocyclyl, Aryl und Heteroaryl können wiederum mit C₁-C₆-Alkyl, C₁-C₆-Alkoxy, Halogen, Trifluormethyl, Trifluormethoxy oder OH zumindest monosubstituiert sein;
oder unsubstituiertes oder zumindest monosubstituiertes Heterocyclyl, Aryl oder Heteroaryl,
wobei die Substituenten ausgewählt sind aus der Gruppe bestehend aus: Halogen, -CN, NO₂, -CH₂-R⁷, -CH₂-NH₂, -CH₂-NH(C₁-C₆-Alkyl), -CH₂- N(C₁-C₆-Alkyl)₂, -CH₂-OH, -CH₂-O-(C₁-4-Alkyl), -OR⁷, -C(O)R⁷,-C(O)OR⁷, -O-C(O)R⁷, -NR⁷R⁸, -NHC(O)R⁷, -C(O)NR⁷R⁸,-NHC(S)R⁷, -C(S)NR⁷R⁸, -SR⁷, -S(O)R⁷, -SO₂R⁷, -NHSO₂R⁷,-SO₂NR⁷R⁸, -O-SO₂R⁷, -SO₂-O-R⁷, Aryl, Heteroaryl, Trifluormethyl und Trifluormethoxy,
und Aryl und Heteroaryl können wiederum mit C₁-C₆-Alkyl, C₁-C₆-Alkoxy, Halogen, Trifluormethyl, Trifluormethoxy oder OH zumindest monosubstituiert sein;
- R²: ist Wasserstoff oder C₁-C₁₀-Alkyl;
- R³: ist ausgewählt aus der Gruppe bestehend aus:
Wasserstoff, Halogen, -CN, NO₂, -CH₂-R⁸, -CH₂-NH₂, -CH₂-NH(C₁-C₆-Alkyl), -CH₂-N(C₁-C₆-Alkyl)₂, -CH₂-OH, -CH₂-O-(C₁-C₆-Alkyl),-OR⁸, -C(O)R⁸, -C(O)OR⁸, -O-C(O)R⁸, -NR⁷R⁸, -NHC(O)R⁸, -C(O)NR⁷R⁸,-NHC(S)R⁸, -C(S)NR⁷R⁸, -SR⁸, -S(O)R⁸, -SO₂R⁸, -NHSO₂R⁸, -SO₂NR⁷R⁸,-O-SO₂R⁸, -SO₂-O-R⁸, Aryl, Heteroaryl, Heterocyclyl, Trifluormethyl und Trifluormethoxy,
und Heterocyclyl, Aryl und Heteroaryl können wiederum mit C₁-C₆-Alkyl, C₁-C₆-Alkoxy, Oxo, Halogen, Trifluormethyl, Trifluormethoxy oder OH zumindest monosubstituiert sein;
- R⁴: ist ausgewählt aus der Gruppe bestehend aus:
Wasserstoff, Halogen, -CN, NO₂, -CH₂-R⁸, -CH₂-NH₂, -CH₂-NH(C₁-C₆-Alkyl), -CH₂-N(C₁-C₆-Alkyl)₂, -CH₂-OH, -CH₂-O-(C₁-C₆-Alkyl),-OR⁸, -C(O)R⁸, -C(O)OR⁸, -O-C(O)R⁸, -NR⁷R⁸, -NHC(O)R⁸, -C(O)NR⁷R⁸,-NHC(S)R⁸, -C(S)NR⁷R⁸, -SR⁸, -S(O)R⁸, SO₂R⁸, NHSO₂R⁸, -SO₂NR⁷R⁸,-O-SO₂R⁸, -SO₂-O-R⁸, Aryl, Heteroaryl, Heterocyclyl, Trifluormethyl und Trifluormethoxy,
und Heterocyclyl, Aryl und Heteroaryl können wiederum mit C₁-C₆-Alkyl, C₁-C₆-Alkoxy, Oxo, Halogen, Trifluormethyl, Trifluormethoxy oder OH zumindest monosubstituiert sein;
- R⁵: ist ausgewählt aus der Gruppe bestehend aus:
Wasserstoff, Halogen, -CN, NO₂, -CH₂-R⁸, -CH₂-NH₂, -CH₂-NH(C₁-C₆-Alkyl), -CH₂-N(C₁-C₆-Alkyl)₂, -CH₂-OH, -CH₂-O-(C₁-C₆-Alkyl),-OR⁸, -C(O)R⁸, -C(O)OR⁸, -O-C(O)R⁸, -NR⁷R⁸, -NHC(O)R⁸, -C(O)NR⁷R⁸,-NHC(S)R⁸, -C(S)NR⁷R⁸, -SR⁸, -S(O)R⁸, -SO₂R⁸, -NHSO₂R⁸, -SO₂NR⁷R⁸,-O-SO₂R⁸, -SO₂-O-R⁸, Aryl, Heteroaryl, Heterocyclyl, Trifluormethyl und Trifluormethoxy,
und Heterocyclyl, Aryl und Heteroaryl können wiederum mit C₁-C₆-Alkyl, C₁-C₆-Alkoxy, Oxo, Halogen, Trifluormethyl, Trifluormethoxy oder OH zumindest monosubstituiert sein;
- R⁶: ist ausgewählt aus der Gruppe bestehend aus:
Wasserstoff, Halogen, -CN, NO₂, -CH₂-R⁸, -CH₂-NH₂, -CH₂-NH(C₁-C₆-Alkyl), -CH₂-N(C₁-C₆-Alkyl)₂, -CH₂-OH, -CH₂-O-(C₁-C₆-Alkyl),-OR⁸, -C(O)R⁸, -C(O)OR⁸, -O-C(O)R⁸, -NR⁷R⁸, -NHC(O)R⁸, -C(O)NR⁷R⁸,-NHC(S)R⁸, -C(S)NR⁷R⁸, -SR⁸, -S(O)R⁸, -SO₂R⁸, -NHSO₂R⁸, -SO₂NR⁷R⁸,-O-SO₂R⁸, -SO₂-O-R⁸, Aryl, Heteroaryl, Heterocyclyl, Trifluormethyl und Trifluormethoxy,
und Heterocyclyl, Aryl und Heteroaryl können wiederum mit C₁-C₆-Alkyl, C₁-C₆-Alkoxy, Oxo, Halogen, Trifluormethyl, Trifluormethoxy oder OH zumindest monosubstituiert sein;
- R⁷: ist H;
unsubstituiertes oder zumindest monosubstituiertes C₁-C₁₀-Alkyl, C₂-C₁₀-Alkenyl, C₂-C₁₀-Alkinyl, Heterocyclyl, Aryl oder Heteroaryl,
wobei die Substituenten ausgewählt sind aus der Gruppe bestehend aus: Heteroaryl, Heterocyclyl, Aryl, Oxo, Halogen, OH, C₁-C₁₀-Alkoxy, (C₁-C₁₀-Alkyl)thio-, -C(O)OH, -C(O)O-(C₁-C₆-alkyl), -C(O)NH₂,-C(O)NH(C₁-C₆-Alkyl), -C(O)N(C₁-C₁₀-Alkyl)₂, Trifluormethyl, Trifluormethoxy, -CN, -NH₂, -NH(C₁-C₁₀-Alkyl) und -N(C₁-C₁₀-Alkyl)₂,
und Heterocyclyl, Aryl und Heteroaryl können wiederum mit C₁-C₆-Alkyl, C₁-C₆-Alkoxy, Oxo, Trifluormethyl, Trifluormethoxy, Fluor, Chlor oder OH zumindest monosubstituiert sein;
- R⁸: ist H;
unsubstituiertes oder zumindest monosubstituiertes C₁-C₁₀-Alkyl, C₂-C₁₀-Alkenyl, C₂-C₁₀-Alkinyl, Heterocyclyl, Aryl oder Heteroaryl,
wobei die Substituenten ausgewählt sind aus der Gruppe bestehend aus: Heteroaryl, Heterocyclyl, Aryl, Halogen, OH, Oxo, C₁-C₁₀-Alkoxy, (C₁-C₁₀-Alkyl)thio-, -C(O)OH, -C(O)O-(C₁-C₆-alkyl), -C(O)NH₂,-C(O)NH(C₁-C₆-Alkyl), -C(O)N(C₁-C₁₀-Alkyl)₂, Trifluormethyl, Trifluormethoxy, -CN, -NH₂, -NH(C₁-C₁₀-Alkyl) und -N(C₁-C₁₀-Alkyl)₂,
und Heterocyclyl, Aryl und Heteroaryl können wiederum mit C₁-C₆-Alkyl, C₁-C₆-Alkoxy, Oxo, Trifluormethyl, Trifluormethoxy, Fluor, Chlor oder OH zumindest monosubstituiert sein;
Heteroaryl ist ein 5 bis 10-gliedriger, aromatischer, mono- oder bicyclischer Heterocyclus, der ein oder mehrere Heteroatome ausgewählt aus N, O und S enthält;
Aryl ist ein 5 bis 10-gliedriger, aromatischer, Mono- oder Bicyclus;
Heterocyclyl ist ein 5 bis 10-gliedriger, nicht-aromatischer, mono- oder bicyclischer Heterocyclus, der ein oder mehrere Heteroatome ausgewählt aus N, O und S enthält;
oder ein physiologisch verträgliches Salz davon;
unter der Voraussetzung, dass R¹ nicht unsubstituiertes oder zumindest mono-substituiertes Pyrazolo[1,5-a]pyridinyl ist.

Die vorstehenden Bedeutungen der Substituenten R¹ bis R⁸, A, B, D, E, Heteroaryl, Heterocyclyl und Aryl sind die Grundbedetungen (Definitionen) der jeweiligen Substituenten.

Sofern in den Verbindungen gemäß der Formel (I) Gruppen, Fragmente, Reste oder Substituenten wie beispielsweise Aryl, Heteroaryl, Alkyl, Alkoxy usw. mehr als einfach vorhanden sind, haben sie alle unabhängig voneinander die oben aufgeführten Bedeutungen und können somit in jedem (Einzel)-Fall entweder eine identische oder eine voneinander unabhängige Bedeutung haben. Die nachfolgenden Ausführungen gelten für (beispielsweise) Aryl sowie jeden beliebigen anderen Rest unabhängig von dessen Bezeichnung als Arylgruppe, -substituent, -fragment oder -rest. Zum Beispiel können in einer -N(C₁-C₆-Alkyl)₂-Gruppe (Di(C₁-C₆-alkyl)amino-Gruppe) die beiden Alkylsubstituenten entweder identisch oder unterschiedlich sein (beispielsweise zweimal Ethyl oder einmal Propyl und einmal Hexyl).

Sofern in den vorstehenden Definitionen für Verbindungen gemäß Formel (I) ein Substituent, beispielsweise Aryl, unsubstituiert oder zumindest monosubstituiert mit einer Gruppe von weiteren Substituenten, beispielsweise C₁-C₆-Alkyl, C₁-C₆-Alkoxy, Halogen usw., sein kann, so gilt für diejenigen Fälle, in denen eine Mehrfachsubstitution von Aryl vorliegt, dass die Auswahl aus der Reihe von weiteren Substituenten unabhängig voneinander erfolgt. Somit sind beispielsweise bei einer Zweifachsubstitution von Aryl sämtliche Kombinationen der weiteren Substituenten mit umfasst. Aryl kann somit beispielsweise zweifach-substituiert mit Ethyl sein, Aryl kann jeweils monosubstituiert mit Methyl und Ethoxy sein, Aryl kann jeweils monosubstituiert mit Ethyl und Fluor sein, Aryl kann zweifach-substituiert mit Methoxy sein, usw..

Alkylreste können entweder linear oder verzweigt, acyclisch oder cyclisch sein. Dies trifft auch zu, sofern sie Teil einer anderen Gruppe wie beispielsweise Alkoxygruppen (C₁-C₁₀-Alkyl-O-), Alkoxycarbonylgruppen oder Aminogruppen sind oder wenn sie substituiert sind.

Beispiele für Alkylgruppen sind: Methyl, Ethyl, Propyl, Butyl, Pentyl, Hexyl, Heptyl, Octyl, Nonyl oder Decyl. Dabei sind sowohl die n-Isomere dieser Reste als auch Isopropyl, Isobutyl, Isopentyl, sec-Butyl, tert-Butyl, Neopentyl, 3,3-Dimethylbutyl usw. mit umfasst. Sofern nicht anders beschrieben, beinhaltet der Begriff Alkyl darüber hinaus auch Alkylreste, die unsubstituiert oder gegebenenfalls mit einem oder mehreren weiteren Resten substituiert sind, beispielsweise 1, 2, 3 oder 4 identische oder unterschiedliche Reste, wie beispielsweise Aryl, Heteroaryl, Alkoxy oder Halogen. Dabei können die zusätzlichen Substituenten in jeder beliebigen Position des Alkylrestes auftreten. Weiterhin umfasst der Begriff Alkyl auch Cycloalkyl sowie Cycloalkyl-alkyl- (Alkyl, das wiederum mit Cycloalkyl substituiert ist), wobei Cycloalkyl mindestens 3 Kohlenstoffatome aufweist. Beispiele für Cycloalkylreste sind: Cyclopropyl, Cyclobutyl, Cyclopentyl, Cyclohexyl, Cycloheptyl, Cyclooctyl, Cyclononyl und Cyclodecyl. Gegebenenfalls kann es sich auch um polycyclische Ringsysteme handeln, wie Decalinyl, Norbornanyl, Bornanyl oder Adamantanyl. Die Cycloalkylreste können unsubstituiert oder gegebenenfalls mit einem oder mehreren weiteren Resten substituiert sein, wie vorstehend bei den Alkylresten beispielhaft aufgeführt.

Beispiele für Alkenyl- und Alkinylgruppen sind: Vinyl, 1-Propenyl, 2-Propenyl (Allyl), 2-Butenyl, 2-Methyl-2-propenyl, 3-Methyl-2-butenyl, Ethinyl, 2-Propinyl (Propargyl), 2-Butinyl oder 3-Butinyl. Der Begriff Alkenyl umfasst hier ausdrücklich auch Cycloalkenylreste sowie Cycloalkenyl-Alkylreste (Alkyl, das mit Cycloalkenyl substituiert ist), die mindestens drei Kohlenstoffatome enthalten. Beispiele für Cycloalkenyl sind: Cyclopentenyl, Cyclohexenyl, Cycloheptenyl und Cyclooctenyl.

Die Alkenylreste können ein bis drei konjugierte oder nicht konjugierte Doppelbindungen (also auch Alk-dienyl- sowie Alk-trienylreste), vorzugsweise eine Doppelbindung in einer geraden oder verzweigten Kette aufweisen, für Alkinylreste gilt das gleiche für die Dreifachbindungen. Die Alkenyl- und Alkinylreste können unsubstituiert oder gegebenenfalls mit einem oder mehreren weiteren Resten substituiert sein, wie vorstehend bei den Alkylresten beispielhaft aufgeführt.

Sofern nicht anders ausgeführt, können die vorgenannten Aryl-, Heteroaryl- und Heterocyclylreste sowohl unsubstituiert als auch einen oder mehrere, beispielsweise 1, 2, 3 oder 4 weitere, der vorgenannten Substituenten in jeder beliebigen Position aufweisen. Beispielsweise kann in monosubstituierten Phenylresten der Substituent in der Position 2, 3 oder 4, in disubstituierten Phenylresten können die Substituenten in der 2,3-Position, 2,4-Position, 2,5-Position, 2,6-Position, 3,4-Position oder 3,5-Position sein. In dreifach substituierten Phenylresten können die Substituenten in der 2,3,4-Position, 2,3,5-Position, 2,3,6-Position, 2,4,5-Position, 2,4,6-Position oder 3,4,5-Position sein. In vierfach substituierten Phenylresten können die Substituenten in der 2,3,4,5-Position, der 2,3,4,6-Position oder in der 2,3,5,6-Position sein.

Die vorgenannten beziehungsweise die nachfolgenden Definitionen, die sich auf einwertige Reste beziehen, gelten genauso für zweiwertige Reste wie Phenylen, Naphthylen oder Heteroarylen. Diese zweiwertigen Reste (Fragmente) können mit dem benachbarten Gruppen über jedes beliebige Ring-Kohlenstoffatom verknüpft sein. Im Falle von Phenylenresten kann dies in der 1,2-Position (Ortho-Phenylen), 1,3-Position (meta-Phenylen) oder 1,4-Position (Para-Phenylen) sein. Im Falle eines 5-gliedrigen Aromaten, der ein Heteroatom enthält, wie beispielsweise Thiophen oder Furan können die beiden freien Bindungen in der 2,3-Position, 2,4-Position, 2,5-Position oder der 3,4-Position sein. Ein zweiwertiger Rest, der von einem 6-gliedrigen Aromaten mit einem Heteroatom abgeleitet ist, wie beispielsweise Pyridin, kann ein 2,3-, 2,4-, 2,5-, 2,6-, 3,4- oder 3,5-Pyridindiylrest sein. Im Falle von unsymmetrischen zweiwertigen Resten beinhaltet die vorliegende Erfindung auch alle Positionsisomere, d.h. im Falle von beispielsweise einem 2,3-Pyridindiylrest ist die Verbindung, in der die eine benachbarte Gruppe sich in der Position 2 und die andere benachbarte Gruppe in der Position 3 sich befindet, genauso wie die Verbindung, in der die eine benachbarte Gruppe in der Position 3 und die andere benachbarte Gruppe sich in der Position 2 befindet, mit umfasst.

Soweit nicht anders aufgeführt, sind Heteroarylreste, Heteroarylenreste, Hetetrocyclykeste und Heterocyclylenreste sowie Ringe, die durch zwei an Stickstoff gebundene Gruppen gebildet werden, vorzugsweise abgeleitet von vollständig gesättigten, teilweise oder ganz ungesättigten Heterocyclen (d.h. Heterocycloalkane, Heterocycloalkene, Heteroaromaten), die 1, 2, 3 oder 4 Heteroatome enthalten, die sowohl unterschiedlich als auch gleich sein können. Vorzugsweise sind sie von Heterocyclen abgeleitet, die 1, 2 oder 3, besonders bevorzugt 1 oder 2, Heteroatome enthalten, die gleich oder unterschiedlich sein können. So lange nicht anders aufgeführt, sind die Heterocyclen mono- oder polycyclisch, zum Beispiel monocyclisch, bicyclisch oder tricyclisch. Vorzugsweise sind sie monocyclisch oder bicyclisch. Vorzugsweise sind es 5-gliedrige, 6-gliedrige oder 7-gliedrige Ringe, besonders bevorzugt 5-gliedriger oder 6-gliedrige Ringe. Im Fall von polycyclischen Heterocyclen mit 2 und mehr Heteroatomen können diese alle im gleichen Ring vorkommen oder auf mehrere Ringe verteilt sein.

Als Heteroaryl werden in der vorliegenden Erfindung Reste bezeichnet, die von monocyclischen oder bicyclischen, aromatischen Heterocyclen abgeleitet sind. Beispiele für Heteroaryl sind: Pyrrolyl, Furanyl (=Furyl), Thiophenyl (=Thienyl), Imidazolyl, Pyrazolyl, 1,2,3-Triazolyl, 1,2,4-Triazolyl, 1,3-Oxazolyl (=Oxazolyl), 1,2-Oxazolyl (=Isoxazolyl), Oxadiazolyl, 1,3-Thiazolyl (=Thiazolyl), 1,2-Thiazolyl (=Isothiazolyl), Tetrazolyl, Pyridinyl (=Pyridyl), Pyridazinyl, Pyrimidinyl, Pyrazinyl, 1,2,3-Triazinyl, 1,2,4-Triazinyl, 1,3,5-Triazinyl, 1,2,4,5-Tetrazinyl, Indazolyl, Indolyl, Benzothiophenyl, Benzofuranyl, Benzothiazolyl, Benzimidazolyl, Chinolinyl (= Chinolyl), Isochinolinyl (= Isochinolyl), Chinazolinyl, Cinnolinyl, Chinoxalinyl, Phthalazinyl, Thienothiophenyl, 1,8-Naphthyridinyl, andere Naphthyridinyle, Purinyl, Pteridinyl oder Thiazolo[3,2-b][1,2,4]-triazolyl. Sofern es sich nicht um monocyclische Systeme handelt, ist bei jedem der vorgenannten Heteroaryle für den zweiten Ring auch die gesättigte Form (Perhydroform) oder die teilweise ungesättigte Form (beispielsweise die Dihydroform oder Tetrahydroform) oder die maximal ungesättigte (nicht-aromatische) Form mitumfasst, sofern die jeweiligen Formen bekannt und stabil sind. Die Bezeichnung Heteroaryl umfasst somit in der vorliegenden Erfindung beispielsweise auch bicyclische Reste, in denen sowohl beide Ringe aromatisch sind als auch bicyclische Reste, in denen nur ein Ring aromatisch ist. Solche Beispiele für Heteroaryl sind: 3H-Indolinyl, 2(1H)-Chinolinonyl, 4-Oxo-1,4-dihydrochinolinyl, 2H-1-Oxoisochinolyl, 1,2-Dihydrochinolinyl, 3,4-Dihydrochinolinyl, 1,2-Dihydroisochinolinyl, 3,4-Dihydroisochinolinyl, Chromonyl, Chromanyl, 1,3-Benzodioxolyl, Oxindolyl, 1,2,3,4-Tetrahydroisochinolinyl, 1,2,3,4-Tetrahydrochinolinyl, 5,6-Dihydrochinolyl, 5,6-Dihydroisochinolyl, 5,6,7,8-Tetrahydrochinolinyl oder 5,6,7,8-Tetrahydroisochinolyl.

Als Heterocyclyl werden in der vorliegenden Erfindung Reste bezeichnet, die von monocyclischen oder bicyclischen, nicht-aromatischen Heterocyclen abgeleitet sind. Unter nicht-aromatischen Heterocyclen werden nachfolgend insbesondere Heterocycloalkane (vollständig gesättigte Heterocyclen) sowie Heterocycloalkene (teilweise ungesättigte Heterocyclen) verstanden. Im Fall der Heterocycloalkene werden auch Verbindungen mit zwei oder mehreren Doppelbindungen mit umfasst, die gegebenenfalls auch miteinander konjugiert sein können. Beispiele für Heterocyclyl sind: Pyrrolidinyl, Piperidinyl, Piperazinyl, Imidazolidinyl, Pyrazolidinyl, Isothiazolidinyl, Thiazolidinyl, Isoxazolidinyl, Oxazolidinyl, Tetrahydrofuranyl, Tetrahydrothiophenyl, 1,3-Dioxolanyl, 1,4-Dioxinyl, Pyranyl, Thiopyranyl, Tetrahydro-1,2-Oxazinyl, Tetrahydro-1,3-Oxazinyl, Morpholinyl, Thiomorpholinyl, 1,2-Thiazinyl, 1,3-Thiazinyl, 1,4-Thiazinyl, Azepinyl, 1,2-Diazepinyl, 1,3-Diazepinyl, 1,4-Diazepinyl, 1,3-Oxazepinyl, 1,3-Thiazepinyl, Azepanyl, 2-Oxoazepanyl, 1,4-Oxazepanyl, 1,2,3,4-Tetrahydropyridinyl, 1,2-Dihydropyridinyl, 1,4-Dihydropyridinyl, Dihydropyridinonyl wie 6-Oxo-1,6-dihydro-pyridinyl (= 1,6-Dihydropyridonyl) oder 2-Oxo-1,2-dihydro-pyridinyl (= 1,2-Dihydropyridonyl), 1,2,3,6-Tetrahydropyridinyl, 4(3H)-pyrimidonyl, 1,4,5,6-Tetrahydropyrimidinyl, 2-Pyrrolinyl, 3-Pyrrolinyl, 2-Imidazolinyl, 2-Pyrazolinyl, 3,4-Dihydro-2H-pyranyl, Dihydrofuranyl, 7-Oxabicyclo[2.2.1]heptenyl, Dihydrothiophenyl und Dihydrothiopyranyl. Der Grad der Sättigung von heterocyclischen Gruppen ist in der jeweiligen Definition angezeigt.

Substituenten, die von diesen Heterocyclen abgeleitet sind, können über jedes geeignete Kohlenstoffatom verknüpft sowie mit weiteren Substituenten versehen sein. Reste, die von stickstoffhaltigen Heterocyclen abgeleitet sind, können am entsprechenden Stickstoffatom ein Wasserstoffatom oder einen anderen Substituenten aufweisen. Beispiele umschließen Pyrrol, Imidazol, Pyrrolidin, Morpholin, Piperazinreste usw.. Diese stickstoffhaltigen heterocyclischen Reste können auch über das Ring-Stickstoffatom gebunden sein, insbesondere wenn der entsprechende heterocyclische Rest an ein Kohlenstoffatom gebunden ist. Beispielsweise kann ein Thienylrest als 2-Thienyl oder 3-Thienyl vorliegen, ein Piperidinylrest als 1-Piperidinyl (Piperidino), 2-Piperidinyl, 3-Piperidinyl oder 4-Piperidinyl. Geeignete stickstoffhaltige Heterocyclen können auch als N-Oxide oder als quarternäre Salze, die ein Gegenion aufweisen, das von einer physiologisch annehmbaren Säure abgeleitet ist, vorliegen. Beispielsweise können Pyridylreste als Pyridin-N-oxide vorliegen. Geeignete schwefelhaltige Heterocyclen können auch als S-Oxid oder S,S-Dioxid vorliegen.

Als Aryl werden in der vorliegenden Erfindung Reste bezeichnet, die von monocyclischen oder bicyclischen Aromaten abgeleitet sind, die keine Ringheteroatome enthalten. Sofern es sich nicht um monocyclische Systeme handelt, ist bei der Bezeichnung Aryl für den zweiten Ring auch die gesättigte Form (Perhydroform) oder die teilweise ungesättigte Form (beispielsweise die Dihydroform oder Tetrahydroform), sofern die jeweiligen Formen bekannt und stabil sind. Die Bezeichnung Aryl umfasst in der vorliegenden Erfindung auch beispielsweise bicyclische Reste, in denen sowohl beide Ringe aromatisch sind als auch bicyclische Reste, in denen nur ein Ring aromatisch ist. Beispiele für Aryl sind: Phenyl, Naphthyl, Indanyl, 1,2-Dihydronaphthenyl, 1,4-Dihydronaphthenyl, Indenyl oder 1,2,3,4-Tetrahydronaphthyl.

Arylalkyl (wie Aryl-(C₁-C₆-alkyl)-) bedeutet einen Alkylrest (wie C₁-C₆-Alkyl), der wiederum mit einem Arylrest substituiert ist. Heteroarylalkyl wie (Heteroaryl-(C₁-C₆-alkyl)- ) bedeutet einen Alkylrest (wie C₁-C₆-Alkyl), der wiederum mit einem Heteroarylrest substituiert ist. Heterocyclylalkyl (wie Heterocyclyl-(C₁-C₆-alkyl)- ) bedeutet einen Alkylrest (wie C₁-C₆-Alkyl), der wiederum mit einem Heterocyclylrest substituiert ist. Solche Arylalkyl, Heteroarylalkyl oder Heterocyclylalkylreste können wiederum ein Substituent eines anderen Substituenten oder Fragments (wie Heterocyclyl-(C₁-C₆-alkyl)-NH-) sein, was bedeutet, dass ein Substituent oder Fragment (wie -NH-) wiederum mit einem Heterocyclylalkylrest (wie Heterocyclyl-(C₁-C₆-alkyl)-) substituiert sein kann. Weitere mögliche Substitution für einen Alkylrest schließen auch Beispiele mit ein wie H₂N-(C₁-C₆-Alkyl)-, (C₁-C₆-Alkyl)HN-(C₁-C₆-alkyl)- oder (C₁-C₆-Alkyl)₂N-(C₁-C₆-alkyl)-, was einen Alkylrest (wie C₁-C₆-Alkyl) bedeutet, der wiederum mit -NH₂,-NH(C₁-C₆-Alkyl) beziehungsweise -N(C₁-C₆-Alkyl)₂ substituiert ist. Zusätzlich kann ein Rest wie (C₁-C₆-Alkyl)HN-(C₁-C₆-Alkyl)- selbst ein Substituent eines anderen Substituenten oder Fragments sein (wie (C₁-C₆-Alkyl)HN-(C₁-C₆-alkyl)-O-), was bedeutet, dass ein Substituent oder Fragment (wie -O-) wiederum mit einem substituierten Alkylrest (wie (C₁-C₆-Alkyl)HN-(C₁-C₆-alkyl)-) substituiert ist. Für die Definitionen und Substitutionsmöglichkeiten von Alkyl, Heteroaryl, Heterocyclyl und Aryl wird auf die vorstehenden Definitionen verwiesen.

Halogen ist Fluor, Chlor, Brom oder Iod, bevorzugt ist Fluor, Chlor, oder Brom, besonders bevorzugt ist Fluor oder Chlor.

Die vorliegende Erfindung schließt alle stereo-isomeren Formen von Verbindungen gemäß der Formel (I) mit ein. Asymmetrische Kohlenstoffatome in Verbindungen gemäß der Formel (I) können unabhängig voneinander S-Konfigurationen oder R-Konfigurationen aufweisen. Die Erfindung schließt alle möglichen Enantiomere und Diastereomere und Mischungen aus zwei oder mehr Stereo-Isomeren, zum Beispiel Mischungen aus Enantiomeren und/oder Diastereomeren, in allen Mengen und Verhältnissen mit ein. Somit können Verbindungen der vorliegenden Erfindung, die als Enantiomere existieren, in Enantiomeren-Reinform, sowohl als rechtsdrehende als auch linksdrehende Antipoden, in Form von Racematen und in Form von Mischungen der zwei Enantiomeren in allen Verhältnissen vorliegen. Im Fall von Cis/Trans-Isomeren schließt die Erfindung sowohl die Cis-Form, als auch die Trans-Form sowie Mischungen von diesen Formen in allen Verhältnissen mit ein. All diese Formen sind Gegenstand der vorliegenden Erfindung. Die Herstellung der einzelnen Stereo-Isomeren kann, falls gewünscht, durch Trennung eines Gemisches mit herkömmlichen Methoden, zum Beispiel durch Chromatographie oder Kristallisation, durch die Verwendung von stereochemisch einheitlichen Ausgangsmaterialien zur Synthese oder durch stereoselektive Synthese erfolgen. Alternativ kann auch einer Derivatisierung vor der Trennung der Stereo-Isomeren durchgeführt werden. Die Trennung eines Gemisches aus Stereo-Isomeren kann mit den Verbindungen der Formel (I) oder mit den entsprechenden Zwischenprodukten während der Synthese durchgeführt werden. Weiterhin umfasst die vorliegende Erfindung auch alle tautomeren Formen von Verbindungen gemäß Formel (I), insbesondere Keto-/Enoltautomerie, d.h. die entsprechenden Verbindungen können entweder in ihrer Keto-Form oder in ihrer Enolform oder in Mischungen davon in allen Verhältnissen vorliegen.

Sofern die Verbindungen gemäß Formel (I) eine oder mehrere saure oder basische Gruppen enthalten, umfasst die vorliegende Erfindung auch die entsprechend physiologisch oder toxikologisch verträgliche Salze.

Physiologisch verträgliche Salze sind aufgrund ihrer höheren Wasserlöslichkeit gegenüber den Ausgangs- bzw. Basisverbindungen besonders geeignet für medizinische Anwendungen. Diese Salze müssen ein physiologisch verträgliches Anion oder Kation aufweisen. Geeignete physiologisch verträgliche Säureadditionssalze der erfindungsgemäßen Verbindungen sind Salze anorganischer Säuren, wie Salzsäure, Bromwasserstoff-, Phosphor-, Metaphosphor-, Salpeter-, Sulfon- und Schwefelsäure sowie organischer Säuren, wie z.B. Essigsäure, Theophyllinessigsäure, Methylen-bis-b-oxynaphthoe-, Benzolsulfon-, Benzoe-, Zitronen-, Ethansulfon-, Salicyl-, Fumar-, Glucon-, Glykol-, Isethion-, Milch-, Lactobion-, Malein-, Äpfel-, Methansulfon-, Bernstein-, p-Toluolsulfon-, Wein- und Trifluoressigsäure. Geeignete pharmazeutisch verträgliche basische Salze sind Ammoniumsalze,.Alkalimetallsalze (wie Natrium- und Kaliumsalze) und Erdalkalisalze (wie Magnesium- und Calciumsalze).

Salze mit einem nicht pharmazeutisch verträglichen Anion gehören ebenfalls in den Rahmen der Erfindung als nützliche Zwischenprodukte für die Herstellung oder Reinigung pharmazeutisch verträglicher Salze und/oder für die Verwendung in nicht-therapeutischen, zum Beispiel in-vitro-Anwendungen.

Sofern die Verbindungen der Formel (I) gleichzeitig saure und basische Gruppen in demselben Molekül enthalten, schließt die vorliegende Erfindung - zusätzlich zu den vorher aufgeführten Salzformen - auch innere Salze oder Betaine (Zwitterionen) mit ein.

Die entsprechenden Salze der Verbindungen gemäß Formel (I) können durch herkömmliche Methoden, die dem Fachmann bekannt sind, erhalten werden, beispielsweise durch Umsetzung mit einer organischen oder anorganischen Säure oder Base in einem Lösungsmittel oder Dispergiermittel, oder durch Anionen- oder Kationenaustausch mit anderen Salzen.

Die erfindungsgemäßen Verbingunden können auch als Solvate von Verbindungen gemäß Formel (I) beispielsweise Hydrate oder Addukte mit Alkohol, aktive Metaboliten von Verbindungen gemäß Formel (I) sowie Derivate, die eine physiologisch annehmbare und abspaltbare Gruppe enthalten, beispielsweise Ester oder Amide, vorliegen.

Der hier verwendete Begriff "physiologisch funktionelles Derivat" bezeichnet jedes physiologisch verträgliche Derivat einer erfindungsgemäßen Verbindung der Formel I, z.B. einen Ester, der bei Verabreichung an einen Säuger, wie z.B. den Menschen, in der Lage ist, (direkt oder indirekt) eine Verbindung der Formel I oder einen aktiven Metaboliten hiervon zu bilden.

Zu den physiologisch funktionellen Derivaten zählen auch Prodrugs der erfindungsgemäßen Verbindungen. Solche Prodrugs können in vivo zu einer erfindungsgemäßen Verbindung metabolisiert werden. Diese Prodrugs können selbst wirksam sein oder nicht und sind ebenfalls Gegenstand der vorliegenden Erfindung.

Die erfindungsgemäßen Verbindungen können auch in verschiedenen polymorphen Formen vorliegen, z.B. als amorphe und kristalline polymorphe Formen. Alle polymorphen Formen der erfindungsgemäßen Verbindungen gehören in den Rahmen der Erfindung und sind ein weiterer Aspekt der Erfindung.

Bevorzugte Verbindungen der allgemeinen Formel (I) sind diejenigen Verbindungen, in denen einer, mehrere oder alle der vorstehend aufgeführten Substituenten R¹ bis R⁸, A, B, D, E, Heteroaryl, Heterocyclyl und Aryl unabhängig voneinander die nachstehend aufgeführten Bedeutungen (Definitionen) haben, wobei alle möglichen Kombinationen von bevorzugten, mehr bevorzugten, noch mehr bevorzugten, besonders bevorzugten und ganz besonders bevorzugten Bedeutungen (Definitionen), ebenso in Kombination mit den Substituenten in ihrer Grundbedeutung, Gegenstand der vorliegenden Erfindung sind.
A ist bevorzugt CR³;
B ist bevorzugt CR⁴;
D ist bevorzugt CR⁵;
E ist bevorzugt CR⁶;

Sofern nicht jeder der Substituenten A, B, D und E seine bevorzugte Bedeutung aufweist, sind bevorzugt nur zwei der Substituenten A, B, D und E gleich N; ist mehr bevorzugt nur einer der Substituenten A, B, D und E gleich N; noch mehr bevorzugt ist nur der Substituent B gleich N.
R¹ ist bevorzugt:
   Fluor; Chlor; Brom;
   unsubstituiertes oder zumindest monosubstituiertes C₁-C₆-Alkyl, wobei die Substituenten ausgewählt sind aus der Gruppe bestehend aus: Halogen,-OR⁷, -C(O)R⁷, -C(O)OR⁷, -NR⁷H, -NR⁷(C₁-C₆-Alkyl), -C(O)NR⁷H, -SR⁷, Aryl, Heteroaryl, Heterocyclyl, Trifluormethyl und Trifluormethoxy,
   und Heterocyclyl, Aryl und Heteroaryl können wiederum mit C₁-C₆-Alkyl, C₁-C₆-Alkoxy, Halogen, Trifluormethyl, Trifluormethoxy oder OH zumindest monosubstituiert sein;
   oder unsubstituiertes oder zumindest monosubstituiertes Heterocyclyl, Aryl oder Heteroaryl,
   wobei die Substituenten ausgewählt sind aus der Gruppe bestehend aus: Halogen, - CH₂-R⁷, -CH₂-NH₂, -CH₂-NH(C₁-C₆-Alkyl), -CH₂-N(C₁-C₆-Alkyl)₂, -CH₂-OH, - CH₂-O-(C₁-C₆-Alkyl), -OR⁷, -C(O)R⁷, -C(O)OR⁷, -NR⁷H, -NR⁷(C₁-C₆-Alkyl-), -C(O)NR⁷H, -SR⁷, Aryl, Heteroaryl, Trifluormethyl und Trifluormethoxy,
   und Aryl und Heteroaryl können wiederum mit C₁-C₆-Alkyl, C₁-C₆-Alkoxy, Halogen, Trifluormethyl, Trifluormethoxy oder OH zumindest monosubstituiert sein;
R¹ ist mehr bevorzugt:
   Chlor;
   unsubstituiertes oder zumindest monosubstituiertes C₁-C₆-Alkyl,
   wobei die Substituenten ausgewählt sind aus der Gruppe bestehend aus: Fluor, Chlor, -OH, C₁-C₆-Alkoxy, -NH₂, -NH(C₁-C₆-Alkyl), -N(C₁-C₆-Alkyl)₂, Heterocyclyl-(C₁-C₆-alkyl)-NH-, Aryl-(C₁-C₆-alkyl)-NH-, Heterocyclyl, Aryl und Heteroaryl,
   und die Heterocyclyl-, Aryl- und Heteroaryl-Fragmente dieser Substituenten können wiederum mit C₁-C₄-Alkyl, C₁-C₄-Alkoxy, Fluor, Chlor, Trifluormethyl, Trifluormethoxy oder OH zumindest monosubstituiert sein;
   oder unsubstituiertes oder zumindest monosubstituiertes Phenyl, Pyridinyl, Pyrimidinyl, Pyrazolyl, Thiophenyl, Oxazolyl, Isoxazolyl, Thiazolyl, Imidazolyl, Morpholinyl, Dihydropyridinonyl, Benzo[b]thiophenyl, 1,3-Benzodioxolyl oder Thiazolo[3,2-b] [1,2,4]-triazolyl,
   wobei die Substituenten ausgewählt sind aus der Gruppe bestehend aus: Halogen,-CH₂-R⁷, -CH₂-NH₂, -CH₂-NH(C₁-C₆-Alkyl), -CH₂-N(C₁-C₆-Alkyl)₂, -CH₂-OH,-CH₂-O-(C₁-C₆-Alkyl), -OR⁷, -C(O)R⁷, -C(O)OR⁷, -NR⁷H, -NR⁷(C₁-C₆-Alkyl-), -C(O)NR⁷H, -SR⁷, Aryl, Heteroaryl, Trifluormethyl und Trifluormethoxy,
   und Aryl und Heteroaryl können wiederum mit C₁-C₆-Alkyl, C₁-C₆-Alkoxy, Halogen, Trifluormethyl, Trifluormethoxy oder OH zumindest monosubstituiert sein;
R¹ ist noch mehr bevorzugt:
   unsubstituiertes oder zumindest monosubstituiertes Phenyl, Pyridinyl, Pyrimidinyl, Pyrazolyl, Thiophenyl, Oxazolyl, Isoxazolyl, Thiazolyl, Imidazolyl, Morpholinyl, Dihydropyridinonyl, Benzo[b]thiophenyl, Benzodioxolyl oder Thiazolo[3,2-b][1,2,4]-triazolyl,
   wobei die Substituenten ausgewählt sind aus der Gruppe bestehend aus: Halogen,-CH₂-R⁷, -CH₂-NH₂, -CH₂-NH(C₁-C₆-Alkyl), -CH₂-N(C₁-C₆-Alkyl)₂, -CH₂-OH,-CH₂-O-(C₁-C₆-Alkyl), -OR⁷, -C(O)R⁷, -C(O)OR⁷, -NR⁷H, -NR⁷(C₁-C₆-Alkyl), -C(O)NR⁷H, -SR⁷, Aryl, Heteroaryl, Trifluormethyl und Trifluormethoxy,
   und Aryl und Heteroaryl können wiederum mit C₁-C₆-Alkyl, C₁-C₆-Alkoxy, Halogen, Trifluormethyl, Trifluormethoxy oder OH zumindest monosubstituiert sein;
R¹ ist viel mehr bevorzugt:
   unsubstituiertes oder zumindest monosubstituiertes Phenyl, Pyridinyl, Pyrimidinyl, Pyrazolyl, Thiophenyl, Oxazolyl, Isoxazolyl, Thiazolyl, Imidazolyl, Morpholinyl, Dihydropyridinonyl, Benzo[b]thiophenyl, Benzodioxolyl oder Thiazolo[3,2-b][1,2,4]-triazolyl,
   wobei die Substituenten ausgewählt sind aus der Gruppe bestehend aus: Halogen, C₁-C₆-Alkyl, Phenyl-(C₁-C₆-alkyl)-, -OH, C₁-C₆-Alkoxy, (C₁-C₆-Alkyl)thio-, -O-Phenyl, -NH₂, -N(C₁-C₆-Alkyl)₂, -NH(C₁-C₆-Alkyl), HO(O)C-(C₁-C₆-Alkyl)-NH-, (C₁-C₆-Alkyl)-O(O)C-(C₁-C₆-alkyl)-NH-, H₂N(O)C-(C₁-C₆-Alkyl)-NH-, (C₁-C₆-Alkyl)HN(O)C-(C₁-C₆-alkyl)-NH-, H₂N-(C₁-C₆-Alkyl)-, (C₁-C₆-Alkyl)HN-(C₁-C₆-alkyl)-, (C₁-C₆-Alkyl)₂N-(C₁-C₆-alkyl)-, Heterocyclyl-(C₁-C₆-Alkyl)-O-, H₂N-(C₁-C₆-alkyl)-NH-, (C₁-C₆-Alkyl)HN-(C₁-C₆-alkyl)-NH-, (C₁-C₆-Alkyl)₂N-(C₁-C₆-alkyl)-NH-, Heterocyclyl-(C₁-C₆-alkyl)-NH-, Heteroaryl-(C₁-C₆-alkyl)-NH-, Phenyl-(C₁-C₆-alkyl)-NH-, (C₁-C₆-Alkyl)-O-(C₁-C₆-alkyl)-NH-, HO-(C₁-C₆-Alkyl)-NH-, Heterocyclyl-(C₁-C₆-alkyl)-, Trifluormethyl, Trifluormethoxy, Phenyl und Heteroaryl,
   und die Heterocyclyl-, Phenyl- und Heteroaryl-Fragmente dieser Substituenten können wiederum mit C₁-C₃-Alkyl, C₁-C₃-Alkoxy, Fluor, Chlor, Trifluormethyl, Trifluormethoxy oder OH zumindest monosubstituiert sein;
R¹ ist besonders bevorzugt:
   unsubstituiertes oder zumindest monosubstituiertes Phenyl, Thiophenyl, Pyridinyl oder Pyrimidinyl,
   wobei die Substituenten ausgewählt sind aus der Gruppe bestehend aus: C₁-C₄-Alkyl, -CH₂-NH(C₁-C₄-Alkyl), OH, C₁-C₄-Alkoxy, (C₁-C₄-Alkyl)thio-, Trifluormethyl, Trifluormethoxy und -NH(C₁-C₄-Alkyl),
   und -NH(C₁-C₄-Alkyl) kann wiederum mit Phenyl, Piperazinyl, Piperidinyl oder Morpholinyl monosubstituiert sein.
R¹ ist ganz besonders bevorzugt:
   Pyridin-4-yl, 4-Hydroxy-3-methoxy-5-(methylaminomethyl)-phenyl, 2-Ethylamino-pyrimidin-4-yl, 3,5-Dimethyl-4-hydroxyphenyl, 2-(1-Phenylethylamino)-pyrimidin-4-yl, 2-(2-Morpholin-4-ylethylamino)-pyrimidin-4-yl, 2-Methylamino-pyrimidin-4-yl, 6-Methyl-2-(2-morpholin-4-ylethylamino)-pyrimidin-4-yl, 3-Methoxy-4-hydroxy-phenyl, 2-Methylsulfanyl-pyrimidin-4-yl oder 4-Butylamino-pyrimidin-4-yl.
R² ist bevorzugt Wasserstoff oder C₁-C₆-Alkyl; R² ist besonders bevorzugt Wasserstoff.
R³ ist bevorzugt ausgewählt aus der Gruppe bestehend aus:
   Wasserstoff, Halogen, -CN, -CH₂-R⁸, -CH₂-NH₂, -CH₂-NH(C₁-C₆-Alkyl),-CH₂-N(C₁-C₆-Alk)₂, -CH₂-OH, -CH₂-O-(C₁-C₆-Alkyl), -OR⁸, -C(O)R⁸,-C(O)OR⁸, -NR⁸H, -NR⁸(C₁-C₆-Alkyl), -C(O)NR⁸H, -SR⁸, -SO₂NR⁸H, SO₂R⁸, Aryl, Heteroaryl, Heterocyclyl, Trifluormethyl und Trifluormethoxy,
   und Heterocyclyl, Aryl und Heteroaryl können wiederum mit C₁-C₆-Alkyl, C₁-C₆-Alkoxy, Oxo, Halogen, Trifluormethyl, Trifluormethoxy oder OH zumindest monosubstituiert sein;
R³ ist mehr bevorzugt ausgewählt aus der Gruppe bestehend aus:
   Wasserstoff, Fluor, Chlor, Brom, -CN, -CH₂-R⁸, -CH₂-NH₂, -CH₂-NH(C₁-C₆-Alkyl), -CH₂-N(C₁-C₆-Alkyl)₂, -CH₂-OH, -CH₂-O-(C₁-C₆-Alkyl), -OR⁸,-C(O)R⁸, -C(O)OR⁸, -NR⁸H, -NR⁸(C₁-C₆-Alkyl), -C(O)NR⁸H, -SR⁸,-SO₂NR⁸H, -SO₂-R⁸, Heterocyclyl, Trifluormethyl und Trifluormethoxy,
   und Heterocyclyl kann wiederum mit C₁-C₆-Alkyl, C₁-C₆-Alkoxy, Halogen, Trifluormethyl, Trifluormethoxy oder OH zumindest monosubstituiert sein;
R³ ist viel mehr bevorzugt ausgewählt aus der Gruppe bestehend aus:
   Wasserstoff, Fluor, Chlor, Brom, -CN, C₁-C₆-Alkyl, Phenyl-(C₁-C₆-alkyl)-, -OH, C₁-C₆-Alkoxy, -O-Phenyl, -C(O)OH, -C(O)O-(C₁-C₆-Alkyl), -NR⁸H, -NR⁸(C₁-C₆-Alkyl), -C(O)NR⁸H, Heterocyclyl, Trifluormethyl und Trifluormethoxy,
   und die Heterocyclyl- und Phenyl-Fragmente dieser Gruppe können wiederum mit C₁-C₆-Alkyl, C₁-C₆-Alkoxy, Halogen, Trifluormethyl, Trifluormethoxy oder OH zumindest monosubstituiert sein;
R³ ist noch viel mehr bevorzugt ausgewählt aus der Gruppe bestehend aus:
   Wasserstoff, Fluor, Chlor, Brom, -CN, C₁-C₆-Alkyl, -OH, C₁-C₆-Alkoxy, - C(O)OH, -C(O)O-(C₁-C₆-Alkyl), -NH₂, -N(C₁-C₆-Alkyl)₂, -NH(C₁-C₆-Alkyl), H₂N-(C₁-C₆-alkyl)-NH-, Hydroy-y-(C₁-C₆-alkyl)-NH-, (C₁-C₆-Alkyl)HN-(C₁-C₆-alkyl)-NH-, (C₁-C₆-Alkyl)₂N-(C₁-C₆-alkyl)-NH-, Heterocyclyl-(C₁-C₆-alkyl)- NH-Heteroaryl-(C₁-C₆-alkyl)-NH-, Phenyl-(C₁-C₆-alkyl)-NH-, -C(O)NH₂, -C(O)N(C₁-C₆-Alkyl)₂, -C(O)NH(C₁-C₆-Alkyl), H₂N-(C₁-C₆-alkyl)-NHC(O)-, Hydroxy-(C₁-C₆-alkyl)-NHC(O)-, (C₁-C₆-Alkyl)HN-(C₁-C₆-alkyl)-NHC(O)-, (C₁-C₆-alkyl)₂N-(C₁-C₆-alkyl)-NHC(O)-, Heterocyclyl-(C₁-C₆-alkyl)-NHC(O)-, Heteroaryl-(C₁-C₆-alkyl)-NHC(O)-, Phenyl-(C₁-C₆-alkyl)-NHC(O)-, Heterocyclyl, Heterocyclyl-(C₁-C₆-alkyl)-, Trifluormethyl und Trifluormethoxy,
   und die Heteroaryl-, Heterocyclyl- und Phenyl-Fragmente dieser Gruppe können wiederum mit C₁-C₆-Alkyl, C₁-C₆-Alkoxy, Halogen, Trifluormethyl, Trifluormethoxy oder OH zumindest monosubstituiert sein;
R³ ist besonders bevorzugt ausgewählt aus der Gruppe bestehend aus:
   Wasserstoff, Fluor, Chlor, Brom, -CN, -C(O)O-Methyl, (2-Diethylamino-ethyl)-NHC(O)-, COOH, Methoxy, Ethoxy, (2-Cyclohexylamino-ethyl)-NHC(O)-, (3-(4-Methyl-piperazin-1-yl)-propyl)-NHC(O)-, (3-Hydroxy-propyl)-NHC(O)-, (3-Cyclohexylamino-propyl)-NHC(O)-, (3-Imidazol-1-yl-propyl)-NHC(O)-, Methyl, Ethyl, 4-Methyl-piperazin-1-yl, Trifluormethyl und Trifluormethoxy;
R³ ist ganz besonders bevorzugt ausgewählt aus der Gruppe bestehend aus:
   Wasserstoff, Fluor, Chlor, Brom, Methyl und Ethyl;
R⁴ ist bevorzugt ausgewählt aus der Gruppe bestehend aus:
   Wasserstoff, Halogen, -CN, -CH₂-R⁸, -CH₂-NH₂, -CH₂-NH(C₁-C₆-Alkyl), CH₂-N(C₁-C₆-Alkyl)₂, -CH₂-OH, -CH₂-O-(C₁-C₆-Alkyl), -OR⁸, -C(O)R⁸, C(O)OR⁸, -NR⁸H, -NR⁸(C₁-C₆-Alkyl), -C(O)NR⁸H, -SR⁸, -SO₂NR⁸H, -SO₂-R⁸, Aryl, Heteroaryl, Heterocyclyl, Trifluormethyl und Trifluormethoxy, und Heterocyclyl, Aryl und Heteroaryl können wiederum mit C₁-C₆-Alkyl, C₁-C₆-Alkoxy, Oxo, Halogen, Trifluormethyl, Trifluormethoxy oder OH zumindest monosubstituiert sein;
R⁴ ist mehr bevorzugt ausgewählt aus der Gruppe bestehend aus:
   Wasserstoff, Fluor, Chlor, Brom, -CN, -SO₂NR⁸H, -SO₂-R⁸, -CH₂-R⁸, -CH₂-NH₂,-CH₂-NH(C₁-C₆-Alkyl), -CH₂-N(C₁-C₆-Alkyl)₂, -CH₂-OH, -CH₂-O-(C₁-C₆-Alkyl),-OR⁸, -C(O)R⁸, -C(O)OR⁸, -NR⁸H, -NR⁸(C₁-C₆-Alkyl), -C(O)NR⁸H, -SR⁸, Heterocyclyl, Trifluormethyl und Trifluormethoxy,
   und Heterocyclyl kann wiederum mit C₁-C₆-Alkyl, C₁-C₆-Alkoxy, Halogen, Trifluormethyl, Trifluormethoxy oder OH zumindest monosubstituiert sein;
R⁴ ist viel mehr bevorzugt ausgewählt aus der Gruppe bestehend aus:
   Wasserstoff, Fluor, Chlor, Brom,- CN, C₁-C₆-Alkyl, Phenyl-(C₁-C₆-alkyl)-, -OH, C₁-C₆-Alkoxy, -O-Phenyl, -C(O)OH, -C(O)O-(C₁-C₆-Alkyl), -NR⁸H, -NR⁸(C₁-C₆-Alkyl), -C(O)NR⁸H, Heterocyclyl, Trifluormethyl und Trifluormethoxy,
   und die Heterocyclyl- und Phenyl-Fragmente dieser Gruppe können wiederum mit C₁-C₆-Alkyl, C₁-C₆-Akoxy, Halogen, Trifluormethyl, Trifluormethoxy oder OH zumindest monosubstituiert sein;
R⁴ ist noch viel mehr bevorzugt ausgewählt aus der Gruppe bestehend aus:
   Wasserstoff, Fluor, Chlor, Brom, -CN, C₁-C₆-Alkyl, -OH, C₁-C₆-Alkoxy, - C(O)OH, -C(O)O-(C₁-C₆-Alkyl), -NH₂, -N(C₁-C₆-Alkyl)₂, -NH(C₁-C₆-Alkyl), H₂N-(C₁-C₆-alkyl)-NH-, Hydroxy-(C₁-C₆-alkyl)-NH-, (C₁-C₆-Alkyl)HN-(C₁-C₆-alkyl)-NH-, (C₁-C₆-Alkyl)₂N-(C₁-C₆-alkyl)-NH-, Heterocyclyl-(C₁-C₆-alkyl)-NH-, Heteroaryl-(C₁-C₆-alkyl)-NH-, Phenyl-(C₁-C₆-alkyl)-NH-, -C(O)NH₂, -C(O)N(C₁-C₆-Alkyl)₂, -C(O)NH(C₁-C₆-Alkyl), H₂N-(C₁-C₆-alkyl)-NHC(O)-, Hydroxy-(C₁-C₆-alkyl)-NHC(O)-, (C₁-C₆-Alkyl)HN-(C₁-C₆-alkyl)-NHC(O)-, (C₁-C₆-alkyl)₂N-(C₁-C₆-alkyl)-NHC(O)-, Heterocyclyl-(C₁-C₆-alkyl)-NHC(O)-, Heteroaryl-(C₁-C₆-alkyl)-NHC(O)-, Phenyl-(C₁-C₆-alkyl)-NHC(O)-, Heterocyclyl, Heterocyclyl-(C₁-C₆-alkyl)-, Trifluormethyl und Trifluormethoxy,
   und die Heteroaryl-, Heterocyclyl- und Phenyl-Fragmente dieser Gruppe können wiederum mit C₁-C₆-Alkyl, C₁-C₆-Alkoxy, Halogen, Trifluormethyl, Trifluormethoxy oder OH zumindest monosubstituiert sein;
R⁴ ist besonders bevorzugt ausgewählt aus der Gruppe bestehend aus:
   Wasserstoff, Fluor, Chlor, Brom, -CN, -C(O)O-Methyl, (2-Diethylamino-ethyl)-NHC(O)-, COOH, Methoxy, Ethoxy, (2-Cyclohexylamino-ethyl)-NHC(O)-, (3-(4-Methyl-piperazin-1-yl)-propyl)-NHC(O)-, (3-Hydroxy-propyl)-NHC(O)-, (3-Cyclohexylamino-propyl)-NHC(O)-, (3-Imidazol-1-yl-propyl)-NHC(O)-, Methyl, Ethyl, 4-Methyl-piperazin-1-yl, Trifluormethyl und Trifluormethoxy;
R⁵ ist bevorzugt ausgewählt aus der Gruppe bestehend aus:
   Wasserstoff, Halogen, -CN, -CH₂-R⁸, -CH₂-NH₂, -CH₂-NH(C₁-C₆-Alkyl), - CH₂-N(C₁-C₆-Alkyl)₂, -CH₂-OH, -CH₂-O-(C₁-C₆-Alkyl), -OR⁸, -C(O)R⁸, - C(O)OR⁸, -NR⁸H, -NR⁸(C₁-C₆-Alkyl), -C(O)NR⁸H, -SR⁸, -SO₂NR⁸H, -SO₂-R⁸, Aryl, Heteroaryl, Heterocyclyl, Trifluormethyl und Trifluormethoxy,
   und Heterocyclyl, Aryl und Heteroaryl können wiederum mit C₁-C₆-Alkyl, C₁-C₆-Alkoxy, Oxo, Halogen, Trifluormethyl, Trifluormethoxy oder OH zumindest monosubstituiert sein;
R⁵ ist mehr bevorzugt ausgewählt aus der Gruppe bestehend aus:
   Wasserstoff, Fluor, Chlor, Brom, -CN, -CH₂-R⁸, -CH₂-NH₂, -CH₂-NH(C₁-C₆-Alkyl), -CH₂-N(C₁-C₆-Alkyl)₂, -CH₂-OH, -CH₂-O-(C₁-C₆-Alkyl), -OR⁸, - C(O)R⁸, -C(O)OR⁸, -NR⁸H, -NR⁸(C₁-C₆-Alkyl), -C(O)NR⁸H, -SR⁸, - SO₂NR⁸H, -SO₂-R⁸ Heterocyclyl, Trifluormethyl und Trifluormethoxy,
   und Heterocyclyl kann wiederum mit C₁-C₆-Alkyl, C₁-C₆-Alkoxy, Halogen, Trifluormethyl, Trifluormethoxy oder OH zumindest monosubstituiert sein;
R⁵ ist viel mehr bevorzugt ausgewählt aus der Gruppe bestehend aus:
   Wasserstoff, Fluor, Chlor, Brom, -CN, C₁-C₆-Alkyl, Phenyl-(C₁-C₆-alkyl)-, -OH, C₁-C₆-Alkoxy, -O-Phenyl, -C(O)OH, -C(O)O-(C₁-C₆-Alkyl), -NR⁸H, -NR⁸(C₁-C₆-Alkyl), -C(O)NR⁸H, Heterocyclyl, Trifluormethyl und Trifluormethoxy,
   und die Heterocyclyl- und Phenyl-Fragmente dieser Gruppe können wiederum mit C₁-C₆-Alkyl, C₁-C₆-Alkoxy, Halogen, Trifluormethyl, Trifluormethoxy oder OH zumindest monosubstituiert sein;
R⁵ ist noch viel mehr bevorzugt ausgewählt aus der Gruppe bestehend aus:
   Wasserstoff, Fluor, Chlor, Brom, -CN, C₁-C₆-Alkyl, -OH, C₁-C₆-Alkoxy,-C(O)OH, -C(O)O-(C₁-C₆-Alkyl), -NH₂, -N(C₁-C₆-Alkyl)₂, -NH(C₁-C₆-Alkyl), H₂N-(C₁-C₆-alkyl)-NH-, Hydroxy-(C₁-C₆-alkyl)-NH-, (C₁-C₆-Alkyl)HN-(C₁-C₆-alkyl)-NH-, (C₁-C₆-Alkyl)₂N-(C₁-C₆-alkyl)-NH-, Heterocyclyl-(C₁-C₆-alkyl)- NH-Heteroaryl-(C₁-C₆-alkyl)-NH-, Phenyl-(C₁-C₆-alkyl)-NH-, -C(O)NH₂, -C(O)N(C₁-C₆-Alkyl)₂, -C(O)NH(C₁-C₆-Alkyl), H₂N-(C₁-C₆-alkyl)-NHC(O)-, Hydroxy-(C₁-C₆-alkyl)-NHC(O)-, (C₁-C₆-Alkyl)HN-(C₁-C₆-alkyl)-NHC(O)-, (C₁-C₆-alkyl)₂N-(C₁-C₆-alkyl)-NHC(O)-, Heterocylyl-(C₁-C₆-alkyl)-NHC(O)-, Heteroaryl-(C₁-C₆-alkyl)-NHC(O)-, Phenyl-(C₁-C₆-alkyl)-NHC(O)-, Heterocyclyl, Heterocyclyl-(C₁-C₆-alkyl)-, Trifluormethyl und Trifluormethoxy,
   und die Heteroaryl-, Heterocyclyl- und Phenyl-Fragmente dieser Gruppe können wiederum mit C₁-C₆-Alkyl, C₁-C₆-Alkoxy, Halogen, Trifluormethyl, Trifluormethoxy oder OH zumindest monosubstituiert sein;
R⁵ ist besonders bevorzugt ausgewählt aus der Gruppe bestehend aus:
   Wasserstoff, Fluor, Chlor, Brom, -CN, -C(O)O-Methyl, (2-Diethylamino-ethyl)-NHC(O)-, COOH, Methoxy, Ethoxy, (2-Cyclohexylamino-ethyl)-NHC(O)-, (3-(4-Methyl-piperazin-1-yl)-propyl)-NHC(O)-, (3-Hydroxy-propyl)-NHC(O)-, (3-Cyclohexylamino-propyl)-NHC(O)-, (3-Imidazol-1-yl-propyl)-NHC(O)-, Methyl, Ethyl, 4-Methyl-piperazin-1-yl, Trifluormethyl und Trifluormethoxy;
R⁶ ist bevorzugt ausgewählt aus der Gruppe bestehend aus:
   Wasserstoff, Halogen, -CN, -CH₂-R⁸, -CH₂-NH₂, -CH₂-NH(C₁-C₆-Akyl),-CH₂-N(C₁-C₆-Alkyl)₂, -CH₂-OH, -CH₂-O-(C₁-C₆-Alkyl), -OR⁸, -C(O)R⁸,-C(O)OR⁸, -NR⁸H, -NR⁸(C₁-C₆-Alkyl), -C(O)NR⁸H, -SR⁸, -SO₂NR⁸H, -SO₂-R⁸, Aryl, Heteroaryl, Heterocyclyl, Trifluormethyl und Trifluormethoxy,
   und Heterocyclyl, Aryl und Heteroaryl können wiederum mit C₁-C₆-Alkyl, C₁-C₆-Alkoxy, Oxo, Halogen, Trifluormethyl, Trifluormethoxy oder OH zumindest monosubstituiert sein;
R⁶ ist mehr bevorzugt ausgewählt aus der Gruppe bestehend aus:
   Wasserstoff, Fluor, Chlor, Brom, -CN, -CH₂-R⁸, -CH₂-NH₂, -CH₂-NH(C₁-C₆-Alkyl), -CH₂-N(C₁-C₆-Alkyl)₂, -CH₂-OH, -CH₂-O-(C₁-C₆-Alkyl), -OR⁸,-C(O)R⁸, -C(O)OR⁸, -NR⁸H, -NR⁸(C₁-C₆-Alkyl), -C(O)NR⁸H, -SR⁸,-SO₂NR⁸H, -SO₂-R⁸, Heterocyclyl, Trifluormethyl und Trifluormethoxy,
   und Heterocyclyl kann wiederum mit C₁-C₆-Alkyl, C₁-C₆-Alkoxy, Halogen, Trifluormethyl, Trifluormethoxy oder OH zumindest monosubstituiert sein;
R⁶ ist viel mehr bevorzugt ausgewählt aus der Gruppe bestehend aus:
   Wasserstoff, Fluor, Chlor, Brom, -CN, C₁-C₆-Alkyl, Phenyl-(C₁-C₆-alkyl)-, -OH, C₁-C₆-Alkoxy, -O-Phenyl, -C(O)OH, -C(O)O-(C₁-C₆-Alkyl), -NR⁸H, -NR⁸(C₁-C₆-Alkyl), -C(O)NR⁸H, Heterocyclyl, Trifluormethyl und Trifluormethoxy,
   und die Heterocyclyl- und Phenyl-Fragmente dieser Gruppe können wiederum mit C₁-C₆-Alkyl, C₁-C₆-Alkoxy, Halogen, Trifluormethyl, Trifluormethoxy oder OH zumindest monosubstituiert sein;
R⁶ ist noch viel mehr bevorzugt ausgewählt aus der Gruppe bestehend aus:
   Wasserstoff, Fluor, Chlor, Brom, -CN, C₁-C₆-Alkyl, -OH, C₁-C₆-Alkoxy,-C(O)OH, -C(O)O-(C₁-C₆-Alkyl), -NH₂, -N(C₁-C₆-Mkyl)₂, -NH(C₁-C₆-Alkyl), H₂N-(C₁-C₆-alkyl)-NH-, Hydroxy-(C₁-C₆-allcyl)-NH-, (C₁-C₆-Alkyl)HN-(C₁-C₆-alkyl)-NH-, (C₁-C₆-Alkyl)₂N-(C₁-C₆-alkyl)-NH-, Heterocyclyl-(C₁-C₆-alkyl)-NH-Heteroaryl-(C₁-C₆-alkyl)-NH-, Phenyl-(C₁-C₆-alkyl)-NH-, -C(O)NH₂, -C(O)N(C₁-C₆-Alkyl)₂, -C(O)NH(C₁-C₆-Alkyl), H₂N-(C₁-C₆-alkyl)-NHC(O)-, Hydroxy-(C₁-C₆-alkyl)-NHC(O)-, (C₁-C₆Alkyl)HN-(C₁-C₆alkyl)-NHC(O)-, (C₁-C₆-alkyl)₂N-(C₁-C₆-alkyl)-NHC(O)-, Heterocyclyl-(C₁-C₆-alkyl)- NHC(O)-, Heteroaryl-(C₁-C₆-alkyl)-NHC(O)-, Phenyl-(C₁-C₆-alkyl)-NHC(O)-, Heterocyclyl, Heterocyclyl-(C₁-C₆-alkyl)-, Trifluormethyl und Trifluormethoxy,
   und die Heteroaryl-, Heterocyclyl- und Phenyl-Fragmente dieser Gruppe können wiederum mit C₁-C₆-Alkyl, C₁-C₆-Alkoxy, Halogen, Trifluormethyl, Trifluormethoxy oder OH zumindest monosubstituiert sein;
R⁶ ist besonders bevorzugt ausgewählt aus der Gruppe bestehend aus:
   Wasserstoff, Fluor, Chlor, Brom, -CN, -C(O)O-Methyl, (2-Diethylamino-ethyl)-NHC(O)-, COOH, Methoxy, Ethoxy, (2-Cyclohexylamino-ethyl)-NHC(O)-, (3-(4-Methyl-piperazin-1-yl)-propyl)-NHC(O)-, (3-Hydroxy-propyl)-NHC(O)-, (3-Cyclohexylamino-propyl)-NHC(O)-, (3-Imidazol-1-yl-propyl)-NHC(O)-, Methyl, Ethyl, 4-Methyl-piperazin-1-yl, Trifluormethyl und Trifluormethoxy;
R⁶ ist ganz besonders bevorzugt Wasserstoff;
R⁷ ist bevorzugt:
   H;
   unsubstituiertes oder zumindest monosubstituiertes C₁-C₆-Alkyl, Heterocyclyl, Phenyl oder Heteroaryl,
   wobei die Substituenten ausgewählt sind aus der Gruppe bestehend aus: Heteroaryl, Heterocyclyl, Phenyl, Fluor, Chlor, Brom, -OH, C₁-C₆-Alkoxy, -C(O)OH,-C(O)O-(C₁-C₆-alkyl), -C(O)NH₂, -C(O)NH(C₁-C₆-Alkyl), -C(O)N(C₁-C₁₀-Alkyl)₂, Trifluormethyl, Trifluormethoxy, -NH₂, -NH(C₁-C₆-Alkyl) und-N(C₁-C₆-alkyl)₂,
   und Heterocyclyl, Phenyl und Heteroaryl können wiederum mit C₁-C₃-Alkyl, C₁-C₃-Alkoxy, Oxo, Trifluormethyl, Trifluormethoxy, Fluor, Chlor oder OH zumindest monosubstituiert sein;
R⁷ ist mehr bevorzugt:
   unsubstituiertes oder zumindest monosubstituiertes C₁-C₆-Alkyl,
   wobei die Substituenten ausgewählt sind aus der Gruppe bestehend aus: Heteroaryl, Heterocyclyl, -OH, -C(O)OH, -C(O)O-(C₁-C₆-alkyl), -C(O)NH₂, -C(O)NH(C₁-C₆-Alkyl), -C(O)N(C₁-C₁₀-Alkyl)₂, -NH₂, -NH(C₁-C₆-Alkyl) und -N(C₁-C₆-alkyl)₂,
   und Heterocyclyl, und Heteroaryl können wiederum mit C₁-C₃-Alkyl, C₁-C₃-Alkoxy, Trifluormethyl, Trifluormethoxy, Fluor, Chlor oder OH zumindest monosubstituiert sein;
R⁷ ist besonders bevorzugt:
   unsubstituiertes oder zumindest monosubstituiertes C₁-C₄-Alkyl,
   wobei die Substituenten ausgewählt sind aus der Gruppe bestehend aus: Morpholinyl, Piperazinyl, Piperidinyl, Imidazolyl, -NH₂, -NH(C₁-C₆-Alkyl) und-N(C₁-C₆-alkyl)₂,
   und Morpholinyl, Piperazinyl, Piperidinyl und Imidazolyl können wiederum mit C₁-C₃-Alkyl, C₁-C₃-Alkoxy, Trifluormethyl, Trifluormethoxy, Fluor, Chlor oder OH monosubstituiert sein;
R⁸ ist bevorzugt:
   H;
   unsubstituiertes oder zumindest monosubstituiertes C₁-C₆-Alkyl, Heterocyclyl, Phenyl oder Heteroaryl,
   wobei die Substituenten ausgewählt sind aus der Gruppe bestehend aus: Heteroaryl, Heterocyclyl, Phenyl, Fluor, Chlor, Brom, -OH, C₁-C₆-Alkoxy, -C(O)OH,-C(O)O-(C₁-C₆-alkyl), -C(O)NH₂, -C(O)NH(C₁-C₆-Alkyl), -C(O)N(C₁-C₁₀-Alkyl)₂, Trifluormethyl, Trifluormethoxy, -NH₂, -NH(C₁-C₆-Alkyl) und-N(C₁-C₆-alkyl)₂,
   und Heterocyclyl, Phenyl und Heteroaryl können wiederum mit C₁-C₃-Alkyl, C₁-C₃-Alkoxy, Oxo, Trifluormethyl, Trifluormethoxy, Fluor, Chlor oder OH zumindest monosubstituiert sein;
R⁸ ist mehr bevorzugt:
   unsubstituiertes oder zumindest monosubstituiertes C₁-C₆-Alkyl,
   wobei die Substituenten ausgewählt sind aus der Gruppe bestehend aus: Heteroaryl, Heterocyclyl, -OH, -C(O)OH, -C(O)O-(C₁-C₆-alkyl), -C(O)NH₂, -C(O)NH(C₁-C₆-Alkyl), -C(O)N(C₁-C₁₀-Alkyl)₂, -NH₂, -NH(C₁-C₆-Alkyl) und -N(C₁-C₆-alkyl)₂,
   und Heterocyclyl, und Heteroaryl können wiederum mit C₁-C₃-Alkyl, C₁-C₃-Alkoxy, Trifluormethyl, Trifluormethoxy, Fluor, Chlor oder OH zumindest monosubstituiert sein;
R⁸ ist besonders bevorzugt:
   unsubstituiertes oder zumindest monosubstituiertes C₁-C₄-Alkyl,
   wobei die Substituenten ausgewählt sind aus der Gruppe bestehend aus: Morpholinyl, Piperazinyl, Piperidinyl, Imidazolyl, -NH₂, -NH(C₁-C₆-Alkyl) und-N(C₁-C₆-alkyl)₂,
   und Morpholinyl, Piperazinyl, Piperidinyl und Imidazolyl können wiederum mit C₁-C₃-Alkyl, C₁-C₃-Alkoxy, Trifluormethyl, Trifluormethoxy, Fluor, Chlor oder OH monosubstituiert sein;
Heteroaryl ist bevorzugt Imidazolyl, Thiophenyl, Furanyl, Thiazolyl, Imidazolyl, Oxazolyl, Isoxazolyl, Pyridinyl, Pyrimidinyl, Pyrazolyl, Benzo[b]thiophenyl, Thiazolo[3,2-b][1,2,4]-triazolyl, Pyrrolyl, Chinolinyl, .Isochinolinyl, 1,2,3,4-Tetrahydrochinolinyl, Benzimidazolyl, Indolyl oder 1,3-Benzodioxolyl; Heteroaryl ist besonders bevorzugt Pyridinyl, Thiophenyl oder Pyrimidinyl;
Aryl ist bevorzugt Naphthyl, Indanyl oder Phenyl; Aryl ist besonders bevorzugt Phenyl.
Heterocyclyl ist bevorzugt 2-Oxo-azepanyl, 1,4-Oxazepanyl, Dihydropyridinonyl, Tetrahydrofuranyl, 1,3-Dioxolanyl, Morpholinyl, Pyrrolidinyl, Piperazinyl oder Piperidinyl; Heterocyclyl ist besonders bevorzugt Piperidinyl, Morpholinyl oder Piperazinyl;

Beispiele für Ausführungsformen mit bevorzugten Verbindungen der allgemeinen Formel (I) unter Bezugnahme auf die vorstehend beschriebenen Bedeutungen (Definitionen) sind:
i) R¹, R², R³, R⁴, R⁵, R⁶, R⁷, R⁸, A, B, D, E, Heteroaryl, Heterocyclyl und Aryl haben ihre bevorzugte Bedeutung; oder
ii) R¹ hat seine bevorzugte Bedeutung und alle anderen Substituenten haben ihre Grundbedeutung; oder
iii) R² hat seine bevorzugte Bedeutung und alle anderen Substituenten haben ihre Grundbedeutung; oder
iv) R³ hat seine bevorzugte Bedeutung und alle anderen Substituenten haben ihre Grundbedeutung; oder
v) R⁴ hat seine bevorzugte Bedeutung und alle anderen Substituenten haben ihre Grundbedeutung; oder
vi) R⁵ hat seine bevorzugte Bedeutung und alle anderen Substituenten haben ihre Grundbedeutung; oder
vii) R⁶ hat seine bevorzugte Bedeutung und alle anderen Substituenten haben ihre Grundbedeutung; oder
viii) R⁷ hat seine bevorzugte Bedeutung und alle anderen Substituenten haben ihre Grundbedeutung; oder
ix) R⁸ hat seine bevorzugte Bedeutung und alle anderen Substituenten haben ihre Grundbedeutung; oder
x) A hat seine bevorzugte Bedeutung und alle anderen Substituenten haben ihre Grundbedeutung; oder
xi) B hat seine bevorzugte Bedeutung und alle anderen Substituenten haben ihre Grundbedeutung; oder
xii) D hat seine bevorzugte Bedeutung und alle anderen Substituenten haben ihre Grundbedeutung; oder
xiii) E hat seine bevorzugte Bedeutung und alle anderen Substituenten haben ihre Grundbedeutung; oder
xiv) Heteroaryl hat seine bevorzugte Bedeutung und alle anderen Substituenten haben ihre Grundbedeutung; oder
xv) Heterocyclyl hat seine bevorzugte Bedeutung und alle anderen Substituenten haben ihre Grundbedeutung; oder
xvi) Aryl hat seine bevorzugte Bedeutung und alle anderen Substituenten haben ihre Grundbedeutung; oder
xvii) R¹ R², R³, R⁴, R⁵, R⁶, R⁷, R⁸, A, E, Heteroaryl, Heterocyclyl und Aryl haben ihre bevorzugte Bedeutung und B und D haben ihre Grundbedeutung; oder
xviii) R¹, R³, R⁴, R⁵ und R⁶ haben ihre mehr bevorzugte Bedeutung, R⁷, R⁸, A, D, E, Heteroaryl, Heterocyclyl und Aryl haben ihre bevorzugte Bedeutung, R² hat seine besonders bevorzugte Bedeutung und B hat seine Grundbedeutung; oder
xix) R¹ hat seine viel mehr bevorzugte Bedeutung, R³, R⁴, R⁵ und R⁶ haben ihre noch viel mehr bevorzugte Bedeutung, A, D, E, Heteroaryl und Heterocyclyl haben ihre bevorzugte Bedeutung, R² hat seine besonders bevorzugte Bedeutung und B hat seine Grundbedeutung; oder
xx) R¹ hat seine ganz besonders bevorzugte Bedeutung, R², R³, R⁴, R⁵ und R⁶ haben ihre besonders bevorzugte Bedeutung, A, D und E haben ihre bevorzugte Bedeutung, und B hat seine Grundbedeutung; oder
xxi) R¹, R³ und R⁶ haben ihre ganz besonders bevorzugte Bedeutung, R², R⁴ und R⁵ haben ihre besonders bevorzugte Bedeutung, A, D und E haben ihre bevorzugte Bedeutung, und B hat seine Grundbedeutung; oder
xxii) R³ und R⁶ haben ihre ganz besonders bevorzugte Bedeutung, R¹, R², R⁴ und R⁵ haben ihre besonders bevorzugte Bedeutung, A, D und E haben ihre bevorzugte Bedeutung, und B hat seine Grundbedeutung; oder
xxiii) R¹, R², R³, R⁴, R⁵ und R⁶ haben ihre besonders bevorzugte Bedeutung, A, D und E haben ihre bevorzugte Bedeutung, und B hat seine Grundbedeutung; oder
xxiv) R¹ hat seine viel mehr bevorzugte Bedeutung, R², R³, R⁴, R⁵ und R⁶ haben ihre besonders bevorzugte Bedeutung, A, D, E, Heteroaryl und Heterocyclyl haben ihre bevorzugte Bedeutung und B hat seine Grundbedeutung; oder
xxv) R¹ hat seine viel mehr bevorzugte Bedeutung, R³ und R⁶ haben ihre ganz besonders bevorzugte Bedeutung, R², R⁴ und R⁵ haben ihre besonders bevorzugte Bedeutung, A, D, E, Heteroaryl und Heterocyclyl haben ihre bevorzugte Bedeutung und B hat seine Grundbedeutung; oder
xxvi) R¹ hat seine viel mehr bevorzugte Bedeutung, R³ und R⁶ haben ihre ganz besonders bevorzugte Bedeutung, R², R⁴, R⁵, Heteroaryl und Heterocyclyl haben ihre besonders bevorzugte Bedeutung, A, D und E haben ihre bevorzugte Bedeutung und B hat seine Grundbedeutung; oder
xxvii) R¹ hat seine viel mehr bevorzugte Bedeutung, R², R³, R⁴, R⁵, R⁶, Heteroaryl und Heterocyclyl haben ihre besonders bevorzugte Bedeutung, A, D und E haben ihre bevorzugte Bedeutung und B hat seine Grundbedeutung; oder
xxviii) R¹ hat seine viel mehr bevorzugte Bedeutung, R³, R⁴, R⁵, R⁶, Heteroaryl und Heterocyclyl haben ihre besonders bevorzugte Bedeutung, R², A, D und E haben ihre bevorzugte Bedeutung und B hat seine Grundbedeutung; oder
xxix) R¹, R⁴, R⁵, R⁷, R⁸, A, E, Heteroaryl, Heterocyclyl und Aryl haben ihre bevorzugte Bedeutung, R³ und R⁶ haben ihre ganz besonders bevorzugte Bedeutung, R² hat seine besonders bevorzugte Bedeutung und B und D haben ihre Grundbedeutung; oder
xxx) R¹, R³, R⁴, R⁵, R⁶, A, E, Heteroaryl, Heterocyclyl und Aryl haben ihre bevorzugte Bedeutung, R⁷ und R⁸ haben ihre mehr bevorzugte Bedeutung, R² hat seine besonders bevorzugte Bedeutung und B und D haben ihre Grundbedeutung; oder
xxxi) R¹, R³, R⁴, R⁵, R⁶ haben ihre mehr bevorzugte Bedeutung, R², A, D, E, Heteroaryl, Heterocyclyl und Aryl haben ihre bevorzugte Bedeutung, R⁷ und R⁸ haben ihre besonders bevorzugte Bedeutung und B hat seine Grundbedeutung; oder
xxxii) R¹, R³, R⁴, R⁵, R⁶ haben ihre mehr bevorzugte Bedeutung, A, D, E, Heteroaryl, Heterocyclyl und Aryl haben ihre bevorzugte Bedeutung, R², R⁷ und R⁸ haben ihre besonders bevorzugte Bedeutung und B und D haben ihre Grundbedeutung; oder
xxxiii) R¹ hat seine mehr bevorzugte Bedeutung, R⁷, A, D, E, Heteroaryl, Heterocyclyl und Aryl haben ihre bevorzugte Bedeutung, R⁴ und R⁵ haben ihre noch viel mehr bevorzugte Bedeutung, R³ und R⁶ haben ihre ganz besonders bevorzugte Bedeutung, R² hat seine besonders bevorzugte Bedeutung und B und D haben ihre Grundbedeutung; oder
xxxiv) R¹ hat seine viel mehr bevorzugte Bedeutung, R³, R⁴, R⁵ und R⁶ haben ihre mehr bevorzugte Bedeutung, R⁸, A, D, E, Heteroaryl, Heterocyclyl und Aryl haben ihre bevorzugte Bedeutung, R² hat seine besonders bevorzugte Bedeutung und B hat seine Grundbedeutung; oder
xxxv) R¹ hat seine viel mehr bevorzugte Bedeutung, R³, R⁴, R⁵ und R⁶ haben ihre noch viel mehr bevorzugte Bedeutung, B, D, E, Heteroaryl und Heterocyclyl haben ihre bevorzugte Bedeutung, R² hat seine besonders bevorzugte Bedeutung und A hat seine Grundbedeutung; oder
xxxvi) R¹ hat seine ganz besonders bevorzugte Bedeutung, R², R³, R⁴, R⁵ und R⁶ haben ihre besonders bevorzugte Bedeutung, B, D und E haben ihre bevorzugte Bedeutung, und A hat seine Grundbedeutung;

Wie oben aufgeführt, sind die bevorzugten Verbindungen der allgemeinen Formel (I) nicht beschränkt auf die vorgenannten Beispiele. Vielmehr sind alle Kombinationen der einzelnen Substituenten in ihrer Grundbedeutung mit den bevorzugten, mehr bevorzugten, noch mehr bevorzugten, viel mehr bevorzugten, noch viel mehr bevorzugten, besonders bevorzugten oder ganz besonders bevorzugten Bedeutungen der übrigen Substituenten oder alle Kombinationen der bevorzugten, mehr bevorzugten, noch mehr bevorzugten, viel mehr bevorzugten, noch viel mehr bevorzugten, besonders bevorzugten oder ganz besonders bevorzugten Bedeutungen der einzelnen Substituenten, die vorstehend nicht als Beispiel aufgeführt sind, auch Gegenstand dieser Erfindung. Dies trifft selbstverständlich nur dann zu, so weit die Definitionen der jeweiligen Substituenten eine solche Kombination zulassen.

Besonders bevorzugte Verbindungen gemäß der allgemeinen Formel (I) sind ausgewählt aus der Gruppe bestehend aus: 4-(1H-Benzimidazol-2-yl)-6-(4-hydroxy-3-methoxy-5-methylaminomethyl-phenyl)-2H- pyridazin-3-on, 2-(3-Oxo-6-pyridin-4-yl-2,3-dihydropyridazin-4-yl)-1H-benzimidazol-5-carbonsäure-(2-diethylamino-ethyl)-amid, 4-(6-Trifluormethyl-1H-benzimidazol-2-yl)-6-pyridin-4-yl-2H-pyridazin-3-on, 4-(6-Methoxy-1H-benzimidazol-2-yl)-6-pyridin-4-yl-2H-pyridazin-3-on, 4-(5-Chlor-1H-benzimidazol-2-yl)-6-methyl-2H-pyridazin-3-on, 4-(6-Chlor-1H-benzimidazol-2-yl)-6-(4-hydroxy-3,5-dimethyl-phenyl)-2H-pyridazin-3-on, 4-(5-Fluor-1H-benzimidazol-2-yl)-6-pyridin-4-yl-2H-pyridazin-3-on, 6-(4-Hydroxy-3-methoxy-phenyl)-4-(6-trifluormethyl-1H-benzimidazol-2-yl)-2H-pyridazin-3-on, 6-(2-Butylamino-pyrimidin-4-yl)-4-(6-chlor-1H-benzimidazol-2-yl)-2H-pyridazin-3-on, 4-(1H-Benzimidazol-2-yl)-6-pyridin-4-yl-2H-pyridazin-3-on, 6-[2-(2-Morpholin-4-yl-ethylamino)-pyrimidin-4.-yl]-4-(6-trifluormethyl-1H-benzimidazol-2-yl)-2H-pyridazin-3-on und 4-(3H-Imidazol[4,5-c]pyridin-2-yl)-6-pyridin-4-yl-2H-pyridazin-3-on;

Es wird nochmals ausdrücklich darauf hingewiesen, dass auch für die bevorzugten und besonders bevorzugten Verbindungen gemäß Formel (I) die vorstehenden Ausführungen bezüglich der Salze, Stereoisomeren, Prodrugs, N-Oxide usw. gelten; insbesondere sind die jeweiligen physiologisch verträglichen Salze mitumfasst.

In einer weiteren Ausführungsform der vorliegenden Erfindung sind die Verbindungen gemäß Formel (I) wie folgt definiert:
A ist CR³ oder N;
B ist CR⁴ oder N;
D ist CR⁵ oder N;
E ist CR⁶ oder N;
wobei maximal drei der Substituenten A, B, D und E gleichzeitig N sein können;
- R¹: ist Halogen;
unsubstituiertes oder zumindest monosubstituiertes C₁-C₁₀-Alkyl,
wobei die Substituenten ausgewählt sind aus der Gruppe bestehend aus: Halogen, -CN, NO₂, -OR⁷, -C(O)R⁷, -C(O)OR⁷, -O-C(O)R⁷, -NR⁷R⁸, -NHC(O)R⁷, -C(O)NR⁷R⁸, -NHC(S)R⁷, -C(S)NR⁷R⁸, -SR⁷, -S(O)R⁷,-SO₂R⁷, -NHSO₂R⁷ -SO₂NR⁷R⁸, -O-SO₂R⁷, -SO₂-O-R⁷, Aryl, Heteroaryl, Heterocyclyl, Trifluormethyl und Trifluormethoxy,
und Heterocyclyl, Aryl und Heteroaryl wiederum mit C₁-C₆-Alkyl, C₁-C₆-Alkoxy, Halogen, Trifluormethyl, Trifluormethoxy oder OH zumindest monosubstituiert sein können;
unsubstituiertes oder zumindest monosubstituiertes Aryl oder Heteroaryl,
wobei die Substituenten ausgewählt sind aus der Gruppe bestehend aus: Halogen, -CN, NO₂, -CH₂-R⁷, -OR⁷, -C(O)R⁷, -C(O)OR⁷, -O-C(O)R⁷,-NR⁷R⁸, -NHC(O)R⁷, -C(O)NR⁷R⁸, -NHC(S)R⁷, -C(S)NR⁷R⁸, -SR⁷, -S(O)R⁷, -SO₂R⁷, -NHSO₂R⁷, -SO₂NR⁷R⁸, -O-SO₂R⁷, -SO₂-O-R⁷, Aryl, Heteroaryl, Trifluormethyl und Trifluormethoxy,
und Aryl und Heteroaryl können wiederum mit C₁-C₆-Alkyl, C₁-C₆-Alkoxy, Halogen, Trifluormethyl, Trifluormethoxy oder OH zumindest monosubstituiert sein;
- R²: ist Wasserstoff oder C₁-C₁₀-Alkyl;
- R³, R⁴, R⁵ und R⁶: sind unabhängig voneinander ausgewählt aus der Gruppe bestehend aus: Wasserstoff, Halogen, -CN, NO₂, -CH₂-R⁸, -OR⁸, -C(O)R⁸,-C(O)OR⁸, -O-C(O)R⁸, -NR⁷R⁸; -NHC(O)R⁸, -C(O)NR⁷R⁸, -NHC(S)R⁸,-C(S)NR⁷R⁸, -SR⁸, -S(O)R⁸, -SO₂R⁸, -NHSO₂R⁸, -SO₂NR⁷R⁸, -O-SO₂R⁸,-SO₂-O-R⁸, Aryl, Heteroaryl, Heterocyclyl, Trifluormethyl und Trifluormethoxy,
und Heterocyclyl, Aryl und Heteroaryl können wiederum mit C₁-C₆-Alkyl, C₁-C₆-Alkoxy, Oxo, Halogen, Trifluormethyl, Trifluormethoxy oder OH zumindest monosubstituiert sein;
- R⁷ und R⁸: sind unabhängig voneinander:
H;
unsubstituiertes oder zumindest monosubstituiertes C₁-C₁₀-Alkyl, C₂-C₁₀-Alkenyl, C₂-C₁₀-Alkinyl, Heterocyclyl, Aryl oder Heteroaryl,
wobei die Substituenten ausgewählt sind aus der Gruppe bestehend aus: Heteroaryl, Heterocyclyl, Aryl, Halogen, OH, Oxo, C₁-C₁₀-Alkoxy, (C₁-C₁₀-Alkyl)thio-, COOH, -COO-(C₁-C₆-alkyl), -CONH₂, Trifluormethyl, Trifluormethoxy; CN, NH₂, (C₁-C₁₀-Alkyl)amino- und Di-(C₁-C₁₀-alkyl)amino-,
und Heterocyclyl, Aryl und Heteroaryl können wiederum mit C₁-C₆-Alkyl, C₁-C₆-Alkoxy, Oxo, Trifluormethyl, Trifluormethoxy, Fluor, Chlor oder OH zumindest monosubstituiert sein;
Heteroaryl ist ein 5 bis 10-gliedriger, aromatischer, mono- oder bicyclischer Heterocyclus, der ein oder mehrere Heteroatome ausgewählt aus N, O und S enthält;
Aryl ist ein 5 bis 10-gliedriger, aromatischer, Mono- oder Bicyclus.
Heterocyclyl ist ein 5 bis 10-gliedriger, nicht-aromatischer, mono- oder bicyclischer Heterocyclus, der ein oder mehrere Heteroatome ausgewählt aus N, O und S enthält;
oder ein physiologisch verträgliches Salz davon.

Vorzugsweise ist in dieser weiteren Ausführungsform R¹ nicht unsubstituiertes oder zumindest mono-substituiertes Pyrazolo[1,5-a]pyridinyl.

Mehr bevorzugte Verbindungen dieser weiteren Ausführungsform sind wie folgt definiert:
- A: ist CR³;
- B: ist CR⁴ oder N;
- D: ist CR⁵;
- E: ist CR⁶;
- R¹: ist unsubstituiertes oder zumindest monosubstituiertes Phenyl, Pyridinyl, Pyrimidinyl, Pyrazolyl, Thiophenyl, Oxazolyl, Isoxazolyl, Benzo[b]thiophenyl, Benzodioxolyl oder Thiazolo[3,2-b][1,2,4]-triazolyl,
wobei die Substituenten ausgewählt sind aus der Gruppe bestehend aus: Halogen, C₁-C₆-Alkyl, Phenyl-(C₁-C₆-alkyl)-, -OH, C₁-C₆-Alkoxy, (C₁-C₆-Alkyl)thio-, -O-Phenyl, -NH₂, -N(C₁-C₆-Alkyl)₂, -NH(C₁-C₆-Alkyl),-NH(Amino-(C₁-C₆-alkyl-)), -NH((C₁-C₆-Alkyl)amino-(C₁-C₆-alkyl-)),-NH(Di-(C₁-C₆-alkyl)amino-(C₁-C₆-alkyl-)), -NH(Heterocyclyl-(C₁-C₆-alkyl-)), -NH(Heteroaryl-(C₁-C₆-alkyl-)), -NH(Phenyl-(C₁-C₆-alkyl-)), Trifluormethyl, Trifluormethoxy, Phenyl und Heteroaryl,
und Heterocyclyl. Phenyl und Heteroaryl können wiederum mit C₁-C₃-Alkyl, C₁-C₃-Alkoxy, Fluor, Chlor, Trifluormethyl, Trifluormethoxy oder OH zumindest monosubstituiert sein;
- R²: ist Wasserstoff;
- R³, R⁴, R⁵ und R⁶: sind unabhängig voneinander ausgewählt aus der Gruppe bestehend aus:
Wasserstoff, Fluor, Chlor, Brom, -CN, C₁-C₆-Alkyl, -OH, C₁-C₆-Alkoxy,-C(O)OH, -C(O)O-(C₁-C₆-Alkyl), -NH₂, -N(C₁-C₆-Alkyl)₂, -NH(C₁-C₆-Alkyl), -NH(Ammo-(C₁-C₆-alkyl-)), -NH(Hydroxy-(C₁-C₆-alkyl-)),-NH((C₁-C₆-Alkyl)amino-(C₁-C₆-alkyl-)), -NH(Di-(C₁-C₆-alkyl)amino-(C₁-C₆-alkyl-)), -NH(Heterocyclyl-(C₁-C₆-alkyl-)), -NH(Heteroaryl-(C₁-C₆-alkyl-)), -NH(Phenyl-(C₁-C₆-alkyl-)), -C(O)NH₂, -C(O)NH-(C₁-C₆-alkyl), -C(O)N(C₁-C₆-Alkyl)₂, -C(O)NH(C₁-C₆-Alkyl), -C(O)NH(Amino-(C₁-C₆-alkyl-)), -C(O)NH(Hydroxy-(C₁-C₆-alkyl-)), -C(O)NH((C₁-C₆-Alkyl)amino-(C₁-C₆-alkyl-)), -C(O)NH(Di-(C₁-C₆-akyl)amino-(C₁-C₆-alkyl-)), -C(O)NH(Heterocyclyl-(C₁-C₆-alkyl-)), -C(O)NH(Heteroaryl-(C₁-C₆-alkyl-)), -C(O)NH(Phenyl-(C₁-C₆-alkyl-)), Heterocyclyl, Trifluormethyl und Trifluormethoxy,
und Heteroaryl, Heterocyclyl und Phenyl können wiederum mit C₁-C₆-Alkyl, C₁-C₆-Alkoxy, Halogen, Trifluormethyl, Trifluormethoxy oder OH zumindest monosubstituiert sein;
Heteroaryl ist Imidazolyl, Thiophenyl, Furanyl, Oxazolyl, Isoxazolyl, Pyridinyl, Pyrimidinyl, Pyrazolyl, Benzo[b]thiophenyl, Thiazolo[3,2-b][1,2,4]-triazolyl, Pyrrolyl, Chinolinyl, Isochinolinyl, 1,2,3,4-Tetrahydrochinolinyl, Benzoimidazolyl, Indolyl oder 1,3-Benzodioxolyl;
Heterocyclyl ist 2-Oxo-azepanyl, Tetrahydrofuranyl, 1,3-Dioxolanyl, Morpholinyl, Pyrrolidinyl, Piperazinyl oder Piperidinyl;
oder ein physiologisch verträgliches Salz davon.

Die Herstellung der Verbindungen erfolgt nach an sich bekannten Verfahren dadurch, dass man aus aktiven Säurederivaten der Formel (II), wobei Y eine Austrittsgruppe, vorzugsweise -OH, C₁-C₁₀-Alkoxy, Chlor, -O-C(O)-(C₁-C₁₀-alkyl oder -O-C(O)-O-(C₁-C₁₀-alkyl) bedeutet, und 1,2-Diaminophenyl- oder 1,2-Diaminoheterozyklyl-derivaten der Formel (III) die Monoacylderivate (IV) herstellt und diese in geeigneter Weise zyklisiert. Geeignete Zyklisierungsmittel können Säuren wie Eisessig, Salzsäure, Schwefelsäure, Phosphorsäure oder Wasser entziehende Mittel wie Phosphorpentoxid sein. Nach der Zyklisierung können die Substituenten A, B, D, E, R¹ und R² (sowie die weiteren Substituenten) gegebenenfalls nach bekannten Verfahren modifiziert werden zu den beanspruchten Verbindungen der Formel (I).

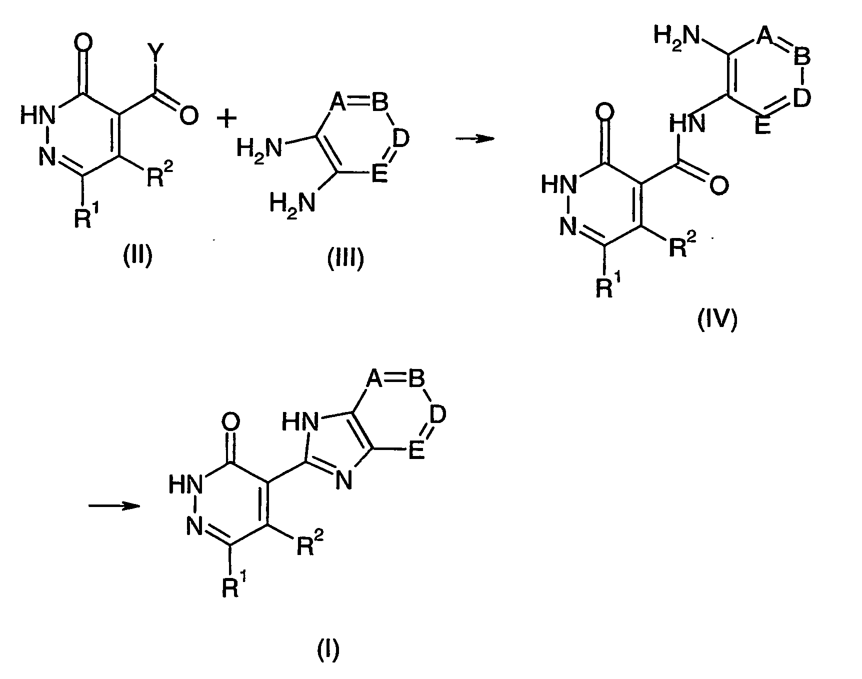

Eine weitere bekannte Methode zur Herstellung besteht in der Umsetzung von Aldehyd, d.h. in der Formel (II) ist Y gleich Wasserstoff mit den Verbindungen der Formel (III), wobei die primär gebildeten Dihydroverbindungen durch Luft oder (reinen) Sauerstoff oder andere Oxidantien umgewandelt werden zu den Verbindungen der Formel (I).

Weiterhin kann man die Verbindungen der allgemeinen Formel (I) herstellen durch Palladium katalysierte Kupplung gemäß einer Suzuki-Reaktion (I. Parrot et al., Synthesis; 7; 1999; 1163 bis 1168). Hierbei wird eine Verbindung der Formel (VI), wobei Y1 gleich Halogen, B(OH)₂ oder Sn(C₁-C₁₀-alkyl) und Y2 gleich H oder eine Schutzgruppe ist, mit einer Verbindung der Formel (V) umgesetzt.

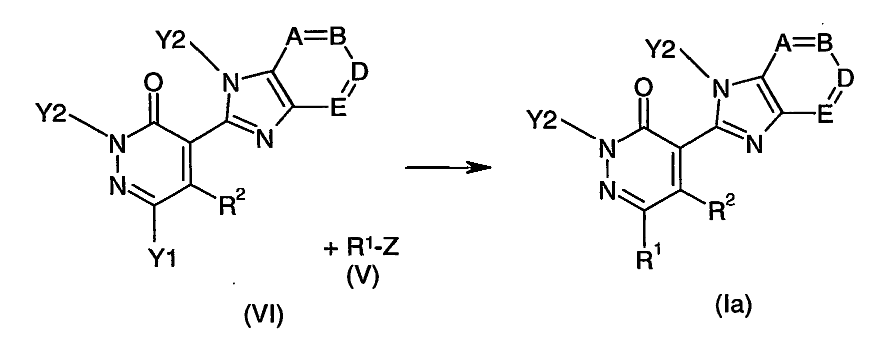

In Formel (V) ist R¹ gleich unsubstituiertes oder zumindest monosubstituiertes Aryl oder Heteroaryl gemäß der Definition von Formel (I). Z kann beispielsweise B(OH)₂, B(C₁-C₁₀-alkyl)₂, Sn (C₁-C₁₀-alkyl)₃, Zn-(C₁-C₁₀-alkyl) oder Halogen sein. Sofern Y2 eine Schutzgruppe ist, wird diese im Anschluß an die Reaktion von (VI) mit (V) mit dem Fachmann bekannten Methoden wieder entfernt. Als Schutzgruppe können alle dem Fachmann bekannten Schutzgruppen verwendet werden, vorzugsweise Trimethylsilylethoxymethyl-. Zur Durchführung der Palladium katalysierten Kopplung eignen sich alle dem Fachmann bekannten Palladiumkomplexe, bevorzugt wird Pd(triphenylphosphin)₄ (Pd-tetrakis-Katalysator) verwendet, das vorzugsweise in situ aus Palladiumacetat gebildet wird. Die Formel (Ia) entspricht der Formel (I) für Y2 = H sowie R¹ gleich unsubstituiertes oder zumindest monosubstituiertes Aryl oder Heteroaryl.
Prinzipiell sind alle Synthesereaktionen für Verbindungen gemäß der Formel (I) dem Fachmann bekannt und können folglich unter Standardbedingungen (gleich oder mit leichten Abwandlungen) wie in der Literatur beschrieben durchgeführt werden (siehe beispielsweise in Houben-Weyl, Methoden der organischen Chemie, Thieme-Verlag, Stuttgart oder Organic Reactions, John Wiley & sons, New York). Bezogen auf die Umstände des Einzelfalls, um Nebenreaktionen während der Herstellung für Verbindungen gemäß Formel (1) zu vermeiden, kann es notwendig oder vorteilhaft sein, funktionelle Gruppen zeitweise durch die Einführung von Schutzgruppen zu blockieren und sie später wieder zu entfernen. Gegebenenfalls können funktionelle Gruppen auch in Form von Precursor-Gruppen eingeführt werden, wobei die Letztgenannten in einem späteren Reaktionsschritt in die gewünschte funktionelle Gruppe umgewandelt werden. Solche Synthesestrategien, Schutzgruppen und Precursor-Gruppen, die für den Einzelfall geeignet sind, sind dem Fachmann bekannt. Sofern erforderlich, können die Verbindungen gemäß Formel (I) durch bekannte Aufarbeitungsmethoden gereinigt werden, zum Beispiel durch Umkristallisation oder Chromatographie. Die Ausgangsmaterialien zur Herstellung von Verbindungen gemäß Formel (I) sind entweder kommerziell erhältlich oder sie können gemäß bekannter Literaturverfahren hergestellt werden. Verbindungen beziehungsweise Zwischenverbindungen, die durch die oben beschriebenen Syntheseverfahren hergestellt werden, sind ein weiterer Gegenstand der vorliegenden Erfindung.

Gegenstand der vorliegenden Erfindung ist auch die Verwendung von Verbindungen gemäß der allgemeinen Formel (I) als Arzneimittel beziehungsweise Medikament. Hinsichtlich der Definitionen der Substituenten A, B, D, E, R¹ und R² (sowie der weiteren, über die vorgenannten Substituenten definierten Substituenten) werden auf die Ausführungen hinsichtlich der Verbindungen als solche verwiesen.

Die Verwendung von Verbindungen gemäß der allgemeinen Formel (I) als Arzneimittel, wobei einer, mehrere oder alle der vorgenannten Substituenten die oben aufgeführte bevorzugte, mehr bevorzugte, noch mehr bevorzugte, viel mehr bevorzugte, noch viel mehr bevorzugte, besonders bevorzugte oder ganz besonders bevorzugte Bedeutung haben, inklusive sämtlicher Kombinationen untereinander, ist ebenfalls Gegenstand der vorliegenden Erfindung.

Die Verbindungen der allgemeinen Formel (I) sind Kinaseinhibitoren und eignen sich folglich zur Behandlung von Krankheiten, die sich in Folge einer anormalen Aktivität von Kinasen ergeben können. Als anormale Kinaseaktivität kann beispielsweise diejenige von PI3K, AkT, GSK-3β und dergleichen erwähnt werden.

Insbesondere werden die erfindungsgemäßen Verbindungen zur Inhibierung der Kinase GSK-3β verwendet. Dieser Effekt ist insbesondere bei der Behandlung von Stoffwechselkränkheiten wie Typ-II-Diabetes oder neurodegenerativen Krankheiten wie der Alzheimer-Krankheit von Bedeutung.

Weiterhin haben die Verbindungen gemäß der allgemeinen Formel (I) einen inhibitorischen Effekt auf die Phosphorylierung des Tau-Proteins. Dieser Effekt ist insbesondere bei der Behandlung von neurodegenerativen Krankheiten wie der Alzheimer-Krankheit von Bedeutung.

Beispiele von Krankheiten, die mit den Verbindungen gemäß der vorliegenden Erfindung behandelt werden können, umfassen: neurodegenerative Erkrankungen, Schlaganfälle, Schädel- und Rückenmarksverletzungen und periphere Neuropathien, Obesitas, metabolische Erkrankungen, Typ-II-Diabetes, essentielle Hypertonie, atherosklerotische Herzkreislauferkrankungen, Stein-Leventhal-Syndrom, Syndrom X, Immundefekte oder Krebs. Die neuro-degenerative Erkrankungen sind vorzugsweise Alzheimer-Krankheit, Parkinson-Krankheit, frontoparietale Demenz, kortikobasale Degeneration und Pick-Krankheit.

Vorzugsweise werden die Verbindungen der vorliegenden Erfindung zur Behandlung von Stoffwechselkrankheiten, insbesondere von Typ-II-Diabetes eingesetzt.

In einer weiteren Ausführungsform der vorliegenden Erfindung werden die Verbindungen gemäß der allgemeinen Formel (I) vorzugsweise zur Behandlung von neurodegenerativen Erkrankungen, insbesondere von der Alzheimer-Krankheit eingesetzt.

In den vorstehenden Ausführungen umfasst der Begriff Behandlung auch die Prophylaxe, Therapie oder Heilung der vorgenannten Krankheiten.

Nachfolgend beziehen sich alle Verweise auf "Verbindung(en) gemäß Formel (I)" auf Verbindung(en) der Formel (I) wie vorstehend beschrieben, sowie ihre Salze, Solvate und physiologisch funktionellen Derivate wie hierin beschrieben.

Die Verbindungen gemäß Formel (I) können Tieren und Menschen, bevorzugt Säugetieren und Menschen, besonders bevorzugt Menschen, verabreicht werden. Die Verbindungen gemäß Formel (I) können dabei selbst als Arzneimittel, in Mischungen miteinander oder in Mischungen mit anderen Arzneimitteln oder in Form von pharmazeutischen Zusammensetzungen verabreicht werden. Folglich sind die Verwendung von Verbindungen gemäß Formel (I) zur Herstellung eines oder mehrerer Medikamente zur Prophylaxe und/oder Behandlung der vorgenannten Krankheiten, pharmazeutische Zusammensetzungen enthaltend eine wirksame Menge von mindestens einer Verbindung gemäß Formel (I) sowie pharmazeutische Zusammensetzungen enthaltend eine wirksame Menge von mindestens einer Verbindung gemäß Formel (I) zur Prophylaxe und/oder Behandlung der vorgenannten Krankheiten ebenfalls Gegenstand der vorliegenden Erfindung

Die Menge einer Verbindung gemäß Formel (I), die erforderlich ist, um den gewünschten biologischen Effekt zu erreichen, ist abhängig von einer Reihe von Faktoren, z.B. der gewählten spezifischen Verbindung, der beabsichtigten Verwendung, der Art der Verabreichung und dem klinischen Zustand des Patienten. Im Allgemeinen liegt die Tagesdosis im Bereich von 0,3 mg bis 100 mg (typischerweise von 3 mg bis 50 mg) pro Tag pro Kilogramm Körpergewicht, z.B. 3-10 mg/kg/Tag. Eine intravenöse Dosis kann z.B. im Bereich von 0,3 mg bis 1,0 mg/kg liegen, die geeigneterweise als Infusion von 10 ng bis 100 ng pro Kilogramm pro Minute verabreicht werden kann. Geeignete Infusionslösungen für diese Zwecke können z.B. von 0,1 ng bis 10 mg, typischerweise von 1 ng bis 10 mg pro Milliliter, enthalten. Einzeldosen können z.B. von 1 mg bis 10 g des Wirkstoffs enthalten. Somit können Ampullen für Injektionen beispielsweise von 1 mg bis 100 mg, und oral verabreichbare Einzeldosisformulierungen, wie zum Beispiel Tabletten oder Kapseln, können beispielsweise von 1,0 bis 1000 mg, typischerweise von 10 bis 600 mg enthalten. Im Falle pharmazeutisch verträglicher Salze beziehen sich die vorgenannten Gewichtsangaben auf das Gewicht der dem Salz zugrunde liegenden freien Verbindung. Zur Prophylaxe oder Therapie der oben genannten Zustände können die Verbindungen gemäß Formel (I) selbst als Verbindung verwendet werden, vorzugsweise liegen sie jedoch mit einem verträglichen Träger in Form einer pharmazeutischen Zusammensetzung vor. Der Träger muss natürlich verträglich sein, in dem Sinne, dass er mit den anderen Bestandteilen der Zusammensetzung kompatibel ist und nicht gesundheitsschädlich für den Patienten ist (physiologisch verträglich). Der Träger kann ein Feststoff oder eine Flüssigkeit oder beides sein und wird vorzugsweise mit der Verbindung als Einzeldosis formuliert, beispielsweise als Tablette, die von 0,05% bis 95 Gew.-% des Wirkstoffs enthalten kann. Weitere pharmazeutisch aktive Substanzen können ebenfalls vorhanden sein, einschließlich weiterer Verbindungen gemäß Formel (I). Die erfindungsgemäßen pharmazeutischen Zusammensetzungen können nach einer der bekannten pharmazeutischen Methoden hergestellt werden, die im Wesentlichen darin bestehen, dass die Bestandteile mit pharmakologisch verträglichen Träger- und/oder Hilfsstoffen gemischt werden.

Neben mindestens einer Verbindung gemäß Formel (I) sowie einem oder mehreren Trägerstoffen können die erfindungsgemäßen pharmazeutischen Zusammensetzungen auch Hilfsstoffe enthalten. Beispielsweise eignen sich als Hilfs- beziehungsweise Zusatzstoffe: Füllstoffe, Bindemittel, Gleitmittel, Netzmittel, Stabilisatoren, Emulgatoren, Dispergiermittel, Konservierungsmittel, Süßstoffe, Farbstoffe, Geschmacksstoffe, Aromastoffe, Verdickungsmittel, Verdünnungsmittel, Puffersubstanzen, Lösungsmittel, Lösungsvermittler, Mittel, mit denen eine Depotwirkung erzielt werden kann, Salze zur Veränderung des osmotischen Druckes, Beschichtungsmittel oder Antioxidantien.

Die erfindungsgemäßen pharmazeutischen Zusammensetzungen können beispielsweise in Form einer Pille, Tablette, beschichteten Tablette, Lutschtablette, Granulat, Kapsel, harten oder weichen Gelatinkapsel, wässrigen Lösung, alkoholischen Lösung, ölartigen Lösung, Sirup, Emulsion, Suspension, Zäpfchen, Pastille, Lösung zur Injektion oder Infusion, Salbe, Tinktur, Creme, Lotion, Puder, Spray, transdermalen therapeutischen Systems, Nasenspray, Aerosol, Aerosol-Mischung, Mikrokapsel, Implantat, Stab (rod) oder Pflaster vorliegen.

Erfindungsgemäße pharmazeutische Zusammensetzungen sind solche, die für orale, rektale, topische, perorale (z.B. sublinguale) und parenterale (z.B. subkutane, intramuskuläre, intradermale oder intravenöse) Verabreichung geeignet sind, wenngleich die geeignetste Verabreichungsweise in jedem Einzelfall von der Art und Schwere des zu behandelnden Zustandes und von der Art der jeweils verwendeten Verbindung gemäß Formel (I) abhängig ist. Auch dragierte Formulierungen und dragierte Retardformulierungen gehören in den Rahmen der Erfindung. Bevorzugt sind säure- und magensaftresistente Formulierungen. Geeignete magensaftresistente Beschichtungen umfassen Celluloseacetatphthalat, Polyvinylacetatphthalat, Hydroxypropylmethylcellulosephthalat und anionische Polymere von Methacrylsäure und Methacrylsäuremethylester.

Geeignete pharmazeutische Verbindungen für die orale Verabreichung können in separaten Einheiten vorliegen, wie zum Beispiel Kapseln, Oblatenkapseln, Lutschtabletten oder Tabletten, die jeweils eine bestimmte Menge der Verbindung gemäß Formel (I) enthalten; als Pulver (Gelatinekapseln oder Beutel) oder Granulate; als Lösung oder Suspension in einer wässrigen oder nicht-wässrigen Flüssigkeit; oder als eine Öl-in-Wasser- oder Wasserin-Öl-Emulsion. Diese Zusammensetzungen können, wie bereits erwähnt, nach jeder geeigneten pharmazeutischen Methode zubereitet werden, die einen Schritt umfasst, bei dem der Wirkstoff und der Träger (der aus einem oder mehreren zusätzlichen Bestandteilen bestehen kann) in Kontakt gebracht werden. Im allgemeinen werden die Zusammensetzungen durch gleichmäßiges und homogenes Vermischen des Wirkstoffs mit einem flüssigen und/oder feinverteilten festen Träger hergestellt, wonach das Produkt, falls erforderlich, geformt wird. So kann beispielsweise eine Tablette hergestellt werden, indem ein Pulver oder Granulat der Verbindung verpresst oder geformt wird, gegebenenfalls mit einem oder mehreren zusätzlichen Bestandteilen. Gepresste Tabletten können durch Tablettieren der Verbindung in frei fließender Form, wie beispielsweise einem Pulver oder Granulat, gegebenenfalls gemischt mit einem Bindemittel, Gleitmittel, inertem Verdünner und/oder einem (mehreren) oberflächenaktiven/dispergierenden Mittel in einer geeigneten Maschine hergestellt werden. Geformte Tabletten können durch Formen der pulverförmigen, mit einem inerten flüssigen Verdünnungsmittel befeuchteten Verbindung in einer geeigneten Maschine hergestellt werden. Als Verdünnungsmittel eignen sich beispielsweise Stärke, Cellulose, Saccharose, Laktose oder Kieselgel. Weiterhin können die erfindungsgemäßen pharmazeutischen Zusammensetzungen auch Substanzen enthalten, bei denen es sich nicht um Verdünnungsmittel handelt, beispielsweise eine oder mehrere Gleitmittel, wie Magnesiumstearat oder Talkum, einen Farbstoff, eine Beschichtung (Dragees) oder einen Lack.

Pharmazeutische Zusammensetzungen, die für eine perorale (sublinguale) Verabreichung geeignet sind, umfassen Lutschtabletten, die eine Verbindung gemäß Formel (I) mit einem Geschmacksstoff enthalten, üblicherweise Saccharose und Gummi arabicum oder Tragant, und Pastillen, die die Verbindung in einer inerten Basis wie Gelatine und Glycerin oder Saccharose und Gummi arabicum umfassen.

Geeignete pharmazeutische Zusammensetzungen für die parenterale Verabreichung umfassen vorzugsweise sterile wässrige Zubereitungen einer Verbindung gemäß Formel (I), die vorzugsweise isotonisch mit dem Blut des vorgesehenen Empfängers sind. Diese Zubereitungen werden vorzugsweise intravenös verabreicht, wenngleich die Verabreichung auch subkutan, intramuskulär oder intradermal als Injektion erfolgen kann. Diese Zubereitungen können vorzugsweise hergestellt werden, indem die Verbindung mit Wasser gemischt wird und die erhaltene Lösung steril und mit dem Blut isotonisch gemacht wird. Injizierbare erfindungsgemäße Zusammensetzungen enthalten im Allgemeinen von 0,1 bis 5 Gew.-% der aktiven Verbindung.

Bei den sterilen Zusammensetzungen zur parenteralen Verabreichung kann es sich vorzugsweise um wässrige oder nicht wässrige Lösungen, um Suspensionen oder Emulsionen handeln. Als Lösungsmittel bzw. Vehikel lassen sich Wasser, Propylenglykol, Polyethylenglykol und Pflanzenöle, insbesondere Olivenöl, organische Ester für die Injektion, beispielsweise Ölsäureethylester, oder andere geeignete organische Lösungsmittel verwenden. Diese Zusammensetzungen können auch Adjuvantien, insbesondere Netzmittel, Mittel zur Einstellung der Isotonizität, Emulgatoren, Dispersionsmittel und Stabilisatoren enthalten. Die Sterilisierung kann auf mehrere Arten erfolgen, beispielsweise durch eine aspetische Filtratrion, durch Einbringen von Sterilisierungsmitteln in die Zusammensetzung, durch Bestrahlen oder durch Erhitzen. Die Zusammensetzungen können auch in Form sterilen festen Zusammensetzungen hergestellt werden, die bei der Verwendung in sterilem Wasser oder einem anderen sterilen Injektionsmedium gelöst werden.

Geeignete pharmazeutische Zusammensetzungen für die rektale Verabreichung liegen vorzugsweise als Einzeldosis-Zäpfchen vor. Diese können hergestellt werden, indem man eine Verbindung gemäß Formel (I) mit einem oder mehreren herkömmlichen festen Trägern, beispielsweise Kakaobutter, mischt und das entstehende Gemisch in Form bringt.

Geeignete pharmazeutische Zusammensetzungen für die topische Anwendung auf der Haut liegen vorzugsweise als Salbe, Creme, Lotion, Paste, Spray, Aerosol oder Öl vor. Als Träger können Vaseline, Lanolin, Polyethylenglycole, Alkohole und Kombinationen von zwei oder mehreren dieser Substanzen verwendet werden. Der Wirkstoff ist im Allgemeinen in einer Konzentration von 0,1 bis 15 Gew.-% der Zusammensetzung vorhanden, beispielsweise von 0,5 bis 2%.

Auch eine transdermale Verabreichung ist möglich. Geeignete pharmazeutische Zusammensetzungen für transdermale Anwendungen können als einzelne Pflaster vorliegen, die für einen langzeitigen engen Kontakt mit der Epidermis des Patienten geeignet sind. Solche Pflaster enthalten geeigneterweise den Wirkstoff in einer gegebenenfalls gepufferten wässrigen Lösung, gelöst und/oder dispergiert in einem Haftmittel oder dispergiert in einem Polymer. Eine geeignete Wirkstoff Konzentration beträgt ca. 1% bis 35%, vorzugsweise ca. 3% bis 15%. Als eine besondere Möglichkeit kann der Wirkstoff, wie beispielsweise in Pharmaceutical Research, 2(6): 318 (1986) beschrieben, durch Elektrotransport oder Iontophorese freigesetzt werden.

Die erfindungsgemäßen pharmazeutischen Zusammensetzungen werden durch die folgenden Beispiele erläutert:

### BEISPIEL A

Gelatinekapseln mit einer Dosis von 50 mg Wirkstoff und der folgenden Zusammensetzung werden auf herkömmliche Weise hergestellt:
- Verbindung der Formel (I) 50 mg
- Cellulose 18 mg
- Laktose 55 mg
- Kolloidales Kieselgel 1 mg
- Natriumcarboxymethylstärke 10 mg
- Talkum 10 mg
- Magnesiumstearat 1 mg

### BEISPIEL B

Gelatinekapseln (Tablette) mit einer Dosis von 50 mg Wirkstoff und der folgenden Zusammensetzung werden auf herkömmliche Weise hergestellt:
- Verbindung der Formel (I) 50 mg
- Laktose 104 mg
- Cellulose 40 mg
- Polyvidon 10 mg
- Natriumcarboxymethylstärke 22 mg
- Talkum 10 mg
- Magnesiumstearat 2 mg
- Kolloidales Kieselgel 2 mg
- Mischung von Hydroxymethylcellulose, Glycerin, Titanoxid (72-3,5-24,5) qs 1 fertige, filmbeschichtete 245-mg-Tablette

### BEISPIEL C

Eine Injektionslösung mit 10 mg Wirkstoff und der folgenden Zusammensetzung wird hergestellt:
- Verbindung der Formel (I) 10 mg
- Benzoesäure 80 mg
- Benzylalkohol 0,06 ml
- Natriumbenzoat 80 mg
- 95%iges Ethanol 0,4 ml
- Natriumhydroxid 24 mg
- Propylenglykol 1,6 ml
- Wasser qs 4 ml

Ein weiterer Gegenstand der vorliegenden Erfindung ist die Kombination von Verbindungen der Formel (I) mit weiteren pharmazeutisch wirksamen Substanzen, die nicht von Formel (I) erfasst werden.

Die Verbindungen der Formel (I) zeichnen sich durch günstige Wirkungen auf den Fettstoffwechsel aus, insbesondere sind sie zur Gewichtsreduktion und nach erfolgter Gewichtsreduktion zum Erhalt eines reduzierten Gewichtes bei Säugetieren und als Anorektika geeignet. Die Verbindungen zeichnen sich durch ihre geringe Toxizität und ihre geringen Nebenwirkungen aus.

Die Verbindungen können allein oder in Kombination mit weiteren gewichtsreduzierenden oder anorektischen Wirkstoffen eingesetzt werden. Solche weiteren anorektischen Wirkstoffe werden z.B. in der Roten Liste, Kapitel 01 unter Abmagerungsmittel/Appetitzügler genannt und können auch solche Wirkstoffe beinhalten, die den Energieumsatz des Organismus erhöhen und damit zu einer Gewichtsreduktion führen oder auch solche, welche den allgemeinen Metabolismus des Organismus so beeinflussen, dass eine erhöhte Kalorienzufuhr nicht zu einer Vergrößerung der Fettdepots und eine normale Kalorienzufuhr zu einer Verringerung der Fettdepots des Organismus führt. Die Verbindungen eignen sich zur Prophylaxe sowie insbesondere zur Behandlung von Übergewicht oder Obesitas.

Die Verbindungen der Formel (I) zeichnen sich durch günstige Wirkungen auf den Glucosestoffwechsel aus, sie senken insbesondere den Blutzuckerspiegel und sind zur Behandlung von Typ I und Typ II Diabetes geeignet. Die Verbindungen können daher allein oder in Kombination mit weiteren Blutzucker-senkenden Wirkstoffen (Antidiabetika) eingesetzt werden. Bei einem weiteren Aspekt der Erfindung können die Verbindungen der Formel I somit in Kombination mit einer oder mehreren weiteren pharmakologisch wirksamen Substanzen verabreicht werden, die beispielsweise ausgewählt sind aus Antidiabetika, Antiadiposita, blutdrucksenkenden Wirkstoffen, Lipidsenkern und Wirkstoffen zur Behandlung und/oder Prävention von Komplikationen, die von Diabetes verursacht werden oder mit Diabetes assoziiert sind.

Geeignete Antidiabetika umfassen Insuline, Amylin, GLP-1- und GLP-2-Derivate wie z.B. diejenigen die in WO 98/08871 von Novo Nordisk A/S offenbart wurden, sowie oral wirksame hypoglykämische Wirkstoffe.

Die oral wirksamen hypoglykämischen Wirkstoffe umfassen vorzugsweise Sulphonylharnstoffe, Biguanide, Meglitinide, Oxadiazolidindione, Thiazolidindione, Glukosidase-Inhibitoren, Glukagon-Rezeptor-Antagonisten, GLP-1-Agonisten, Kaliumkanalöffner wie z.B. diejenigen, die in WO 97/26265 und WO 99/03861 von Novo Nordisk A/S offenbart wurden, Insulin-Sensitizer, Aktivatoren der Insulin Rezeptor Kinase, Inhibitoren von Leberenzymen, die an der Stimulation der Glukoneogenese und/oder Glykogenolyse beteiligt sind, z.B. Inhibitoren der Glycogenphosphorylase, Modulatoren der Glukoseaufnahme und Glukoseausscheidung, den Fettstoffwechsel verändernde Verbindungen wie antihyperlipidämische Wirkstoffe und antilipidämische Wirkstoffe, z.B. HMGCoA-Reduktase-Inhibitoren, Inhibitoren des Cholesteroltransports/der Cholesterolaufnahme, Inhibitoren der Gallensäurerückresorption oder Inhibitoren des mikrosomalen Triglycerid-Transfer Proteins (MTP), Verbindungen, die die Nahrungsmitteleinnahme verringern, PPAR- und RXR-Agonisten und Wirkstoffe, die auf den ATP-abhängigen Kaliumkanal der Betazellen wirken.

Bei einer Ausführungsform der Erfindung werden die vorliegenden Verbindungen in Kombination mit Insulin verabreicht.

Bei einer weiteren Ausführungsform werden die vorliegenden Verbindungen in Kombination mit einem Sulphonylharnstoff wie z.B. Tolbutamid, Glibenclamid, Glimepirid, Glipizid, Gliquidon, Glisoxepid, Glibornurid oder Gliclazid verabreicht. Bei einer anderen Ausführungsform werden die vorliegenden Verbindungen in Kombination mit einem Biguanid wie z.B. Metformin verabreicht. Bei wieder einer anderen Ausführungsform werden die vorliegenden Verbindungen in Kombination mit einem Meglitinid wie z.B. Repaglinid verabreicht. Bei noch einer weiteren Ausführungsform werden die vorliegenden Verbindungen in Kombination mit einem. Thiazolidindion wie z.B. Troglitazon, Ciglitazon, Pioglitazon, Rosiglitazon oder den in WO 97/41097 von Dr. Reddy's Research Foundation offenbarten Verbindungen, insbesondere 5-[[4-[(3,4-Dihydro-3-methyl-4-oxo-2-chinazolinylmethoxy]phenyl]methyl]-2,4-thiazolidindion, verabreicht.

Bei einer weiteren Ausführungsform werden die vorliegenden Verbindungen in Kombination mit einem α-Glukosidase-Inhibitor wie z.B. Miglitol oder Acarbose verabreicht.

Bei einer anderen Ausführungsform werden die vorliegenden Verbindungen in Kombination mit einem Wirkstoff verabreicht, der auf den ATP-abhängigen Kaliumkanal der Betazellen wirkt, wie z.B. Tolbutamid, Glibenclamid, Glimepirid, Glipizid, Gliclazid oder Repaglinid. Bei noch einer anderen Ausführungsform werden die vorliegenden Verbindungen in Kombination mit einem antihyperlipidämischen Wirkstoff oder einem antilipidämischen Wirkstoff wie z.B. Cholestyramin, Colestipol, Clofibrat, Fenofibrat, Gemfibrozil, Lovastatin, Pravastatin, Simvastatin, Atorvastatin, Cerivastatin, Fluvastatin, Probucol, Ezetimibe oder Dextrothyroxin verabreicht.

Bei einer weiteren Ausführungsform werden die vorliegenden Verbindungen in Kombination mit mehr als einer der vorstehend genannten Verbindungen, z.B. in Kombination mit einem Sulphonylharnstoff und Metformin, einem Sulphonylharnstoff und Acarbose, Repaglinid und Metformin, Insulin und einem Sulphonylharnstoff, Insulin und Metformin, Insulin und Troglitazon, Insulin und Lovastatin, etc. verabreicht.

Weiterhin können die erfindungsgemäßen Verbindungen in Kombination mit einem oder mehreren Antiadiposita oder appetitregulierenden Wirkstoffen verabreicht werden.

Solche Wirkstoffe können ausgewählt werden aus der Gruppe bestehend aus CART-Agonisten, NPY-Antagonisten, MC4-Agonisten, Orexin-Antagonisten, H3-Agonisten, TNF-Agonisten, CRF-Agonisten, CRF BP-Antagonisten, Urocortin-Agonisten, β3-Agonisten, MSH (Melanocyt-stimulierendes Hormon)-Agonisten, CCK-Agonisten, Serotonin-Wiederaufnahme-Inhibitoren, gemischte Serotonin- und Noradrenalin-Wiederaufnahme-Inhibitoren, 5HT-Modulatoren, MAO-Hemmer, Bombesin-Agonisten, Galanin-Antagonisten, Wachstumshormon, Wachstumshormon freisetzende Verbindungen, TRH-Agonisten, Modulatoren der Entkopplungsproteine 2 oder 3, Leptin-Agonisten, Dopamin-Agonisten (Bromocriptin, Doprexin), Lipase/Amylase-Inhibitoren, Antagonisten des Cannabinoid Rezeptors 1, Modulatoren des die Acylierung stimulierende Protein (ASP), PPAR-Modulatoren, RXR-Modulatoren, hCNTF-Mimetika oder TR-β-Agonisten.

Bei einer Ausführungsform der Erfindung ist das Antiadipositum Leptin oder modifiziertes Leptin. Bei einer anderen Ausführungsform ist das Antiadipositum Dexamphetamin oder Amphetamin. Bei einer anderen Ausführungsform ist das Antiadipositum Fenfluramin oder Dexfenfluramin. Bei noch einer anderen Ausführungsform ist das Antiadipositum Sibutramin oder die mono- und bisdemethylierten Wirkmetabolite von Sibutramin. Bei einer weiteren Ausführungsform ist das Antiadipositum Orlistat. Bei einer anderen Ausführungsform ist das Antiadipositum Mazindol, Diethylpropion oder Phentermin.

Weiterhin können die vorliegenden Verbindungen in Kombination mit einem oder mehreren antihypertensiven Wirkstoffen verabreicht werden. Beispiele für antihypertensive Wirkstoffe sind Betablocker wie Alprenolol, Atenol, Timolol, Pindolol, Propranolol und Metoprolol, ACE (Angiotensin Converting Enzym)-Hemmer wie z.B. Benazepril, Captopril, Enalapril, Fosinopril, Lisinopril, Quinapril und Ramipril, Calciumkanal-Blocker wie Nifedipin, Felodipin, Nicardipin, Isradipin, Nimodipin, Diltiazem und Verapamil, sowie Alphablocker wie Doxazosin, Urapidil, Prazosin und Terazosin. Weiterhin kann verwiesen werden auf Remington: The Science and Practice of Pharmacy, 19. Auflage, Gennaro, Hrsg., Mack Publishing Co., Easton, PA, 1995.

Es versteht sich, dass jede geeignete Kombination der erfindungsgemäßen Verbindungen mit einer oder mehreren der vorstehend genannten Verbindungen und wahlweise einer oder mehreren weiteren pharmakologisch wirksamen Substanzen als unter den Schutzbereich der vorliegenden Erfindung fallend angesehen wird.

Die nachfolgend aufgeführten Beispiele dienen zur Erläuterung der Erfindung.

### Beispiel 1

### 2-(3-Oxo-6-pyridin-4-yl-2,3-dihydro-pyridazin-4-yl)-1H-benzimidazol-5-carbonsäuremethylester

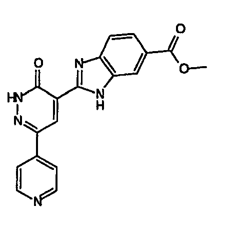

### a) 4-Amino-3-[(3-oxo-6-pyridin-4-yl-2,3-dihydro-pyridazin-4-carbonyl)-amino]-benzoesäure-methylester

Die Mischung bestehend aus 2,1 g 3-Oxo-6-pyridin-4-yl-2,3-dihydro-pyridazin-4-carbonsäure, 4 ml Thionylchlorid und 20 ml Dimethoxyethan wird 5 Stunden bei 100°C gerührt und anschließend im Vakuum zur Trockne eingeengt. Der Rückstand wird in 20 ml Dimethoxyethan aufgeschlämmt, mit 3 g Triethylamin und 1,7 g 3,4-Diaminobenzoesäuremethylester versetzt und über Nacht bei Raumtemperatur gerührt. Die flüchtigen Anteile werden im Vakuum abgezogen und der Rückstand mit 10 ml gesättigter Natriumbicarbonatlösung verrührt und abgesaugt.
Ausbeute: 1,3 g Fp.: 352°C

### b) 2-(3-Oxo-6-pyridin-4-yl-2,3-dihydro-pyridazin-4-yl)-1H-benzimidazol-5-carbonsäure-methylester

Die Mischung von 1,3 g 4-Amino-3-[(3-oxo-6-pyridin-4-yl-2,3-dihydro-pyridazin-4-carbonyl)-amino]-benzoesäure-methylester und 20 ml Eisessig wird unter Umrühren 10 Stunden auf 100°C erhitzt. Der gebildete Niederschlag wird abgesaugt, mit Wasser gewaschen und bei 50°C im Vakuum getrocknet.
Ausbeute: 1,07 g Fp.: >300°C (Zers.)

### Beispiel 2

### 2-(3-Oxo-6-pyridin-4-yl-2,3-dihydro-pyridazin4-yl)-1H-benzimidazol-5-carbonsäure

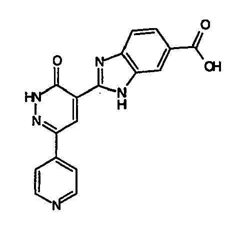

Die Mischung bestehend aus 500 mg 4-Amino-3-[(3-oxo-6-pyridin-4-yl-2,3-dihydropyridazin-4-carbonyl)-amino]-benzoesäure-methylester, 6 ml Tetrahydrofuran (THF), 6 ml Methanol, 6 ml Wasser und 173 mg Lithiumhydroxid wird 5 Stunden bei 50°C gerührt. Nach dem Abkühlen auf Raumtemperatur wird mit 1N HCl ein pH = 4-5 eingestellt, wobei ein Niederschlag ausfällt, der nach dem Absaugen mit Wasser gewaschen und im Vakuum getrocknet wird.
Ausbeute: 380 mg Fp.: >300°C

### Beispiel 3

### 2-(3-Oxo-6-pyridin-4-yl-2,3-dihydro-pyridazin-4-yl)-1H-benzimidazol-5-carbonsäure-(2-diethylamino-ethyl)-amid

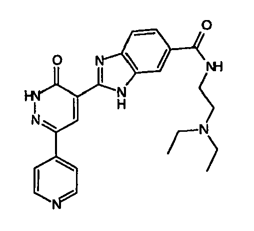

Die Mischung bestehend aus 50 mg 2-(3-Oxo-6-pyridin-4-yl-2,3-dihydro-pyridazin-4-yl)-1H-benzimidazol-5-carbonsäure, 0,065 ml Triethylamin und 1,5 ml Dimethylformamid (DMF) wird 10 Minuten bei Raumtemperatur gerührt, mit 68,4 mg O-(7-azabenzotriazol-1-yl)-N,N,N',N'-tetramethyluronium hexafluorphosphat (Hatu) versetzt und weitere 30 Minuten bei Raumtemperatur gerührt. Dann werden 21 mg Diethylaminoethylamin zugefügt und die Mischung 3 Stunden bei 50°C gerührt. Nach dem Erkalten wird mit 5 ml Wasser verdünnt, der Niederschlag abgesaugt und mit Isopropanol bei 60°C verrührt, abgesaugt und im Vakuum getrocknet.
Ausbeute: 53 mg Fp.: 263°C

### Beispiel 4

### 4-(5-Chlor-1H-benzimidazol-2-yl)-6-(2-ethylamino-pyrimidin-4-yl)-2H-pyridazin-3-on

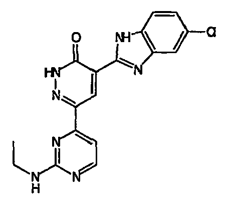

### a) 1-(2-Ethylamino-pyrimidin-4-yl)-ethanon

Die Mischung von 6 g 1-Dimethylami.no-4,4-dimethoxy-pent-1-en-3-one, 3,96 g N-Ethylguanidinhydrochlorid und 26 ml 20%ige ethanolische Natriumethylatlösung wird 2 Stunden am Rückfluss erhitzt. Nach dem Erkalten wird der Feststoff abgesaugt, das Filtrat im Vakuum eingeengt und mit 20 ml Trifluoressigsäure und 2 ml Wasser versetzt und über Nacht bei Raumtemperatur gerührt. Dann wird mit 50 ml Wasser versetzt, mit Soda ein pH = 10 eingestellt und mit zwei mal je 25 ml Essigester ausgeschüttelt. Die organische Phase wird über Natriumsulfat getrocknet und eingeengt. Der erhaltene ölige Rückstand wird säulenchromatografisch (Kieselgel, LM: Methylenchlorid : Methanol = 98 : 2) gereinigt.
Ausbeute: 1,9 g Fp.: 70,9°C

### b) 2-[2-(2-Ethylamino-pyrimidin-4-yl)-2-oxo-ethyl]-2-hydroxy-malonsäure-diethylester

Die Mischung bestehend aus 1,9 g 1-(2-Ethylamino-pyrimidin-4-yl)-ethanon und 1,86 ml Diethylketomalonat wird 18 Stunden auf 110°C erhitzt. Das Gemisch wird säulenchromatografisch (Kieselgel, LM: Methylenchlorid : Methanol = 98 : 2) gereinigt.
Ausbeute: 2g Fp.: Harz

### c) 6-(2-Ethylamino-pyrimidin-4-yl)-3-oxo-2,3-dihydro-pyridazin-4-carbonsäureethylester

Die Mischung von 2 g 2-[2-(2-Ethylamino-pyrimidin-4-yl)-2-oxo-ethyl]-2-hydroxymalonsäure-diethylester, 485 mg Hydrazinhydrochlorid und 20 ml Ethanol wird 24 Stunden am Rückfluss gerührt. Nach dem Kaltrühren wird der Niederschlag abgesaugt, in 4 ml N-Methylpyrrolidinon (NMP) 3 Stunden auf 130°C erhitzt und nach dem Erkalten mit 15 ml nHeptan versetzt und verrührt. Der Niederschlag wird dann abgesaugt und mit Methylenchlorid verrührt, erneut abgesaugt und getrocknet.
Ausbeute: 660 mg Fp.: 234°C

### d) 6-(2-Ethylamino-pyrimidin-4-yl)-3-oxo-2,3-dibydro-pyridazin-4-carbonsäure

Die Mischung von 400 mg 6-(2-Ethylamino-pyrimidin-4-yl)-3-oxo-2,3-dihydro-pyridazin-4-carbonsäure-ethylester, 2 ml THF, 2 ml Wasser, 2 ml Methanol und 100 mg Lithiumhydroxid wird 1 Stunde bei Raumtemperatur gerührt und die flüchtigen Anteile im Vakuum entfernt. Durch Zutropfen von 2 N Salzsäure wird ein pH = 4 eingestellt und der gebildete Niederschlag abgesaugt, mit 10 ml Isopropanol verrührt und abgesaugt und getrocknet.
Ausbeute: 200 mg Fp.: 322°C

### e) 6-(2-Ethylamino-pyrimidin-4-yl)-3-oxo-2,3-dihydro-pyridazin-4-carbonsäure-(2-amino-4-chlor-phenyl)-amid

Die Lösung von 110 mg 6-(2-Ethylamino-pyrimidin-4-yl)-3-oxo-2,3-dihydro-pyridazin-4-carbonsäure, 2 ml DMF und 0,17 ml Triethylamin wird mit 192 mg Hatu versetzt und 30 Minuten bei Raumtemperatur gerührt. Dann werden 66 mg 4-Chlor-Phenylendiamin zugesetzt und über Nacht bei Raumtemperatur gerührt. Die Mischung wird mit 5 ml Wasser versetzt und der Niederschlag nach kurzem Verrühren abgesaugt und getrocknet.
Ausbeute: 57 mg Fp.: >300°C (Zers.)

### f) 4-(5-Chlor-1H-benzimidazol-2-yl)-6-(2-ethylamino-pyrimidin-4-yl)-2H-pyridazin-3-on

6-(2-Ethylamino-pyrimidin-4-yl)-3-oxo-2,3-dihydro-pyridazin-4-carbonsäure-(2-amino-4-chlor-phenyl)-amid (50 mg) werden in 1 ml Eisessig 3 Stunden bei 100°C gerührt. Nach dem Erkalten wird der Niederschlag abgesaugt, mit wässriger Natriumbicarbonatlösung verrührt und erneut abgesaugt, mit Wasser gewaschen und im Vakuum getrocknet.
Ausbeute: 15 mg Fp. >300°C (Zers.)

### Beispiel 5

### 4-(6-Chlor-1H-benzimidazol-2-yl)-6-pyridin-4-yl-2H-pyridazin-3-on

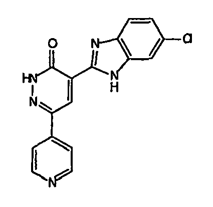

Beispiel 5 wird entsprechend Beispiel 1 hergestellt.
Fp. >300°C (Zers.)

### Beispiel 6

### 4-(6-Trifluormethyl-1H-benzimidazol-2-yl)-6-pyridin-4-yl-2H-pyridazin-3-on

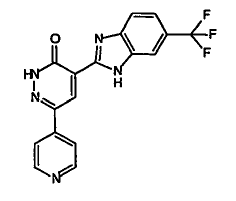

Fp. >300°C (Zers.)
Beispiel 6 wird entsprechend Beispiel 1 hergestellt.

### Beispiel 7

### 4-(6-Methoxy-1H-benzimidazol-2-yl)-6-pyridin-4-yl-2H-pyridazin-3-on

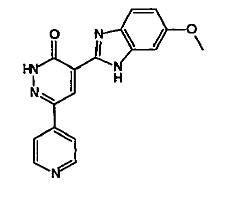

Fp. >300°C (Zers.)
Beispiel 7 wird entsprechend Beispiel 1 hergestellt.

### Beispiel 8

### 2-(3-Oxo-6-pyridin-4-yl-2,3-dihydro-pyridazin-4-yl)-1H-benzimidazol-5-carbonsäure-(2-cyclohexylamino-ethyl)-amid

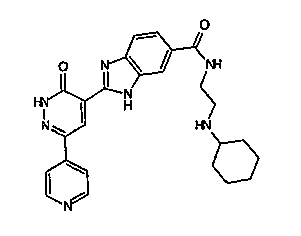

Fp. 271°C
Beispiel 8 wird entsprechend Beispiel 3 hergestellt.

### Beispiel 9

### 2-(3-Oxo-6-pyridin-4-yl-2,3-dihydro-pyridazin-4-yl)-1H-benzimidazol-5-carbonsäure-[3-(4-methyl-piperazin-1-yl)-propyl]-amid

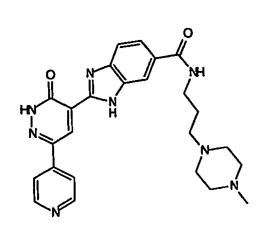

Fp. >30°C (Zers.)
Beispiel 9 wird entsprechend Beispiel 3 hergestellt.

### Beispiel 10

### 2-(3-Oxo-6-pyridin-4-yl-2,3-dihydro-pyridazin-4-yl)-1H-benzimidazol-5-earbonsäure-(3-hydroxy-propyl)-amid

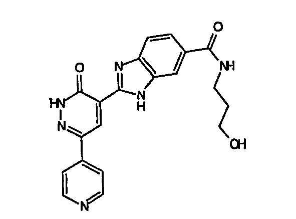

Fp. >300°C (Zers.)
Beispiel 10 wird entsprechend Beispiel 3 hergestellt.

### Beispiel 11

### 4-(5-Chlor-1H-benzimidazol-2-yl)-6-methyl-2H-pyridazin-3-on

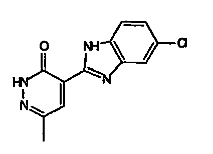

Fp. >300°C (Zers.)
Beispiel 11 wird entsprechend Beispiel 1 hergestellt.

### Beispiel 12

### 2-(3-Oxo-6-pyridin-4-yt-2,3-dihydro-pyridazm-4-yl)-1H-benzimidazol-5-carbonsäure-(3-cyclohexylamino-propyl)-amid

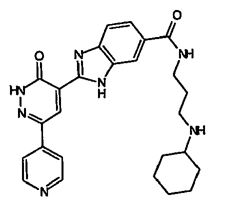

Fp.: Harz
Beispiel 12 wird entsprechend Beispiel 3 hergestellt.

### Beispiel 13

### 2-(3-Oxo-6-pyridin-4-yl-2,3-dihydro-pyridazin-4-yl)-1H-benzimidazol-5-carbonsäure-(3-imidazol-1-yl-propyl)-amid

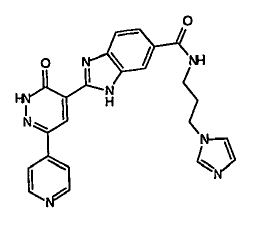

Fp.: Harz
Beispiel 13 wird entsprechend Beispiel 3 hergestellt.

### Beispiel 14

### 4-(5-Chlor-1H-benzimidazol-2-yl)-6-(2-methylamino-pyrimidin-4-yl)-2H-pyridazin-3-on

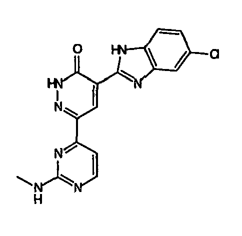

Fp.: >300°C (Zers.)
Beispiel 14 wird entsprechend Beispiel 4 hergestellt.

### Beispiel 15

### 4-(6-Chlor-1H-benzimidazol-2-yl)-6-(4-hydroary-3,5-dimethyl-phenyl)-2H-pyridazin-3-on

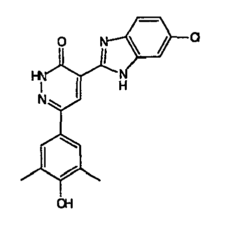

### a) Mischung von 6-Chlor-3-oxo-2,3-dihydro-pyridazin-4-carbonsäure-(2-amino-5-chlor-phenyl)-amid und 6-Chlor-3-oxo-2,3-dihydro-pyridazin-4-carbonsäure-(2-amino-4-chlor-phenyl)-amid

6-Chlor-3-oxo-2,3-dihydro-pyridazin-4-carbonsäure (5g; 28.6mmol) wird in der Mischung aus Tetrahydrofuran (250ml) und DMF (1ml) gelöst, im Eisbad auf 8°C gekühlt und tropfenweise mit Oxalylchlorid (19.42g; 153mmol) versetzt. Die Mischung wird für 2h bei RT gerührt und die Lösemittel im Vakuum abgezogen. Dann wird in THF gelöst und die flüchtigen Anteile erneut im Vakuum bei RT abgezogen. Der Rückstand wird in Tetrahydrofuran/DMF gelöst und 4-Chlor-phenylendiamin (4.08g; 28.6mmol) und Pottasche (7.92g; 57.3mmol) werden zugefügt. Nach 16 stündigem Rühren bei RT werden die flüchtigen Anteile im Vakuum entfernt, der Rückstand in Wasser aufgenommen und die Lösung mit 2N Salzsäure auf pH 2 gestellt. Der Niederschlag wird abgesaugt und das Produkt durch Säulenchromatographie (Kieselgel, Essigester/nHeptan, Gradient: 0-80%) gereinigt.
Ausbeute: 1.0g.

### b) 6-Chlor-4-(6-chlor-1H-benzimidazol-2-yl)-2H-pyridazin-3-on

Die Mischung aus 6-Chlor-3-oxo-2,3-dihydro-pyridazin-4-carbonsäure-(2-amino-5-chlorphenyl)-amid und 6-Chlor-3-oxo-2,3-dihydro-pyridazin-4-carbonsäure-(2-amino-4-chlor-phenyl)-amid (1.0g; 1.67mmol) wird in 100 ml Eisessig gelöst und für 90 Min. auf 120°C erhitzt. Beim Abkühlen fällt ein Niederschlag aus, der abgesaugt und bei 40°C im Vakuum getrocknet wird.
Ausbeute: 315mg.

### c) 6-Chlor-4-[6-chlor-1-(2-trimethylsilanyl-ethoxymethyl)-1H-benzimidazol-2-yl]-2-(2-trimethylsilanyl-ethoxymethyl)-2H-pyridazin-3-on

6-Chlor-4-(6-chlor-1H-benzimidazol-2-yl)-2H-pyridazin-3-on (315mg; 1.12mmol) wird in DMF (8.3m1) gelöst, Cäsiumcarbonate (1.1g; 3.36mmol) und (2-chloromethoxi-ethyl)-trimethyl-silan (467mg; 2.8mmol) werden zugefügt und die Mischung wird bei 60°C für 2h gerührt, abgekühlt, filtriert und das Produkt wird säulenchromatografisch gereinigt (RP-HPLC ,Gradient of 0-100% Acetonitril in Wasser (+0.01% Trifluoressigsäure)).
Ausbeute: 513mg.

### d) 4-[6-Chlor-1-(2-trimethylsilanyl-ethoxymethyl)-1H-benzimidazol-2-yl]-6-(4-hydroxi-3,5- dimethyl-phenyl)-2-(2-trimethylsilanyl-ethoxymethyl)-2H-pyridazin-3-on

6-Chlor-4-(6-chlor-1-(2-trimethylsilanyl-ethoxymethyl)-1H-benzimidazol-2-yl]-2-(2-trimethylsilanyl-ethoxymethyl)-2H-pyridazin-3-on (100mg; 0.185mmol) und Tetrakis-(triphenylphosphin) Palladium (0) (0.15 Äquivalente) werden in DME gelöst und mit Argon 10 Min. begast. 2,6-Dimethyl-4-(4,4,5,5-tetramethyl-1,3,2-dioxaborolan-2-yl)phenol (1 Äquivalent) und 2M wässrige Sodalösung (2 Äquivalente) werden zugefügt und die Mischung 5 Stunden auf 95°C erhitzt. Die flüchtigen Anteile werden im Vakuum entfernt, der Rückstand in DMF aufgenommen und das Produkt wird säulenchromatographisch gereinigt (RP-HPLC, Gradient of 0-100% Acetonitril in Wasser (+0.01% Trifluoressigsäure)).
Ausbeute: 64 mg

### e) 4-(6-Chlor-1H-benzmüdazol-2-yl)-6-(4-hydroxi-3,5-dimethyl-phenyl)-2H-pyridazin-3-on

4-[6-Chlor-1-(2-trimethylsilanyl-ethoxymethyl)-1H-benzimidazol-2-yl]-6-(4-hydroxi-3,5-dimethyl-phenyl)-2-(2-trimethylsilanyl-etlioxymethyl)-2H-pyridazin-3-on wird in Dichloromethan:Trifluoressigsäure/1:1 30 Min. bei RT gerührt. Das Lösemittel wird im Vakuum abgezogen und der Rückstand in Methanol gelöst und mit 2M Natronlauge versetzt. Die Lösung wird 30 Min. bei RT gerührt. Nach beendeter Rektion wird Wasser zugefügt und mit 2N Salzsäure angesäuert. Das ausgefallene Produkt wird abgesaugt und säulenchromatographisch gereinigt (RP-HPLC , Gradient of 0-100% Acetonitril in Wasser (+0.01% Trifluoressigsäure))..
Ausbeute: 12.5 mg. MS (ES+) m/z 367 (M+H).

### Beispiel 16

### 4-(6-Chlor-1H-benzimidazol-2-yl)-6-(4-hydroxy-3-methoxy-phenyl)-2H-pyridazin-3-on

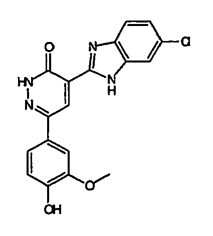

MS (ES+) m/z 369 (M+H).
Beispiel 16 wird entsprechend Beispiel 15 hergestellt.

### Beispiel 17

### 4-(7-Methyl-1H-benzimidazol-2-yl)-6-pyridin-4-yl-2H-pyridazin-3-on

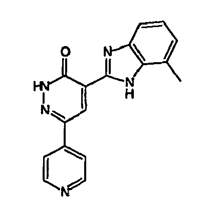

Fp.: >350°C (Zers.)
Beispiel 17 wird entsprechend Beispiel 1 hergestellt.

### Beispiel 18

### 4-(5,6-Dimethyl-1H-benzimidazol-2-yl)-6-pyridin-4-yl-2H-pyridazin-3-on

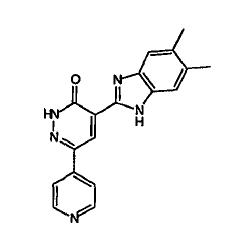

Fp.: >350°C (Zers.)
Beispiel 18 wird entsprechend Beispiel 1 hergestellt.

### Beispiel 19

### 4-[5-(4-Methyl-piperazin-1-yl)-1H-benzimidazol-2-yl]-6-pyridin-4-yl-2H-pyridazin-3-on

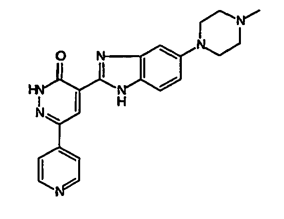

Fp.: >350°C (Zers.)
Beispiel 19 wird entsprechend Beispiel 1 hergestellt.

### Beispiel 20

### 4-(5-Fluor-1H-benzimidazol-2-yl)-6-pyridin-4-yl-2H-pyridazin-3-on

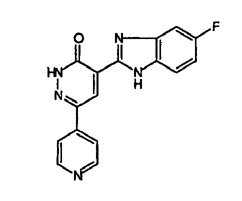

Fp.: >350°C (Zers.)
Beispiel 20 wird entsprechend Beispiel 1 hergestellt.

### Beispiel 21

### 4-(5-Cyano-1H-benzimidazol-2-yl)-6-pyridin-4-yl-2H-pyridazin-3-on

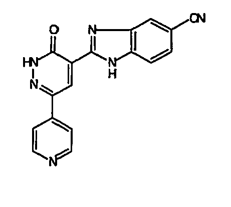

Fp.: >350°C (Zers.)
Beispiel 21 wird entsprechend Beispiel 1 hergestellt.

### Beispiel 22

### 4-(5-Brom-1H-benzimidazol-2-yl)-6-pyridin-4-yl-2H-pyridazin-3-on

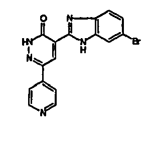

Fp.: >350°C (Zers.)
Beispiel 22 wird entsprechend Beispiel 1 hergestellt.

### Beispiel 23

### 6-Chlor-4-(3H-imidazo[4,5-c]pyridin-2-yl)-2H-pyridazin-3-on

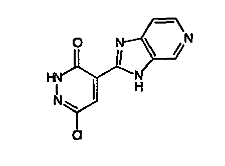

MS (ES+) m/z 248 (M+H).
Beispiel 23 wird entsprechend Beispiel 1 hergestellt.

### Beispiel 24

### 6-(4-Hydroxy-3-methoxy-phenyl)-4-(6-trifluormethyl-1H-benzimidazol-2-yl)-2H-pyridazin-3-on

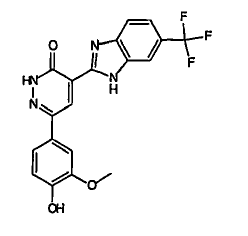

MS (ES+) m/z 403 (M+H).
Beispiel 24 wird entsprechend Beispiel 15 hergestellt.

### Beispiel 25

### 6-(4-Hydroxy-3,5-dimethyl-phenyl)-4-(6-trifluormethyl-1H-benzimidazol-2-yl)-2H-pyridazin-3-on

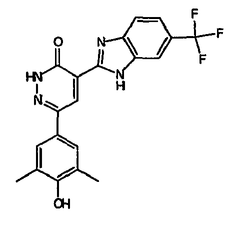

MS (ES+) m/z 401 (M+H).
Beispiel 25 wird entsprechend Beispiel 15 hergestellt.

### Beispiel 26

### 6-(2-Butylamino-pyrimidin-4-yl)-4-(6-chlor-1H-benzimidazol-2-yl)-2H-pyridazin-3-on

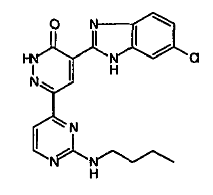

Fp.: 305°C
Beispiel 26 wird entsprechend Beispiel 4 hergestellt.

### Beispiel 27

### 6-(2-Butylamino-pyrimidin-4-yl)-4-(6-trifluormethyl-1H-benzimidazo1-2-yl)-2H-pyridazin-3-on

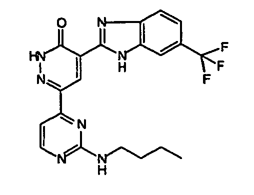

Fp.: 288°C
Beispiel 27 wird entsprechend Beispiel 4 hergestellt.

### Beispiel 28

### 4-(1H-Benzimidazol-2-yl)-6-pyridin-4-yl-2H-pyridazin-3-on

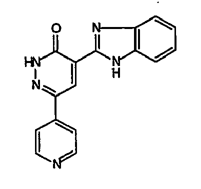

Fp.: >350°C (Zers.)
Beispiel 28 wird entsprechend Beispiel 1 hergestellt.

### Beispiel 29

### 4-(6-Chlor-1H-benzimidazol-2-yl)-6-[2-((R)-1-phenyl-ethylamino)-pyrimidin-4-yl]-2H-pyridazin-3-on

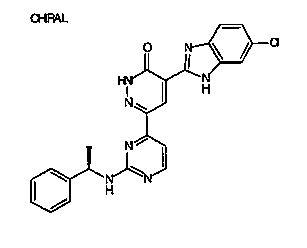

MS (ES+) m/z 444 (M+H)
Beispiel 29 wird entsprechend Beispiel 4 hergestellt.

### Beispiel 30

### 4-(5,6-Dichlor-1H-benzimidazol-2-yl)-6-pyridin-4-yl-2H-pyridazin-3-on; Verbindung mit Essigsäure

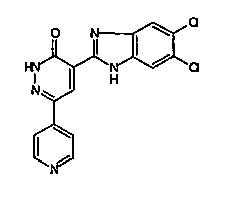

MS (ES+) m/z 358 (M+H)
Beispiel 30 wird entsprechend Beispiel 1 hergestellt.

### Beispiel 31

### 4-(6-Chlor-5-fluor-1H-benzimidazol-2-yl)-6-pyridin-4-yl-2H-pyridazin-3-on; Verbindung mit Essigsäure

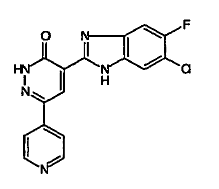

MS (ES+) m/z 342 (M+H)
Beispiel 31 wird entsprechend Beispiel 1 hergestellt.

### Beispiel 32

### 4-(6-Chlor-5-methyl-1H-benzimidazol-2-yl)-6-pyridin-4-yl-2H-pyridazin-3-on; Verbindung mit Essigsäure

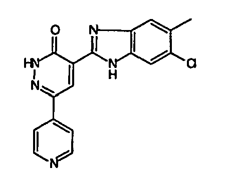

MS (ES+) m/z 338 (M+H)
Beispiel 32 wird entsprechend Beispiel 1 hergestellt.

### Beispiel 33

### 4-(5,7 -Difluor-1H-benzimidazol-2-yl)-6-pyridin-4-yl-2H-pyridazin-3-on; Verbindung mit Essigsäure

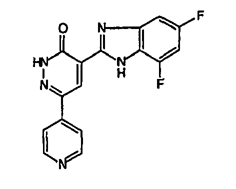

MS (ES+) m/z 326 (M+H)
Beispiel 33 wird entsprechend Beispiel 1 hergestellt.

### Beispiel 34

### 4-(5-Chlor-6-methyl-1H-benzimidazol-2-yl)-6-(4-hydroxy-3-methoxy-phenyl)-2H-pyridazin-3-on

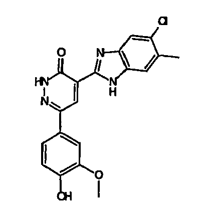

MS (ES+) m/z 383 (M+H)
Beispiel 34 wird entsprechend Beispiel 15 hergestellt.

### Beispiel 35

### 6-[2-(2-Morpholin-4-yl-ethylamino)-pyrimidin-4-yl]-4-(6-trifluormethyl-1H-benzimidazol-2-yl)-2H-pyridazin-3-on; Verbindung mit Trifluoressigsäure

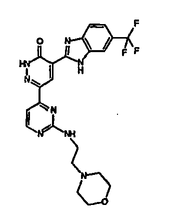

### a) 6-Oxo-5-[6-trifluormethyl-1-(2-trimethylsilanyl-ethoxymethyl)-1H-benzimidazol-2-yl]-1-(2-trimethylsilanyl-ethoxymethyl)-1,6-dihydro-pyridazine-3-boronsäure

6-Chlor-4-[6-trifluormethyl-1-(2-trimethylsilanyl-ethoxymethyl)-1H-benzimidazol-2- yl]-2-(2-trimethylsüanyl-ethoxymethyl)-2H-pyridazin-3-on (hergestellt analog zu Beispiel 15b), Bis(pinacolato)diboron, (1,1'-bis(diphenylphosphin)ferrocene)palladium(II)chloride-Dichlormethan-Komplex und Kaliumacetat werden in DMSO gelöst. Die Lösung wird mit Argon entgast und für 2.5 Stunden auf 95°C erhitzt. Die Reaktionslösung wird über Kieselgel filtriert und dieses mit Dichlormethan gewaschen. Das Lösungsmittel wird am Rotationsverdampfer entfernt und das Rohprodukt via RP-HPLC gereinigt (0-100 % Acetonitril in Wasser (+0.01 % Trifluoressigsäure)). MS (ES+) m/z 585 (M+H).

### b) 6-(2-Chlor-pyrimidin-4-yl)-4-[6-trifluormethyl-1-(2-trimethylsilanylethoxymethyl)-1H- benzimidazol-2-yl]-2-(2-trimethytsilanyl-ethoxymethyl)-2H-pyridazin-3-on

6-Oxo-5-[6-trifluormethyl-1-(2-trimethylsilanyl-ethoxymethyl)-1H-benzimidazol-2-yl]-1-(2-trimethylsilanyl-ethoxymethyl)-1,6-dihydro-pyridazine-3-boronsäure, 2,4-dichlorpyrimidin, Kaliumfluorid und Tris(dibenzylideneaceton)dipalladium(0) werden in trockenem THF gelöst und mit Argon während 10 Minuten entgast. Tri-tert-butylphosphonium Tetrafluorborat wird zugegeben und die Reaktionsmischung während 16 Stunden bei Raumtemperatur gerührt. LC-MS-Analyse zeigt vollständigen Umsatz. Das Lösungsmittel wird am Rotationsverdampfer entfernt, der Rückstand in DMF gelöst und das Rohprodukt mittels preparativer RP-HPLC gereinigt (0-100% Acetonitril in Wasser (+0.01 % Trifluoressigsäure)). MS (ES+) m/z 653 (M+H).

### c) 6-[2-(2-Morpholin-4-yl-ethylamino)-pyrimidin-4-yl]-4-[6-trifluormethyl-1-(2-trimethylsilanyl-ethoxymethyl)-1H-benzimidazol-2-yl]-2-(2-trimethylsilanylethoxymethyl)-2H-pyridazin-3-on

6-(2-Chlor-pyrimidin-4-yl)-4-[6-trifluormethyl-1-(2-trimethylsilanyl-ethoxymethyl)-1H-benzimidazol-2-yl]-2-(2-trimethylsilanyl-ethoxymethyl)-2H-pyridazin-3-on wird in N-(2-Aminoethyl)morpholin suspendiert und die Reaktionsmischung während 20 Minuten in der Mikrowelle auf 100°C erhitzt. Das Produkt wird über eine reversed-phase C18 Kieselgelkartusche filtriert und mit Wasser/Acetonitril eluiert. Das Produkt wird mittels preparativer RP-HPLC gereingt (0-100% Acetonitril in Wasser (+0.01 % Trifluoressigsäure)). MS (ES+) m/z 747 (M+H).

### d) 6-[2-(2-Morpholin-4-yl-ethylamino)-pyrimidin-4-yl]-4-(6-trifluormethyl-1H-benzimidazol-2-yl)-2H-pyridazin-3-on

6-[2-(2-Morpholin-4-yl-ethylamino)-pyrimidin-4-yl]-4-[6-trifluormethyl-1-(2-trimethylsilanyl-ethoxymethyl)-1H-benzimidazol-2-yl]-2-(2-trimethylsilanyl- ethoxymethyl)-2H-pyridazin-3-on wurde analog zu Beispiel 15 umgesetzt.
MS (ES+) m/z 487 (M+H). NMR (500MHz; DMSO-d6) 14.25 (s, 1H), 9.25 (s, 1H), 8.51 (d, 1H), 8.11 (s,1H), 7.95 (d, 1H), 7.61 (d, 1H), 7.35 (d, 1H), 4.02 (brs), 3.75 (brs), 3.40 (brs).

### Beispiel 36

### 4-(5,6-Dichlor-1H-benzimidazol-2-yl)-6-(4-hydroxy-3-methoxy-phenyl)-2H-pyridazin-3-on

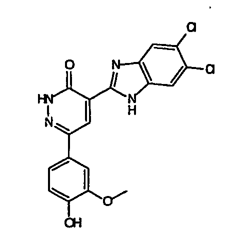

MS (ES+) m/z 403 (M+H)
Beispiel 36 wird entsprechend Beispiel 15 hergestellt.

### Beispiel 37

### 2-[6-(4-Hydroxy-3-methoxy-phenyl)-3-oxo-2,3-dihydro-pyridazin-4-yl]-3H-beuzimidazol-5-carbomäure

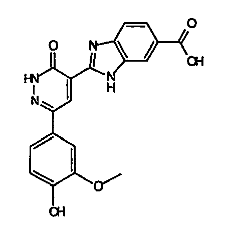

MS (ES+) m/z 379 (M+H)
Beispiel 37 wird entsprechend Beispiel 15 hergestellt.

### Beispiel 38

### 4-(3H-Imidazol[4,5-c]pyridin-2-yl)-6-pyridin-4-yl-2H-pyridazin-3-on

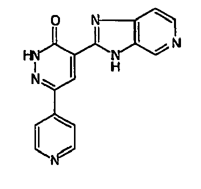

MS (ES+) m/z 291 (M+H)
Beispiel 38 wird entsprechend Beispiel 1 hergestellt.

### Beispiel 39

### 6-[6-Methyl-2-(2-morpholin-4-yl-ethylamino)-pyrimidin-4-yl]-4-(6-trifluormethyl-1H-benzimidazol-2-yl)-2H-pyridazin-3-on

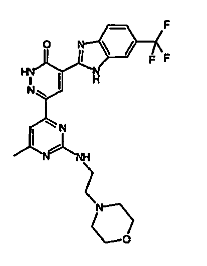

MS (ES+) m/z 501 (M+H)
Beispiel 39 wird analog zu Beispiel 35 hergestellt.

### Beispiel 40

### 4-(6-Chlor-1H-benzimidazol-2-yl)-6-(2-methylsulfanyl-pyrimidin-4-yl)-2H-pyridazin-3-on

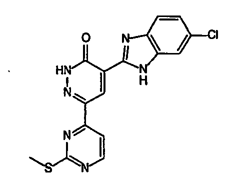

MS (ES+) m/z 371 (M+H)
Beispiel 40 wird entsprechend Beispiel 4 hergestellt.

### Beispiel 41

### 6-(4-Hydroxy-3,5-dimethyl-phenyl)-4-(7-methyl-1H-benzimidazol-2-yl)-2H-pyridazin-3-on

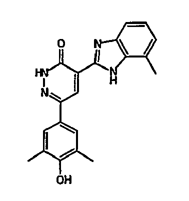

### a) 6-Chlor-3-methoxy-pyridazin-4-carbaldehyd

n-Butyllithium wird bei -75°C zu einer Lösung von 2,2,6,6-Tetramethylpiperidin in THF getropft. Die Lösung wird während 30 Minuten bei 0 °C gerührt. Nach Kühlen auf -75°C wird eine auf -75°C vorgekühlte Lösung von 3-Chlor-6-methoxypyridazin in THF zugetropft. Die Reaktion wird für 30 Minuten bei -75 °C gerührt. Anschließend wird auf - 75 °C vorgekühltes DMF zugetropft und für weitere 90 Minuten bei -75 °C gerührt. Die Reaktionsmischung wird mit einer Mischung aus konz. wässriger HCl (5 ml), Ethanol (20 ml) und THF (25 ml) versetzt und auf Raumtemperatur erwärmt und langsam mit gesättigter wässriger NaHCO₃-Lösung versetzt. THF wird am Rotationsverdampfer entfernt und die wässrige Phase dreimal mit Dichlormethan extrahiert. Die vereinigten organischen Phasen werden mit MgSO4 getrocknet, filtriert und das Lösungsmittel am Rotationsverdampfer entfernt. Der Rückstand wird über Kieselgel gereinigt (Eluent Ethylacetat in Heptan). MS (CI+) m/z 172 (M+).

### b) 2-(6-Chlor-3-methoxy-pyridazin-4-yl)-7-methyl-1H-benzimidazol

6-Chlor-3-methoxy-pyridazin-4-carbaldehyd wird in DMF gelöst und 2,3-diaminotoluene zugegeben. Die Reaktionslösung wird in der Mikrowelle während 30 Minuten auf 120°C erwärmt. Das Rohprodukt wurde über Kieselgel gereinigt (Eluent Ethylacetat in Heptan). MS (ES+) m/z 275 (M+H).

### c) 4-[6-Methoxy-5-(7-methyl-1H-benzimidazol-2-yl)-pyridazin-3-yl]-2,6-dimethyl-phenol

2-(6-Chlor-3-methoxy-pyridazin-4-yl)-7-methyl-1H-benzimidazol wird analog zu Beispiel 15c) mit 2,6-Dimethylphenol-4-boronsäurepinakolester umgesetzt. MS (ES+) m/z 361 (M+H).

### d) 6-(4-Hydroxy-3,5-dimethylphenyl)-4-(7-methyl-1H-benzimidazol-2-yl)-2H-pyridazin-3-on

4-(6-Methoxy-5-(7-methyl-1H-benzimidazol-2-yl)-pyridazin-3-yl]-2,6-dimethyl-phenol wird analog zu Beispiel 15e) umgesetzt. Die Reinigung erfolgt mittels präparativer RP-HPLC (Gradient 0-100% Acetonitril in Wasser (+0.01 % Trifluoressigsäure). MS (ES+) m/z 347 (M+H). NMR(500MHz; DMSO-d6) 13.83 (s, 1H), 8.77 (s, 1H), 8.75 (brs, 1H), 7.65 (d, 1H), 7.54 (s, 2H), 7.26 (t, 1H), 7.17 (m, 1H), 2.65 (s, 3H), 2.27 (s, 6H).

### Beispiel 42

### 4-[6-(4-Methyl-piperazin-1-ylmethyl)-1H-benzimidazol-2-yl]-6-pyridin-4-yl-2H-pyridazin-3-on

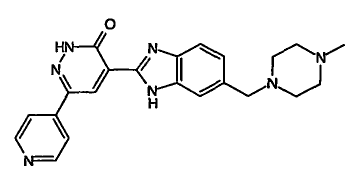

### a) 6-Chlor-4-(6-methoxycarbonyl-1-(2-trimethylsilanyl-ethoxymethyl)-1H-benzimidazol-2-yl]-2-(2-trimethylsilanyl-ethoxymethyl)-2H-pyridazin-3-on

Die Verbindung wird analog zu Beispiel 15c) ausgehend von 6-Chlor-3-oxo-2,3-dihydropyridazin-4-carbonsäure und 3,4-Diaminobenzosäuremethylester hergestellt.

### b) 6-Chlor-4-(6-formyl-1-(2-trimethylsilanyl-ethoxytnethyl)-1H-benzimidazol-2-yl]-2-(2-trimethylsilanyl-ethoxymethyl)-2H-pyridazin-3-on

6-Chlor-4-(6-methoxycarbonyl-1-(2-trimethylsilanyl-ethoxymethyl)-1H-benzimidazol-2-yl]-2-(2-trimethylsilanyl-ethoxymethyl)-2H-pyridazin-3-on (3.0g, 5.3 mmol) wird in Dichlormethan gelöst und die Lösung auf 0 °C gekühlt. 15 % DIBAIH in Toluol wird zugetropft und die Reaktionsmischung während 1 Stunde bei Raumtemperatur gerührt. Anschließend wird nochmals 15 % DIBA1H in Toluol bei 0 °C zugetropft und für 1 Stunde bei Raumtemperatur gerührt. Dann wird direkt Mangan(IV)oxid zugegeben die Reaktionslösung für 3 Stunden zum Rückfluss erhitzt. Es wird alle 3 Stunden zusätzliches Manganoxid zugegeben, bis der Umsatz gemäß LC-MS komplett ist. Die Reaktionslösung wird über Kieselguhr filtriert, der Rückstand mehrmals mit Dichlormethan gewaschen und das Lösungsmittel am Rotationsverdampfer entfernt.
MS (ES+) m/z 565 (M+H).

### c) 6-Chlor-4-(6-(4-methyl-piperazin-1-ylmethyl)-1-(2-trimethylsilanylethoxymethyl)-1H-benzimidazol-2-yl]-2-(2-trimethylsilanyl-ethox-ymethyl)-2H-pyridazin-3-on

6-Chlor-4-(6-formyl-1-(2-trimethylsilanyl-ethoxymethyl)-1H-benzimidazol-2-yl]-2-(2-trimethylsilanyl-ethoxymethyl)-2H-pyridazin-3-on wird in Dichlormethan gelöst. 4-Methylpiperazine und Essigsäure werden zugegeben. Nach 15 Minuten wird Natriumtriacetoxyborohydrid in mehreren Portionen während 3 Stunden zugegeben. Nach vollständigem Umsatz wird gesättigte wässrige NaHCO₃-Lösung zugegeben und die Reaktionslösung gerührt, bis keine CO₂-Entwicklung mehr beobachtet wird. Es wird mit Dichlormethan verdünnt und die wässrige Phase mit Dichlormethan extrahiert. Die vereinigten organischen Phasen werden mit MgSO₄ getrocknet und das Lösungsmittel am Rotationsverdampfer entfernt.
MS (ES+) m/z 535 (M+H).

### d) 4-[6-(4-Methyl-piperazin-1-ylmethyl)-1-(2-trimethylsilanyl-ethoxymethyl)-1H-benzimidazol-2-yl]-6-pyridin-4-yl-]-2-(2-trimethylsilanyl-ethoxymethyl)-2H-pyridazin-3-on

6-Chlor-4-(6-(4-methyl-piperazin-1-ylmethyl)-1-(2-trimethylsilanyl-ethoxymethyl)-1H-benzimidazol-2-yl]-2-(2-trimethylsilanyl-ethoxymethyl)-2H-pyridazin-3-on wird analog zu Beispiel 15d) mit Pyridin-4-boronsäurepinakolester umgesetzt. Das durch Entfernen des Lösungsmittels erhaltene Rohprodukt wird direkt im nächsten Schritt eingesetzt.
MS (ES+) m/z 661 (M+H).

### e) 4-[6-(4-Methyl-piperazin-1-ylmethyl)-1H-benzimidazol-2-yl]-6-pyridin-4-yl-2H-pyridazin-3-on

Rohes 4-[6-(4-methyl-piperazin-1-ylmethyl)-1-(2-trimethylsilanyl-ethoxymethyl)-1H-benzimidazol-2-yl]-6-pyridin-4-yl-]-2-(2-trimethylsilanyl-ethoxymethyl)-2H-pyridazin-3-on wird analog zu Beispiel 15e) umgesetzt.
MS (ES+) m/z 402.27 (M+H).

### Beispiel 43

### 6-(4-Hydroxy-3,5-dimethyl-phenyl)-4-[6-(4-methyl-piperazin-1-ylmethyl)-1H-benzimidazol-2-yl]-2H-pyridazin-3-on

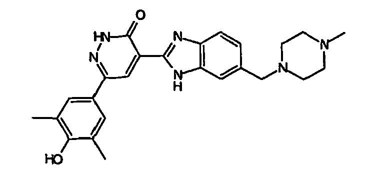

MS (ES+) m/z 445 (M+H).
Beispiel 43 wird entsprechend Beispiel 42 hergestellt.

### Beispiel 44

### 2-[6-(4-Hydroxy-3,5-dimethyl-phenyl)-3-oxo-2,3-dihydro-pyridazin-4-yl]-3H-benzimidazol-5-carbonsäure

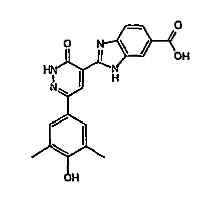

MS (ES+) m/z 377 (M+H).
Beispiel 44 wird entsprechend Beispiel 15 hergestellt.

### Beispiel 45

### 4-(1H-Benzimidazol-2-yl)-6-chlor-2H-pyridazin-3-on

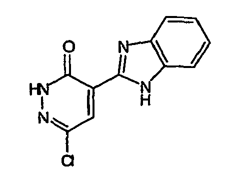

### a) 6-Chlor-3-oxo-2,3-dihydro-pyridazine-4-carbonsäure (2-amino-phenyl)-amid

30,5g 6-Chlor-3-oxo-2,3-dihydro-pyridazine-4-carbonsäure, 18g ortho Phenylendiamin und 2g DMAP werden in 800 ml DMF gelöst, bei Raumtemperatur 32,2g 1-Ethyl-3-(3-dimethylaminopropyl)carbodiimide Hydrochloride hinzugefügt und die resultierende Lösung 4 Stunden bei Raumtemperatur gerührt. Zur Aufarbeitung wird das Lösungsmittel abdestilliert und der Rückstand in 70 Grad warmen Wasser suspendiert. Der Niederschlag wird abfiltriert, mehrfach mit Wasser gewaschen und anschließend im Vakuum getrocknet. MS (ES+) m/z 265 (M+H).

### b) 4-(1H-Benzimidazol-2-yl)-6-chlor-2H-pyridazin-3-on

Eine Lösung von 26,4g 6-Chlor-3-oxo-2,3-dihydro-pyridazine-4-carbonsäure(2-aminophenyl)-amid in 500 ml Essigsäure wird 2,5 Stunden bei 110°C gerührt und anschließend das Lösungsmittel abdestilliert. Das so erhaltene Rohprodukt wird ohne weitere Reinigung eingesetzt.
MS (ES+) m/z 247 (M+H).

### Beispiel 46

### 4-(1H-Benzimidazol-2-yl)-6-thiophen-3-yl-2H-pyridazin-3-on

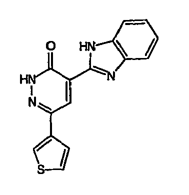

### a) 6-Chlor-2-(2-trimethylsilanyl-ethoxymethyl)-4-[1-(2-trimethylsilanylethoxymethyl)-1H-benzimidazol-2-yl]-2H-pyridazin-3-on

6-Chlor-4-(1H-benzimidazol-2-yl)-2H-pyridazin-3-on (7g; 28,4 mmol, Beispiel 45) wird in DMF (175ml) gelöst, Cäsiumcarbonate (37 g; 113 mmol) und (2-chlormethoxy-ethyl)-trimethyl-silan (15,8 g; 85 mmol) werden zugefügt und die Mischung wird bei Raumtemperatur für 12 h gerührt. Zur Aufarbeitung wird mit 800 ml Wasser verdünnt, mehrfach mit Ethylacetat extrahiert und die organischen Phasen mit gesättigter NaCl Lösung gewaschen und über Magnesiumsulfat getrocknet. Nach Abdampfen des Lösungsmittels wird das Rohprodukt mittels Kieselgel Chromatographie gereinigt.
Ausbeute: 10,5g.

### b) 6-Thiophen-3-yl-2-(2-trimethylsilanyl-ethoxymethyl)-4-[1-(2-trimethylsilanylethoxymethyl)-1H-benzimidazol-2-yl]-2H-pyridazin-3-on

44 mg 6-Chlor-2-(2-trimethylsilanyl-ethoxymethyl)-4-[1-(2-trimethylsilanylethoxymethyl)-1H-benzimidazol-2-yl]-2H-pyridazin-3-on, 14 mg Thiophen-3-boronsäure und 36 mg Kaliumcarbonat werden in 1,5 ml eines 1:2 Gemischs aus Wasser und Dimethoxyethan gelöst und zum Entgasen Argon durch die Lösung geleitet. Nach Zugabe von 7 mg (1,1'-Bis(diphenylphosphin)ferrocen)palladium(II)chlorid-Dichlormethan-Komplex wird die Lösung 4 Stunden bei 85°C gerührt. Zur Aufarbeitung wird mit Wasser verdünnt und mehrfach mit Ethylacetat extrahiert. Die vereinigten organischen Phasen werden mittels Kieselgelkartusche getrocknet und das Lösungsmittel abdestilliert. Das Rohprodukt wird mittels Kieselgelchromatographie (n-Heptan/Ethylacetat) gereinigt.
Ausbeute: 32 mg

### c) 4-(1H-Benzimidazol-2-yl)-6-thiophen-3-yl-2H-pyridazin-3-on

30 mg 6-Thiophen-3-yl-2-(2-trimethylsilanyl-ethoxymethyl)-4-[1-(2-trimethylsilanylethoxymethyl)-1H-benzimidazol-2-yl]-2H-pyridazin-3-on wird in einer Mischung aus Dichlormethan (1 ml) und Triflluoressigsäure (1 ml) gelöst, 18 ul Ethandithiol hinzugegeben und die resultierende Lösung 18 Stunden bei Raumtemperatur gerührt. Nach Abdestillieren des Lösungsmittels wird das Rohprodukt mittels präparativer RP-HPLC gereinigt (0-100 % Acetonitril (+0.05 % Ameisensäure) in Wasser (+0.05 % Ameisensäure)).
Ausbeute: 32mg
MS (ES+) m/z 295 (M+H).

### Beispiel 47

### 4-(1H-Benzimidazol-2-yl)-6-thiazol-2-yl-2H-pyridazin-3-on

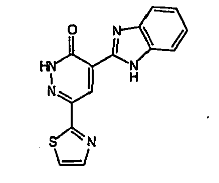

MS (ES+) m/z 296 (M+H).
Beispiel 47 wird entsprechend Beispiel 46 hergestellt.

### Beispiel 48

### 4-(1H-Benzimidazol-2-yl)-6-cyclopropyl-2H-pyridazin-3-on

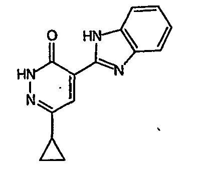

MS (ES+) m/z 253 (M+H).
Beispiel 48 wird entsprechend Beispiel 46 hergestellt.

### Beispiel 49

### 4-[6-(4-Methyl-piperazin-1-ylmethyl)-1H-benzimidazol-2-yl]-6-thiophen-3-yl-2H-pyridazin-3-on

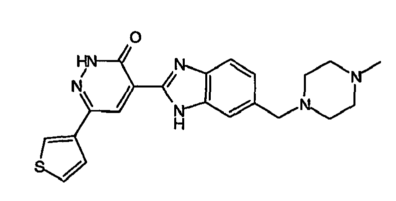

MS (ES+) m/z 407 (M+H).
Beispiel 49 wird entsprechend Beispiel 42 hergestellt.

### Beispiel 50

### 4-(1H-Benzimidazol-2-yl)-6-(1-methyl-6-oxo-1,6-dihydro-pyridin-3-yl)-2H-pyridazin-3-on

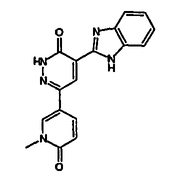

MS (ES+) m/z 320 (M+H).
Beispiel 50 wird entsprechend Beispiel 46 hergestellt.

### Beispiel 51

### 4-(1H-Benzimidazol-2-yl)-6-(3-fluor-pyridin-4-yl)-2H-pyridazin-3-on

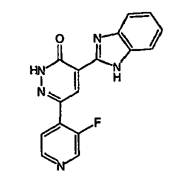

MS (ES+) m/z 308 (M+H).
Beispiel 51 wird entsprechend Beispiel 46 hergestellt.

### Beispiel 52

### 2-[6-(2-Methylsulfanyl-pyrimidin-4-yl)-3-oxo-2,3-dihydro-pyridazin.4-yl]-3H-benzimidazol-5-carbonsäure

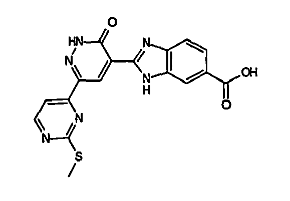

Die Verbindung wird analog zu Beispiel 15 hergestellt. Anstelle der Suzuki-Kupplung wird eine Stille-Kupplung durchgeführt. Dabei werden unter Argon 6-Chlor-4-[5-methoxycarbonyl-1-(2-trimethylsilanyl-ethoxymethyl)-1H-benzimidazol-2-yl]-2-(2-trimethylsilanyl-ethoxymethyl)-2H-pyridazin-3-on, 2-Methylsulfanyl-4-tributylstannylpyrimidin, Tetraethylammoniumchlorid und Bis(triphenylphosphin)palladium(II)chlorid in DMF gelöst und für 6 Stunden auf 80°C erhitzt. Nach Abkühlen auf Raumtemperatur wird 30 % wässrige Kaliumfluorid-Lösung zugegeben und 30 Minuten gerührt. Extraktion mit Ethylacetat und Trocknung über eine Trocknungssäule ergeben nach Reinigung über SiO₂ 4-[5-Methoxycarbonyl-1-(2-trimethylsilanylethoxymethyl)-1H-benzimidazol-2-yl]-6-(2-Methylsulfanyl-pyrimidin-4-yl)-2-(2-trimethylsilanyl-ethoxymethyl)-2H-pyridazin-3-on das analog zu Beispiel 15 weiter umgesetzt wird.
MS (ES+) m/z 381 (M+H).

### Beispiel 53

### 4-[6-(4-Methyl-piperazin-1-ylmethyl)-1H-benzimidazol-2-yl]-6-phenyl-2H-pyridazin-3-on

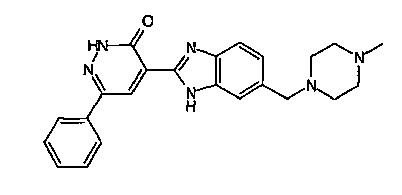

MS (ES+) m/z 401 (M+H).
Beispiel 53 wird entsprechend Beispiel 42 hergestellt.

### Beispiel 54

### 6-(3-Hydrox-y-phenyl)-4-[6-(4-methyl-piperazin-1-ylmethyl)-1H-benzimidazol-2-yl]-2H-pyridazin-3-on

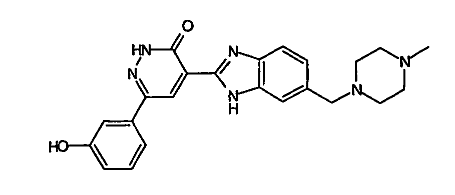

MS (ES+) m/z 417 (M+H).
Beispiel 54 wird entsprechend Beispiel 42 hergestellt.

### Beispiel 55

### 4-(1H-Benzimidazol-2-yl)-6-[2-(2-morpholin-4-yl-ethylamino)-pyrimidin-4-yl]-2H-pyridazin-3-on

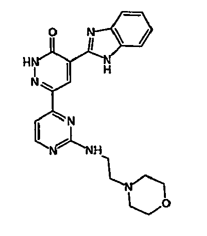

### a) 6-Oxo-1-(2-trimethylsilanyl-ethoxymethyl)-5-[1-(2-trimethylsilanylethoxymethyl)-1H- benzimidazol-2-yl]-1,6-dihydro-pyridazin-3-boronsäure

4g 6-Chlor-2-(2-trimethylsilanyl-ethoxymethyl)-4-[1-(2-trimethylsilanyl-ethoxymethyl)-1H-benzimidazol-2-yl]-2H-pyridazin-3-on (Beispiel 46), 4,1g Bis(pinacolato)diboron, 0.19 g (1,1'-Bis(diphenylphosphin)ferrocen)palladium(Inchlorid-Dichlormethan-Komplex und 2,3 g Kaliumacetat werden in 60 ml DMSO gelöst. Die Lösung wird mit Argon entgast und 2,5 Stunden auf 95°C erhitzt. Zur Aufarbeitung wird mit 600 ml Wasser versetzt, mehrfach mit Dichlormethan extrahiert und die organische Phase mit gesättigter Natriumchloridlösung gewaschen und mittels Phasenseparatorkartusche getrocknet. Nach Abdestillieren des Lösungsmittels wird das Rohprodukt mittels preparativer RP-HPLC gereinigt (0-100 % Acetonitril in Wasser (+0.01 % Trifluoressigsäure)).
Ausbeute: 3,75 g

### b) 6-(2-Chloro-pyrimidin-4-yl)-2-(2-trimethylsilanyl-ethoxymethyl)-4-[1-(2-trimethylsilanyl- ethoxymethyl)-1H-benzimidazol-2-yl]-2H-pyridazin-3-on

1,32 g 6-Oxo-1-(2-trimethylsilanyl-ethoxymethyl)-5-[1-(2-trimethylsilanyl-ethoxymethyl)-1H-benzimidazol-2-yl]-1,6-dihydro-pyridazin-3-boronsäure, 583 mg 2,4-Dichlorpyrimidin, und 3,3 g Cäsiumcarbonat werden in 17 ml eines 1:4 Gemischs aus Wasser und Dioxan gelöst und zum Entgasen Argon durch die Lösung geleitet. Nach Zugabe von 125 mg (1,1'-Bis(diphenylphosphin)ferrocen)palladium(II)chlorid-Dichlormethan-Komplex wird die Lösung 3 Stunden bei 97°C gerührt. Zur Aufarbeitung wird mit 50 ml Wasser verdünnt und mehrfach mit Ethylacetat extrahiert. Die vereinigten organischen Phasen werden mittels Kieselgelkartusche getrocknet und das Lösungsmittel abdestilliert. Das Rohprodukt wird mittels präparativer RP-HPLC gereinigt (0-100 % Acetonitril in Wasser (+0.01 % Trifluoressigsäure)).
Ausbeute: 1g

### c) 6-[2-(2-Morpholin-4-yl-ethylamino)-pyrimidin-4-yl]-2-(2-trimethylsilanylethoxymethyl)-4-[1-(2-trimethylsilanyl-ethoxymethyl)-1H-benzimidazol-2-yl]-2H-pyridazin-3-on

100 mg 6-(2-Chlor-pyrimidin-4-yl)-2-(2-trimethylsilanyl-ethoxymethyl)-4-[1-(2-trimethylsilanyl-ethoxymethyl)-1H-benzimidazol-2-yl]-2H-pyridazin-3-on werden in 0,5 ml N-(2-Aminoethyl)morpholin suspendiert und die Reaktionsmischung 20 Minuten in der Mikrowelle auf 100°C erhitzt. Das Produkt wird mittels präparativer RP-HPLC gereinigt (0-100 % Acetonitril (+0.05 % Ameisensäure) in Wasser (+0.05 % Ameisensäure)).
Ausbeute: 91 mg

### d) 4-(1H-Benzimidazol-2-yl)-6-[2-(2-morpholin-4-yl-ethylamino)-pyrimidin-4-yl]-2H-pyridazin-3-on

88 mg 6-[2-(2-Morpholin-4-yl-ethylamino)-pyrimidin-4-yl]-2-(2-trimethylsilanylethoxymethyl)-4-[1-(2-trimethylsilanyl-ethoxymethyl)-1H-benzimidazol-2-yl]-2H-pyridazin-3-on werden in 2 ml eines 1:1 Gemischs aus Dichlormethan und Trifluoressigsäure gelöst und das Gemisch 2 Stunden bei Raumtemperatur gerührt. Anschließend werden die flüchtigen Komponenten abdestilliert, der Rückstand in 1 ml Methanol gelöst und 1 ml einer zweimolaren, wässrigen Natriumhydroxid Lösung zugegeben. Die Lösung wird 12 Stunden bei Raumtemperatur gerührt und anschließend zur Isolierung des Produkts direkt auf die präparativer RP-HPLC (0-100 % Acetonitril (+0.05 % Ameisensäure) in Wasser (+0.05 % Ameisensäure)) gegeben.
Ausbeute: 43 mg
MS (ES+) m/z 419 (M+H).

### Beispiel 56

### 4-(1H-Benzimidazol-2-yl)-6-[1-(2-morpholin-4-yl-ethyl)-6-oxo-1,6-dihydro-pyridin-3-yl]-2H-pyridazin-3-on

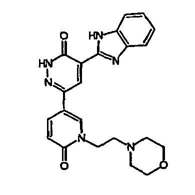

MS (ES+) m/z 419 (M+H).
Beispiel 56 wird entsprechend Beispiel 55d hergestellt.

### Beispiel 57

### 6-(3-Amino-1-methyl-1H-pyrazol-4-yl)-4-(1H-benzimidazol-2-yl)-2H-pyridazin-3-on

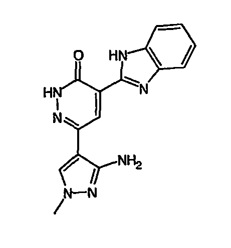

MS (ES+) m/z 308 (M+H).
Beispiel 57 wird entsprechend Beispiel 55d synthetisiert.

### Beispiel 58

### 4-(1H-Benzimidazol-2-yl)-6-(2-cyclopropylamino-pyrimidin-4-yl)-2H-pyridazin-3-on

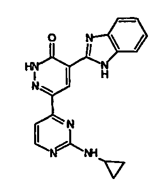

MS (ES+) m/z 346 (M+H).
Beispiel 58 wird entsprechend Beispiel 55 synthetisiert.

### Beispiel 59

### 4-(1H-Benzimidazol-2-yl)-6-[2-(2-hydroxy-1,1-dimethyl-ethylamino)-pyrimidin-4-yl]-2H-pyridazin-3-on

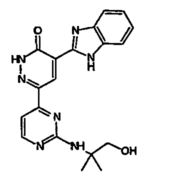

MS (ES+) m/z 378 (M+H).
Beispiel 59 wird entsprechend Beispiel 55 synthetisiert.

### Beispiel 60

### 4-(1H-Benzimidazol-2-yl)-6-(2-dimethylamino-pyrimidin-4-yl)-2H-pyridazin-3-on

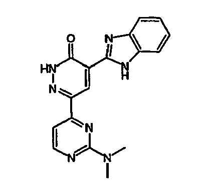

MS (ES+) m/z 334 (M+H).
Beispiel 60 wird entsprechend Beispiel 55 synthetisiert.

### Beispiel 61

### 4-(1H-Benzimidazol-2-yl)-6-[2-(2-methoxy-ethylamino)-pyriniidin-4-yl]-2H-pyridazin-3-on

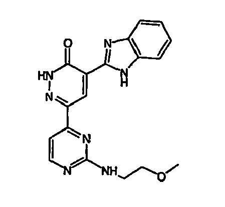

MS (ES+) m/z 364 (M+H).
Beispiel 61 wird entsprechend Beispiel 55 synthetisiert.

### Beispiel 62

### {4-[5-(1H-Benzimidazol-2-yl)-6-oxo-1,6-dihydro-pyridazin-3-yl]-pyrimidin-2-ylamino}essigsäure

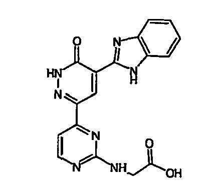

MS (ES+) m/z 364 (M+H).
Beispiel 62 wird entsprechend Beispiel 55 synthetisiert.

### Beispiel 63

### 3-{4-[5-(1H-Benzimidazol-2-yl)-6-oxo-1,6-dihydro-pyridazin-3-yl]-pyrimidin-2-ylamino}-propionsäure

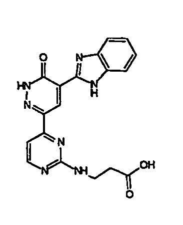

MS (ES+) m/z 378 (M+H).
Beispiel 63 wird entsprechend Beispiel 55 synthetisiert.

### Beispiel 64

### 4-(1H-Benzimidazol-2-yl)-6-morpholin-4-yl-2H-pyridazin-3-on

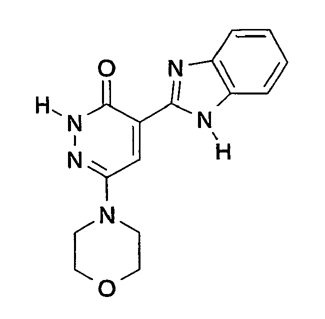

### a) 6-Morpholin-4-yl-2-(2-trimethylsilanyl-ethoxymethyl)-4-[1-(2-trimethylsilanylethoxymethyl)-1H-benzoimidazol-2-yl]-2H-pyridazin-3-on

120 mg 6-Chlor-2-(2-trimethylsilanyl-ethoxymethyl)-4-[1-(2-trimethylsilanyl-ethoxymethyl)-1H-benzimidazol-2-yl]-2H-pyridazin-3-on werden in 0.5 ml Morpholin gelöst und 1 Stunde auf 150°C und 1 Stunde auf 170°C in Mikrowelle erhitzt.
Das Endprodukt wird mittels RPHPLC:(Wasser (0,05% HCOOH), Acetonitril (0,05% HCOOH)) isoliert.
Ausbeute: 76 mg.

### b) 4-(1H-Benzimidazol-2-yl)-6-morpholin-4-yl-2H-pyridazin-3-on

Diese Verbindung wird entsprechend 55d aus 6-Morpholin-4-yl-2-(2-trimethylsilanylethoxymethyl)-4-[1-(2-trimethylsilanyl-ethoxymethyl)-1H-benzoimidazol-2-yl]-2H-pyridazin-3-on dargestellt.
Ausbeute: 41.5 mg
MS (ES+) m/z 298 (M+H).

### Beispiel 65

### 4-(1H-Benzimidazol-2-yl)-6-[2-methylsulfanyl-pyrimidin-4-yl]-2H-pyridazin-3-on

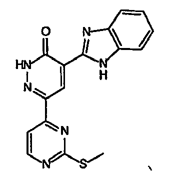

Die Verbindung wird analog zu Beispiel 15 hergestellt. Anstelle der Suzuki-Kupplung wird eine Stille-Kupplung durchgeführt. Dabei werden unter Argon 6-Chlor-4-[1-(2-trimethylsilanyl-ethoxymethyl)-1H-benzimidazol-2-yl]-2-(2-trimethylsilanylethoxymethyl)-2H-pyridazin-3-on, 2-Methylsulfanyl-4-tributylstannylpyrimidin, Tetraethylammoniumchlorid und Bis(triphenylphosphin)palladium(II)chlorid in DMF gelöst und für 6 Stunden auf 80°C erhitzt. Nach Abkühlen auf Raumtemperatur wird 30 % wässrige Kaliumfluorid-Lösung zugegeben und 30 Minuten gerührt. Extraktion mit Ethylacetat und Trocknung über eine Trocknungssäule ergeben nach Reinigung über SiO₂ 4-[1-(2-trimethylsilanyl-ethoxymethyl)-1H-benzimidazol-2-yl]-6-(2-Methylsulfanylpyrimidin-4-yl)-2-(2-trimethylsilanyl-ethoxymethyl)-2H-pyridazin-3-on das analog zu Beispiel 15 weiter umgesetzt wird
MS (ES+) m/z 337 (M+H).

### Beispiel 66

### 6-(4-Hydroxy-3,5-dimethyl-phenyl)-4-(6-[1,4]or.azepan-4-ylmethyl-1H-benzimidazol-2-yl)-2H-pyridazin-3-on

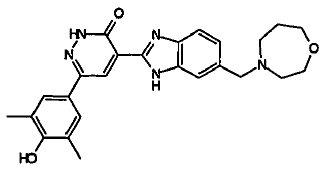

MS (ES+) m/z 446 (M+H).
Beispiel 66 wird entsprechend Beispiel 42 synthetisiert.

### Beispiel 67

### 4-(1H-Benzimidazol-2-yl)-6-(1,5-dimethyl-6-oxo-1,6-dihydro-pyridin-3-yl)-2H-pyridazin-3-on

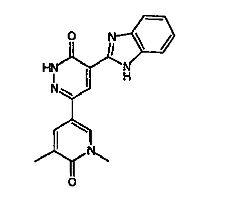

MS (ES+) m/z 334 (M+H).
Beispiel 67 wird entsprechend Beispiel 55d synthetisiert.

### Beispiel 68

### 4-(1H-Benzimidazol-2-yl)-6-imidazol-1-yl-2H-pyridazin-3-on

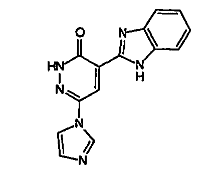

### a) 4-[1-(2-Dimethylsilanyl-ethoxymethyl)-1H-benzimidazol-2-yl]-6-imidazol-1-yl-2-(2-trimethylsilanyl-ethoxymethyl)-2H-pyridazin-3-on

100 mg 6-Oxo-1-(2-trimethylsilanyl-ethoxymethyl)-5-[1-(2-trimethylsilanylethoxymethyl)-1H-benzimidazol-2-yl]-1,6-dihydro-Pyridazin-3-boronsäure (Beispiel 55), 35,5 mg Kupfer(II)acetat und 20 mg Imidazol werden in trockenem Pyridin gelöst und die Lösung 3 Stunden bei 80°C gerührt. Zur Aufarbeitung wird mit 20 ml Wasser verdünnt, mehrfach mit Ethylacetat extrahiert, die organischen Phasen mittels Kieselgelkartusche getrocknet und die flüchtigen Komponenten abdestilliert. Das Rohprodukt wird mittels präperativer RP HPLC (0-100% Acetonitril (+0.05% Ameisensäure) in Wasser (+0.05% Ameisensäure)) gereinigt.
Ausbeute: 21 mg

### b) 4-(1H-Benzimidazol-2-yl)-6-imidazol-1-yl-2H-pyridazin-3-on

40 mg 4-[1-(2-Dimethylsilanyl-ethoxymethyl)-1H-benzimidazol-2-yl]-6-imidazol-1-yl-2-(2-trimethylsilanyl-ethoxymethyl)-2H-pyridazin-3-on werden in 2 ml eines 1:1 Gemischs aus Dichlormethan und Trifluoressigsäure gelöst und das Gemisch 2 Stunden bei Raumtemperatur gerührt. Anschließend werden die flüchtigen Komponenten abdestilliert, der Rückstand in 1 ml Methanol gelöst und 1 ml einer zweimolaren, wässrigen Natriumhydroxid Lösung zugegeben. Die Lösung wird 12 Stunden bei Raumtemperatur gerührt und anschließend zur Isolierung des Produkts direkt auf die präparativer RP-HPLC (0-100 % Acetonitril (+0.05 % Ameisensäure) in Wasser (+0.05 % Ameisensäure)) gegeben.
Ausbeute: 19,3 mg
MS (ES+) m/z 279 (M+H).

### Beispiel 69

### 4-(6-Chlor-3H-imidazo[4,5-b]pyridin-2-yl)-6-(4-hydroxy-3,5-dimethyl-phenyl)-2H-pyridazin-3-on

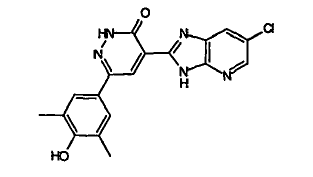

MS (ES+) m/z 368 (M+H).
Beispiel 75 wird entsprechend den Beispielen 45 und 15 synthetisiert.

### Beispiel 70

### 6-Chlor-4-(5-hydroxy-1H-benzimidazol-2-yl)-2H-pyridazin-3-on

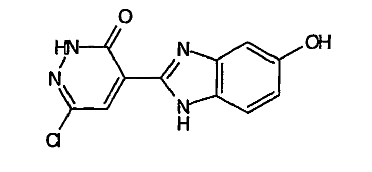

MS (ES+) m/z 263 (M+H).
Beispiel 75 wird entsprechend Beispiel 45 synthetisiert.

### Beispiel 71

### 4-(1H-Benzimidazol-2-yl)-6-pyrazol-1-yl-2H-pyridazin-3-on

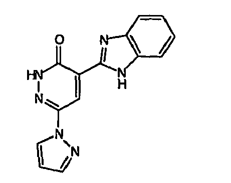

MS (ES+) m/z 279 (M+H).
Beispiel 71 wird entsprechend Beispiel 68 hergestellt.

### Beispiel 72

### 4-(1H-Benzimidazol-2-yl)-6-thiazol-4-yl-2H-pyridazin-3-on

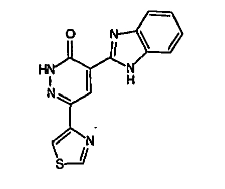

Beispiel 72 wird entsprechend Beispiel 55d hergestellt.

### Beispiel 73

### 4-(1H-Benzimidazol-2-yl)-6-(4-hydroxy-3-methoxy-5-methylaminomethyl-phenyl)-2H- pyridazin-3-on

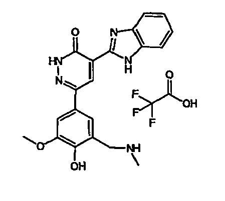

### (a) 5-Brom-3-methoxy-2-(2-trimethylsilanyl-ethoxymethoxy)-benzaldehyd

10 g 5-Brom-2-hydroxy-3-methoxy-benzaldehyd, 23.9 g Kaliumcarbonat und 12,2 g (2-Chlormethoxy-ethyl)-trimethyl-silan wird in 500 ml DMF suspendiert und bei Raumtemperatur 48 Stunden gerührt. Das DMF wird im Vakuum abdestilliert, der Rückstand in Ethylacetat gelöst und die resultierende Lösung mit Wasser extrahiert. Anschließend wird über Magnesiumsulfat getrocknet, filtriert und das Lösungsmittel abdestilliert. Das Rohprodukt wird mittels Kieselgelchromatographie (Heptan/Ethylacetat) gereinigt.
Ausbeute: 15.3g.

### (b) 3-Methoxy-5-(4,4,5,5-tetramethyl-[1,3,2]dioxaborolan-2-yl)-2-(2-trimethylsilanylethoxymethoxy)-benzaldehyd

5g 5-Brom-3-methoxy-2-(2-trimethylsilanyl-ethoxymethoxy)-benzaldehyd, 1.013g (1,1'-Bis(diphenylphosphino)ferrocen)palladium(II)chlorid und 767.3 mg 1,1'-Bis(diphenylphosphino)ferrocen werden in 400 ml Dioxan gelöst und Argon durch die Lösung geleitet. Danach wird 5.27 g Bis(pinacolato)diboron und 4.075 g Kaliumacetat hinzu gefügt und der Ansatz 16 Stunden bei 80°C gerührt. Zur Aufarbeitung wird das Lösungsmittel abdestilliert, der Rückstand in Dichlormethan gelöst, die Lösung filtriert und das Lösungsmittel abdestilliert. Das Rohprodukt wird mittels Kieselgelchromatographie (Heptan/Ethylacetat) gereinigt.
Ausbeute: 5.47g.

### (c) Methoxy-5-{6-oxo-1-(2-trimethylsilanyl-ethoxymethyl)-5-[1-(2-trimethylsilanylethoxymethyl)-1H-benzimidazol-2-yl]-1,6-dihydro-pyridazin-3-yl}-2-(2-trimethylsilanyl-ethoxymethoxy)-benzaldehyd.

Diese Verbindung wird entsprechend Beispiel 15(d) synthetisiert ausgehend von 402 mg 3-Methoxy-5-(4,4,5,5-tetramethyl-[ 1,3,2]dioxaborolan-2-yl)-2-(2-trimethylsilanylethoxymethoxy)-benzaldehyd und 500 mg 6-Chlor-2-(2-trimethylsilanyl-ethoxymethyl)-4-[1-(2-trimethylsilanyl-ethoxymethyl)-1H-benzimidazol-2-yl]-2H-pyridazin-3-on.
Ausbeute 565 mg. MS (ES+) m/z 753 (M+H).

### (d) 6-[3-Methoxy-5-methylaminomethyl-4-(2-trimethylsilanyl-ethoxymethoxy)-phenyl]-2-(2-trimethylsilanyl-ethoxymethyl)-4-[1-(2-trimethylsilanyl-ethoxymethyl)-1H-benzimidazol- 2-yl]-2H-pyridazin-3-on

200 mg Methoxy-5-{6-oxo-1-(2-trimethylsilanyl-ethoxymethyl)-5-[1-(2-trimethylsilanyl-ethoxymethyl)-1H-benzimidazol-2-yl]-1,6-dihydro-pyridazin-3-yl }-2-(2-trimethylsilanyl-ethoxymethoxy)-benzaldehyd werden in 4.5 ml Methanol und Methylamin (0.146 ml einer THF Lösung) gelöst und 18.4 mg Natriumcyanoborohydrid werden hinzu gegeben, gefolgt von 17 mg Essigsäure. Nach 16 Stunden Rühren bei Raumtemperatur werden die flüchtigen Komponenten abdestilliert und das Rohprodukt für die nächste Reaktion ohne weitere Reinigung eingesetzt.

### (e) 4-(1H-Benzimidazol-2-yl)-6-(4-hydroxy-3-methoxy-5-methylanninomethylphenyl)-2H-pyridazin-3-on

180 mg 6-[3-Methoxy-5-methylaminomethyl-4-(2-trimethylsilanyl-ethoxymethoxy)-phenyl]-2-(2-trimethylsilanyl-ethoxymethyl)-4-[1-(2-trimethylsilanyl-ethoxymethyl)-1H-benzimidazol-2-yl]-2H-pyridazin-3-on werden in einem 1:1 Gemisch aus Triflluoressigsäure und Dichlormethan gelöst und das Reaktionsgemisch 5 Stunden bei Raumtemperatur gerührt. Anschließend werden die flüchtigen Komponenten abdestilliert und der Rückstand in einem 1:1 Gemisch aus Methanol und 2N NaOH gelöst. Nach zweistündigem Rühren bei Raumtemperatur wird die Lösung mit 2N HCl neutralisiert, die Lösung bis zur Trockenheit eingedampft und der Rückstand mittels RP HPLC (Acetonitril / Wasser (+0.1 %TFA)) gereinigt.
Ausbeute 29 mg.
MS (ES+) m/z 378 (M+H).

### Beispiel 74

### 4-(1H-Benzimidazol-2-yl)-6-(1-methyl-1H-imidazol-4-yl)-2H-pyridazin-3-on

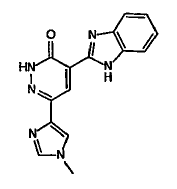

Beispiel 74 wird entsprechend Beispiel 55d synthetisiert

### Beispiel 75

### 6-(4-Hydroxy-3,5-dimethyl-phenyl)-4-(3H-imidazo[4,5-c]pyridin-2-yl)-2H-pyridazin-3-on

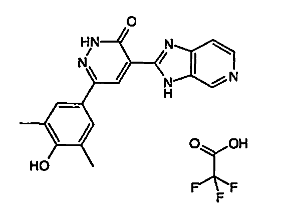

MS (ES+) m/z 344 (M+H).
Beispiel 75 wird entsprechend Beispiel 15 synthetisiert.

### Beispiel 76

### 6-(4-Hydroxy-3,5-dimethyl-phenyl)-4-(3H-imidazo[4,5-b]pyridin-2-yl)-2H-pyridazin-3-on

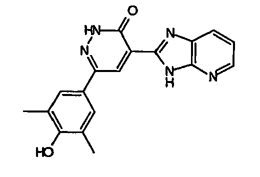

MS (ES+) m/z 344 (M+H).
Beispiel 76 wird entsprechend Beispiel 15 synthetisiert.

### Beispiel 77

### 4-(1H-Benzimidazol-2-yl)-6-[6-(2-morpholin-4-yl-ethylamino)-pyrimidin-4-yl]-2H-pyridazin-3-on

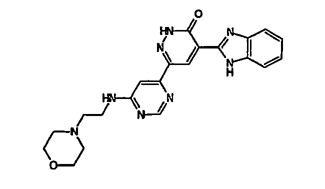

MS (ES+) m/z 419 (M+H).
Beispiel 77 wird entsprechend Beispiel 55 synthetisiert.

### Beispiel 78

### 4-(1H-Benzimidazol-2-yl)-6-[6-(2-methoxy-ethylamino)-pyrimidin-4-yl]-2H-pyridazin-3-on

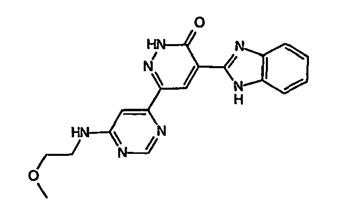

MS (ES+) m/z 364 (M+H).
Beispiel 78 wird entsprechend Beispiel 55 synthetisiert.

### Beispiel 79

### 4-(1H-Benzimidazol-2-yl)-6-[6-(2-hydroxy-1,1-dimethyl-ethylamino)-pyrimidin-4-yl]-2H-pyridazin-3-on

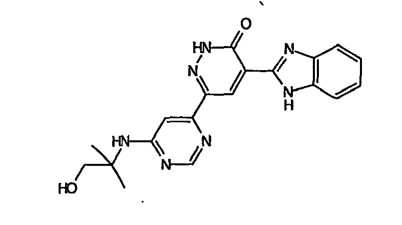

MS (ES+) m/z 378 (M+H).
Beispiel 79 wird entsprechend Beispiel 55 synthetisiert.

### Beispiel 80

### 2-[6-(2-Methyl-6-methylamino-pyrimidin-4-yl)-3-oxo-2,3-dihydro-pyridazin-4-yl]-3H-benzimidazol-5-carbonsäuremethylester

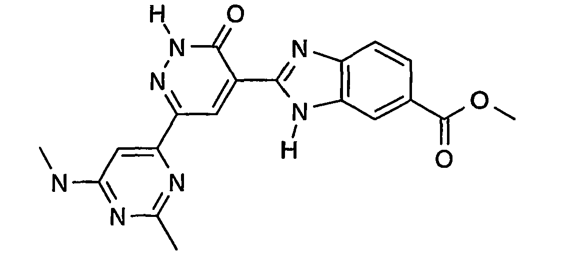

MS (ES+) m/z 392 (M+H).
Beispiel 80 wird entsprechend Beispiel 55 ausgehend von 6-Chlor-4-(6-methoxycarbonyl-1-(2-trimethylsilanyl-ethoxymethyl)-1H-benzimidazol-2-yl]-2-(2-trimethylsilanylethoxymethyl)-2H-pyridazin-3-on (Beispiel 42a) synthetisiert.

### Beispiel 81

### 4-(1H-Benzimidazol-2-yl)-6-(2-methoxy-pyrimidin-4-yl)-2H-pyridazin-3-on

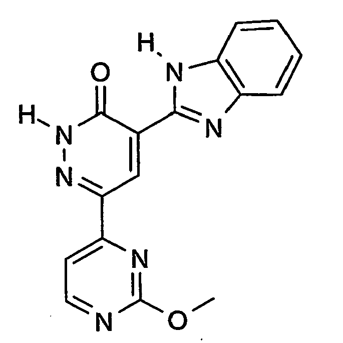

MS (ES+) m/z 321 (M+H).
Beispiel 81 wird analog Beispiel 55 synthetisiert.

### Beispiel 82

### 4-(1H-Benzimidazol-2-yl)-6-(4-hydroxy-3-methoxy-5-oxazol-5-yl-phenyl)-2H-pyridazin-3-on

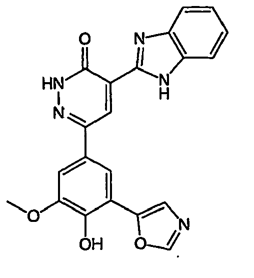

MS (ES+) m/z 402 (M+H).

### (a) 5-[5-Brom-3-methoxy-2-(2-trimethylsilanyl-ethoxymethoxy)-phenyl]-oxazol

1g 5-Brom-3-methoxy-2-(2-trimethylsilanyl-ethoxymethoxy)-benzaldehyd und 0.54g p-Toluolsulfonylmethylisocyanid werden in 40ml of Methanol gelöst, 0.38g o-Kaliumcarbonat portionsweise hinzugefügt und die Reaktionsmischung 18h zum Rückfluß erhitzt. Anschließend wird das Lösungsmittel abdestilliert und das Rohprodukt mittels Kieselgelchromatogroahpie (Ethylacetat/Heptan) gereinigt.
Ausbeute 600mg.
MS (ES+) m/z 401 (M+H).

### (b) 6-[3-Methoxy-5-oxazol-5-yl-4-(2-trimethylsilanyl-ethoxymethoxy)-phenyl]-2-(2-trimethylsilanyl-ethoxymethyl)-4-[1-(2-trimethylsilanyl-ethoxymethyl)-1H-benzimidazol-2-yl]-2H-pyridazin-3-on

Diese Verbindung wird entsprechend Beispiel 35(b) aus 360mg of 5-[5-Brom-3-methoxy-2-(2-trimethylsilanyl-ethoxymethoxy)-phenyl]-oxazol und 310mg 2-(2-trimethylsilanylethoxymethyl)-4-[1-(2-trimethylsilanyl-ethoxymethyl)-1H-benzimidazol-2-yl]-2H-pyridazin-3-on-6-boronsäure synthetisiert.
Ausbeute: 135mg.
MS (ES+) m/z 792 (M+H).

### (c) 4-(1H-Benzimidazol-2-yl)-6-(4-hydroxy-3-methoxy-5-oxazol-5-yl-phenyl)-2H-pyridazin-3-on

Diese Verbindung wird entsprechend Beispiel 15(e) dargestellt.
Ausbeute: 40mg.
MS (ES+) m/z 402 (M+H).

### Beispiel 83

### 6-(3-Chlor-phenyl)-4-[6-(4-methyl-piperazin-1-ylmethyl)-1H-benzimidazol-2-yl]-2H-pyridazin-3-on

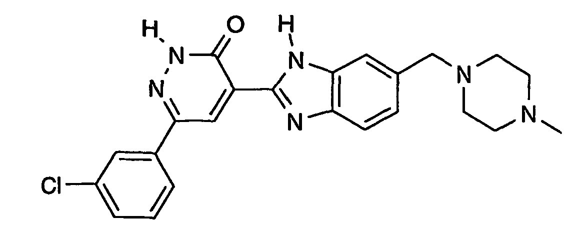

MS (ES+) m/z 435 (M+H).
Beispiel 83 wird entsprechend Beispiel 55 synthetisiert.

### Beispiel 84

### 4-(1H-Benzimidazol-2-yl)-6-[2-(2-morpholin-4-yl-ethoxy)-pyrimidin-4-yl]-2H-pyridazin-3-on

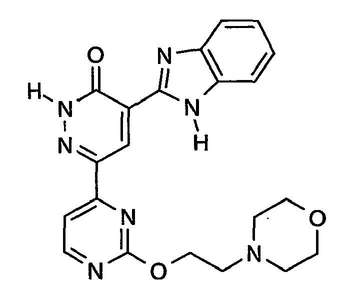

MS (ES+) m/z 420 (M+H).
Beispiel 84 wird analog Beispiel 55 synthetisiert.

### Beispiel 85

### 4-(1H-Benzimidazol-2-yl)-6-[2-(2-diethylamino-ethylamino)-pyrimidin-4-yl]-2H-pyridazin-3-on

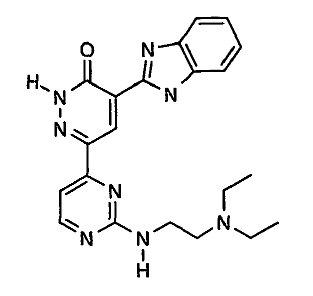

MS (ES+) m/z 405 (M+H).
Beispiel 85 wird analog Beispiel 55 synthetisiert.

### Beispiel 86

### 4-(1H-Benzimidazol-2-yl)-6-(2-cyclopropyl-pyrimidin-4-yl)-2H-pyridazin-3-on

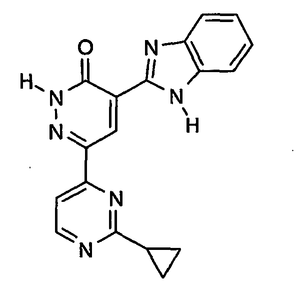

### a) 6-(2-Cyclopropyl-pyrimidin-4-yl)-2-(2-trimethylsilanyl-ethoxymethyl)-4-[1-(2-trimethylsilanyl-ethoxymethyl)-1H-benzoimidazol-2-yl]-2H-pyridazin-3-on

100 mg 6-(2-Chloro-pyrimidin-4-yl)-2-(2-trimethylsilanyl-ethoxymethyl)-4-[1-(2-trimethylsilanyl-ethoxymethyl)-1H-benzimidazol-2-yl]-2H-pyridazin-3-on, 29,2 mg Cyclopropylboronsäure und 138 mg Kaliumphosphat werden in 1 ml Toluol und 0,05 ml Wasser gelöst und unter einer Argon Atmosphäre 17 mg 1,1'-Bis(diphenylphosphino)ferrocene Palladium(II)Chlorid (1:1 Komplex mit CH2Cl2) hinzugefügt. Die Mischung wird 9h bei 100°C gerührt. Nach Abkühlen auf Raumtemperatur wird mit 15 ml Wasser verdünnt, mehrfach mit Ethylacetat extrahiert und die vereinigten organischen Phasen nochmals mit gesättigter Natriumchlorid-Lösung extrahiert und mittels einer Varian Chem Elut CE 1005 cartridge getrocknet. Das Rohprodukt wird mittels RP HPLC (Acetonitril (+0.05%HCOOH) / Wasser (+0.05%HCOOH)) gereinigt.
Ausbeute 28.4 mg

### b) 4-(1H-Benzimidazol-2-yl)-6-(2-cyclopropyl-pyrimidin-4-yl)-2H-pyridazin-3-on

27 mg 6-(2-Cyclopropyl-pyrimidin-4-yl)-2-(2-trimethylsilanyl-ethoxymethyl)-4-[1-(2-trimethylsilanyl-ethoxymethyl)-1H-benzimidazol-2-yl]-2H-pyridazin-3-on werden in 2,5 ml 4M Salzsäurelösung in Dioxan und 1 ml Ethanol gelöst und 2 Tage bei Raumtemperatur gerührt. Anschließend wird das Lösungsmittel abdestilliert der Rückstand in 2 ml Dichlormethan und 1 ml Trifluoressigsäure gelöst und nochmals 3h bei Raumtemperatur gerührt. Anschließend wird wiederum abdestilliert und der Rückstand in 1 ml 2M Natriumhydroxidlösung und 2 ml Wasser gelöst und das Gemisch 10 h bei Raumtemperatur gerührt. Zur Aufarbeitung wird mit 2M Salzsäure neutralisiert, die flüchtigen Komponenten abdestilliert und das Rohprodukt mittels RP HPLC (Acetonitril (+0.05%HCOOH) / Wasser (+0.05%HCOOH)) gereinigt.
MS (ES+) m/z 331 (M+H).
Ausbeute 9.2 mg

### Beispiel 87

### 4-(1H-Benzimidazol-2-yl)-6-pyrimidin-2-yl-2H-pyridazin-3-on

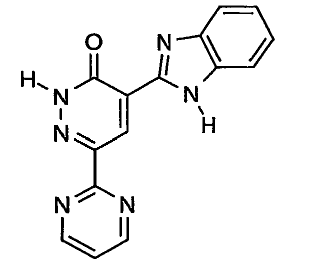

MS (ES+) m/z 291 (M+H).
Beispiel 87 wrd entsprechend Beispiel 55b synthetisiert.

### Beispiel 88

### 4-(1H-Benzimidazol-2-yl)-6-(4-methoxy-pyrimidin-2-yl)-2H-pyridazin-3-on

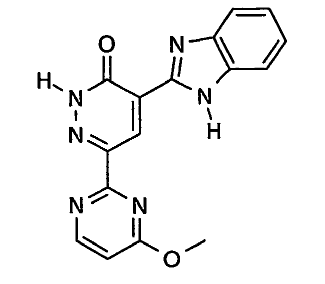

MS (ES+) m/z 321 (M+H).
Beispiel 88 wrd entsprechend Beispiel 55b synthetisiert.

### Beispiel 89

### 4-(1H-Benzimidazol-2-yl)-6-(4-dimethylamino-pyrimidin-2-yl)-2H-pyridazin-3-on

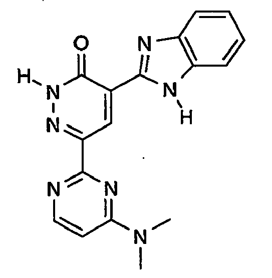

MS (ES+) m/z 334 (M+H).
Beispiel 89 wird entsprechend Beispiel 55b synthetisiert.

### Beispiel 90

### 4-(1H-Benzimidazol-2-yl)-6-[2-(2-diethylamino-ethylsulfanyl)-pyrimidin-4-yl]-2H-pyridazin-3-on

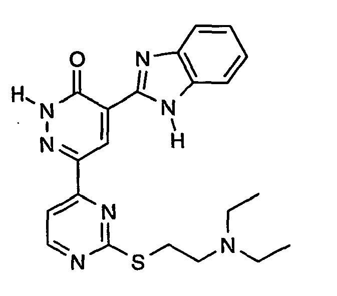

MS (ES+) m/z 422 (M+H).
Beispiel 90 wird entsprechend Beispiel 55 synthetisiert.

### Beispiel 91

### 4-(1H-Benzimidazol-2-yl)-6-[2-(2-hydroxy-1,1-dimethyl-ethylamino)-6-methylpyrimidin-4-yl]-2H-pyridazin-3-on

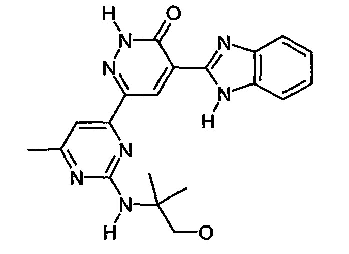

MS (ES+) m/z 392 (M+H).
Beispiel 91 wird entsprechend Beispiel 55 synthetisiert.

### Beispiel 92

### 4-(1H-Benzimidazol-2-yl)-6-(6-methyl-2-methylamino-pyrimidin-4-yl)-2H-pyridazin-3-on

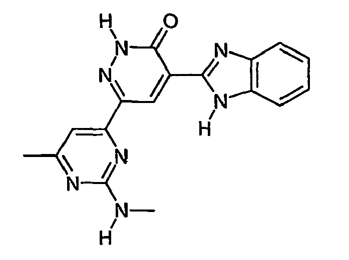

MS (ES+) m/z 334 (M+H).
Beispiel 92 wird entsprechend Beispiel 55 synthetisiert.

### Beispiel 93

### 6-(3,5-Dichloro-phenyl)-4-[6-(4-methyl-piperazin-1-ylmethyl)-1H-benzimidazol-2-yl]-2H- pyridazin-3-on

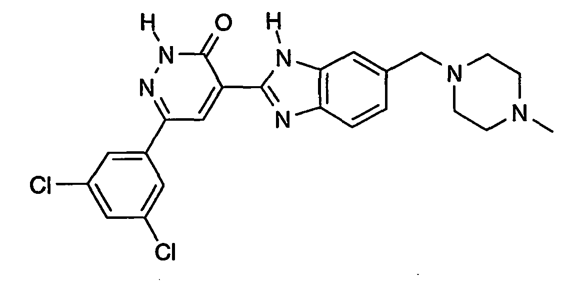

MS (ES+) m/z 470 (M+H).
Beispiel 93 wird entsprechend Beispiel 55 synthetisiert.

### Beispiel 94

### 4-(1H-Benzimidazol-2-yl)-6-{2-[methyl-(2-morpholin-4-yl-ethyl)-amino]-pyrimidin-4-yl}-2H-pyridazin-3-on

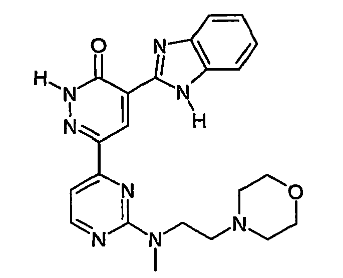

### a) 6-{2-[Methyl-(2-morpholin-4-yl-ethyl)-amino]-pyrimidin-4-yl}-2-(2-trimethylsilanyl- ethoxymethyl)-4-[1-(2-trimethylsilanyl-ethoxymethyl)-1H-benzimidazol-2-yl]-2H- pyridazin-3-on

86 mg 6-(2-Chloro-pyrimidin-4-yl)-2-(2-trimethylsilanyl-ethoxymethyl)-4-[1-(2-trimethylsilanyl-ethoxymethyl)-1H-benzimidazol-2-yl]-2H-pyridazin-3-on (55d) werden in 2 ml THF gelöst, bei 0°C 0.06 ml 2M Lithiumdüsopropylamin Lösung in TBF/Hexan hinzugetropft, 30 min bei 0°C gerührt und 0.008 ml Methyliodid dazu getropft. Nach 20h Rühren wird das Lösungsmittel abdestilliert und die Prozedur zur Vervollständigung des Umsatzes wiederholt. Anschließend wird mit Wasser verdünnt, mit Ethylacetat extrahiert und die vereinigten organischen Phasen getrocknet.

Mittels RPHPLC werden 17 mg eines Substanzgemischs isoliert, dass sowohl das gewünschte Produkt als auch ein Nebenprodukt enthält, das nur noch eine SEM Schutzgruppe trägt. Dieses Gemisch wird für die nächste Reaktion eingesetzt.

### b) 4-(1H-Benzimidazol-2-yl)-6-{2-[methyl-(2-morpholin-4-yl-ethyl)-amino]-pyrimidin-4-yl}-2H-pyridazin-3-on

Diese Verbindung wird aus dem unter 94a beschriebenen Produktgemisch wie für 55d beschrieben synthetisiert
Ausbeute 4.8 mg
MS (ES+) m/z 433 (M+H).

### Beispiel 95

### 4-(1H-Benzimidazol-2-yl)-6-pyrimidin-5-yl-2H-pyridazin-3-on

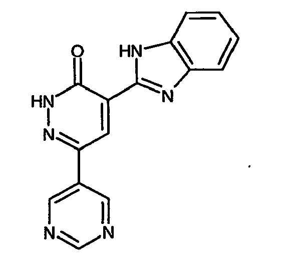

Beispiel 95 wird entsprechend Beispiel 55b synthetisiert.
MS (ES+) m/z 291 (M+H).

### Funktionelle Messungen zur Ermittlung von IC₅₀-Werten:

### GSK-3β

Die GSK-3β-Aktivität wird unter Verwendung von humaner rekombinanter GSK-3β und einem vorbehandelten (vorphosphorylierten) Substratpeptid (abgeleitet von Glykogensynthase und die Phosphorylierungsstellen 3a, b und c enthaltend) auf Grundlage der AlphaScreen Technology im 384-Well-Plattenformat (kleinvolumige Platte, weiss, Greiner) gemessen. In einem Endvolumen von 11 µl werden 2 µl der Verbindung (1 nM-100 mM im Kinasepuffer, DMSO wird auf 0,9 % konstant gehalten) 2 µl von GSK-3β-Lösung (0,18 nM) und 2 µl von biotinyliertem Phosphoglykogensynthasepeptid (34 nM) in Kinasepuffer (20 mM Hepes, pH 7,4, 10 mM MgCl, 200 mM EDTA, 1 mM DTT, 0,1 mg/ml BSA, 10 µM ATP) bei Raumtemperatur für 60 Minuten inkubiert. Nach der Zugabe von 2,5 ml Donor-Beads (20 µg/ml) und 2,5 ml Antibody (Anti-Phospho-GlykogenSynthase 1:2000)-gecoateter Akzeptor-Beads (40 µg/ml) in AlphaScreen Detektionspuffer (20 mM Hepes, pH 7,4, 10 mM MgCl, 40 mM EDTA, 1 mM DTT, 0,1 mg/ml BSA) werden die Platten bei Raumtemperatur (in der Dunkelheit!) über Nacht inkubiert und anschließend in einem Lesegerät (Alphaquest oder Fusion) platziert, um die End-Fluoreszenz zu messen. IC₅₀-Werte werden von der angepassten (fitted) Kurve bestimmt, die für die Blindwerte (Abwesenheit von GSK-3β) berichtigt und in dreifacher Ausführung durchgeführt wird.

| Beispiel Nr. | IC₅₀ [n M] |
|---|---|
| 6 | 16 |
| 21 | 57 |
| 25 | 56 |
| 38 | 32 |
| 42 | 7 |
| 43 | 1,5 |
| 46 | 28 |
| 50 | 157 |
| 53 | 42 |
| 58 | 50 |
| 68 | 63 |

## Patentansprüche

1. Verbindung der allgemeinen Formel (I) 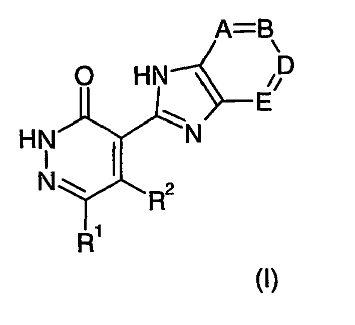
A ist CR³ oder N;
B ist CR⁴ oder N;
D ist CR⁵ oder N;
E ist CR⁶ oder N;
wobei maximal drei der Substituenten A, B, D und E gleichzeitig N sein können;
R¹ ist Halogen;
unsubstituiertes oder zumindest monosubstituiertes C₁-C₁₀-Alkyl,
wobei die Substituenten ausgewählt sind aus der Gruppe bestehend aus: Halogen, CN, NO₂, -OR⁷, -C(O)R⁷, -C(O)OR⁷, -O-C(O)R⁷, -NR⁷R⁸, -NHC(O)R⁷, -C(O)NR⁷R⁸, -NHC(S)R⁷, -C(S)NR⁷R⁸, -SR⁷, -S(O)R⁷,-SO₂R⁷, -NHSO₂R⁷, -SO₂NR⁷R⁸, -O-SO₂R⁷, -SO₂-O-R⁷, Aryl, Heteroaryl, Heterocyclyl, Trifluormethyl und Trifluormethoxy,
und Heterocyclyl, Aryl und Heteroaryl können wiederum mit C₁-C₆-Alkyl, C₁-C₆-Alkoxy, Halogen, Trifluormethyl, Trifluormethoxy oder OH zumindest monosubstituiert sein;
oder unsubstituiertes oder zumindest monosubstituiertes Heterocyclyl, Aryl oder Heteroaryl,
wobei die Substituenten ausgewählt sind aus der Gruppe bestehend aus: Halogen, -CN, NO₂, -CH₂-R⁷, -CH₂-NH₂, -CH₂-NH(C₁-C₆-Alkyl), -CH₂-N(C₁-C₆-Alkyl)₂, -CH₂-OH, -CH₂-O-(C₁₋C₆-Alkyl) -OR⁷, -C(O)R⁷,-C(O)OR⁷, -O-C(O)R⁷, -NR⁷R⁸, -NHC(O)R⁷, -C(O)NR⁷R⁸,-NHC(S)R⁷, -C(S)NR⁷R⁸, -SR⁷, -S(O)R⁷, -SO₂R⁷, -NHSO2R⁷,-SO₂NR⁷R⁸, -O-SO₂R⁷, -SO₂-O-R⁷, Aryl, Heteroaryl, Trifluormethyl und Trifluormethoxy,
und Aryl und Heteroaryl können wiederum mit C₁-C₆-Alkyl, C₁-C₆-Alkoxy, Halogen, Trifluormethyl, Trifluormethoxy oder OH zumindest monosubstituiert sein;
R² ist Wasserstoff oder C₁-C₁₀-Alkyl;
R³, R⁴, R⁵ und R⁶ sind unabhängig voneinander ausgewählt aus der Gruppe bestehend aus:
Wasserstoff, Halogen, -CN, NO₂, -CH₂-R⁸, -CH₂-NH₂, -CH₂-NH(C₁-C₆-Alkyl), -CH₂-N(C₁-C₆-Alkyl)₂, -CH₂-OH, -CH₂-O-(C₁-C₆-Alkyl),-OR⁸, -C(O)R⁸, -C(O)OR⁸, -O-C(O)R⁸, -NR⁷R⁸, -NHC(O)R⁸, -C(O)NR⁷R⁸,-NHC(S)R⁸, -C(S)NR⁷R⁸, -SR⁸, -S(O)R⁸, -SO₂R⁸, -NHSO₂R⁸, -SO₂NR⁷R⁸,-O-SO₂R⁸, -SO₂-O-R⁸, Aryl, Heteroaryl, Heterocyclyl, Trifluormethyl und Trifluormethoxy,
und Heterocyclyl, Aryl und Heteroaryl können wiederum mit C₁-C₆-Alkyl, C₁-C₆-Alkoxy, Oxo, Halogen, Trifluormethyl, Trifluormethoxy oder OH zumindest monosubstituiert sein;
R⁷ und R⁸ sind unabhängig voneinander:
H;
unsubstituiertes oder zumindest monosubstituiertes C₁-C₁₀-Alkyl, C₂-C₁₀-Alkenyl, C₂-C₁₀-Alkinyl, Heterocyclyl, Aryl oder Heteroaryl,
wobei die Substituenten ausgewählt sind aus der Gruppe bestehend aus: Heteroaryl, Heterocyclyl, Aryl, Oxo, Halogen, -OH, C₁-C₁₀-Alkoxy, (C₁-C₁₀-Alkyl)thio-, -C(O)OH, -C(O)O-(C₁-C₆-alkyl), -C(O)NH₂,-C(O)NH(C₁-C₆-Allcyl), -C(O)N(C₁-C₁₀-Alkyl)₂, Trifluormethyl, Trifluormethoxy, -CN, -NH₂, -NH(C₁-C₁₀-Alkyl) und -N(C₁-C₁₀-Alkyl)₂,
und Heterocyclyl, Aryl und Heteroaryl können wiederum mit C₁-C₆-Alkyl, C₁-C₆-Akoxy, Oxo, Trifluormethyl, Trifluormethoxy, Fluor, Chlor oder OH zumindest monosubstituiert sein;
Heteroaryl ist ein 5 bis 10-gliedriger, aromatischer, mono- oder bicyclischer Heterocyclus, der ein oder mehrere Heteroatome ausgewählt aus N, O und S enthält;
Aryl ist ein 5 bis 10-gliedriger, aromatischer, Mono- oder Bicyclus.
Heterocyclyl ist ein 5 bis 10-gliedriger, nicht-aromatischer, mono- oder bicyclischer Heterocyclus, der ein oder mehrere Heteroatome ausgewählt aus N, O und S enthält;
oder ein physiologisch verträgliches Salz davon;
unter der Voraussetzung, dass R¹ nicht unsubstituiertes oder zumindest mono-substituiertes Pyrazolo[1,5-a]pyridinyl ist.

2. Verbindung nach Anspruch 1, worin in der allgemeinen Formel (I) bedeuten:
A ist CR³;
B ist CR⁴ oder N;
D ist CR⁵ oder N;
E ist CR⁶;
R¹ ist Fluor; Chlor; Brom;
unsubstituiertes oder zumindest monosubstituiertes C₁-C₆-Akyl,
wobei die Substituenten ausgewählt sind aus der Gruppe bestehend aus: Halogen, -OR⁷, -C(O)R⁷, -C(O)OR⁷, -NR⁷H, -NR⁷(C₁-C₆-Alkyl), -C(O)NR⁷H, -SR⁷, Aryl, Heteroaryl, Heterocyclyl, Trifluormethyl und Trifluormethoxy,
und Heterocyclyl, Aryl und Heteroaryl können wiederum mit C₁-C₆-Alkyl, C₁-C₆-Alkoxy, Halogen, Trifluormethyl, Trifluormethoxy oder OH zumindest monosubstituiert sein;
oder unsubstituiertes oder zumindest monosubstituiertes Heterocyclyl, Aryl oder Heteroaryl,
wobei die Substituenten ausgewählt sind aus der Gruppe bestehend aus: Halogen, -CH₂-R⁷, -CH₂-NH₂, -CH₂-NH(C₁-C₆-Alkyl), -CH₂-N(C₁-C₆-Alkyl)₂, -CH₂-OH, -CH₂-O-(C₁-C₆-Alkyl), -OR⁷, -C(O)R⁷, -C(O)OR⁷,-NR⁷H, -NR⁷(C₁-C₆-Alkyl-), -C(O)NR⁷H, -SR⁷, Aryl, Heteroaryl, Trifluormethyl und Trifluormethoxy,
und Aryl und Heteroaryl können wiederum mit C₁-C₆-Alkyl, C₁-C₆-Alkoxy, Halogen, Trifluormethyl, Trifluormethoxy oder OH zumindest monosubstituiert sein;
R² ist Wasserstoff oder C₁-C₆-Alkyl;
R³, R⁴, R⁵ und R⁶ sind unabhängig voneinander ausgewählt aus der Gruppe bestehend aus:
Wasserstoff, Halogen, -CN, -CH₂-R⁸, -CH₂-NH₂, -CH₂-NH(C₁-C₆-Alkyl),-CH₂-N(C₁-C₆-Alkyl)₂, -CH₂-OH, -CH₂-O-(C₁-C₆-Alkyl); -OR⁸, -C(O)R⁸,-C(O)OR⁸, -NR⁸H, -NR⁸(C₁-C₆-Alkyl), -C(O)NR⁸H, -SR⁸, -SO₂NR⁸H,-SO₂-R⁸, Aryl, Heteroaryl, Heterocyclyl, Trifluormethyl und Trifluormethoxy,
und Heterocyclyl, Aryl und Heteroaryl können wiederum mit C₁-C₆-Alkyl, C₁-C₆-Alkoxy, Oxo, Halogen, Trifluormethyl, Trifluormethoxy oder OH zumindest monosubstituiert sein;
R⁷ und R⁸ sind unabhängig voneinander:
H;
unsubstituiertes oder zumindest monosubstituiertes C₁-C₆-Alkyl, Heterocyclyl, Phenyl oder Heteroaryl,
wobei die Substituenten ausgewählt sind aus der Gruppe bestehend aus: Heteroaryl, Heterocyclyl, Phenyl, Fluor, Chlor, Brom, OH, C₁-C₆-Alkoxy,-C(O)OH, -C(O)O-(C₁-C₆-alkyl), -C(O)NH₂, -C(O)NH(C₁C₆-Alkyl),-C(O)N(C₁-C₁₀-Alkyl)₂, Trifluormethyl, Trifluormethoxy, -NH₂, -NH(C₁-C₆-Alkyl) und -N(C₁-C₆-akyl)₂,
und Heterocyclyl, Phenyl und Heteroaryl können wiederum mit C₁-C₃-Alkyl, C₁-C₃-Alkoxy, Oxo, Trifluormethyl, Trifluormethoxy, Fluor, Chlor oder OH zumindest monosubstituiert sein;
Heteroaryl ist Imidazolyl, Thiophenyl, Furanyl, Thiazolyl, Imidazolyl, Oxazolyl, Isoxazolyl, Pyridinyl, Pyrimidinyl, Pyrazolyl, Benzo[b]thiophenyl, Thiazolo[3,2-b][1,2,4]-triazolyl, Pyrrolyl, Chinolinyl, Isochinolinyl, 1,2,3,4-Tetrahydrochinolinyl, Benzimidazolyl, Indolyl oder 1,3-Benzodioxolyl;
Aryl ist Naphthyl, Indanyl oder Phenyl;
Heterocyclyl ist 2-Oxo-azepanyl, 1,4-Oxazepanyl, Dihydropyridinonyl, Tetrahydrofuranyl, 1,3-Dioxolanyl, Morpholinyl, Pyrrolidinyl, Piperazinyl oder Piperidinyl;
oder ein physiologisch verträgliches Salz davon.

3. Verbindung nach Anspruch 1 oder 2, worin in der allgemeinen Formel (I) bedeuten:
A ist CR³;
B ist CR⁴ oder N;
D ist CR⁵;
E ist CR⁶;
R¹ ist Chlor;
unsubstituiertes oder zumindest monosubstituiertes C₁-C₆-Alkyl,
wobei die Substituenten ausgewählt sind aus der Gruppe bestehend aus: Fluor, Chlor, -OH, C₁-C₆-Alkoxy, -NH₂, -NH(C₁-C₆-Alkyl), -N(C₁-C₆-Alkyl)₂, Heterocylyl-(C₁-C₆-alkyl)-NH-, Aryl-(C₁-C₆-alkyl)-NH-, Heterocyclyl, Aryl und Heteroaryl,
und die Heterocyclyl-, Aryl- und Heteroaryl-Fragmente dieser Substituenten können wiederum mit C₁-C₄-Alkyl, C₁-C₄-Alkoxy, Fluor, Chlor, Trifluormethyl, Trifluormethoxy oder OH zumindest monosubstituiert sein;
oder unsubstituiertes oder zumindest monosubstituiertes Phenyl, Pyridinyl, Pyrimidinyl, Pyrazolyl, Thiophenyl, Oxazolyl, Isoxazolyl, Thiazolyl, Imidazolyl, Morpholinyl, Dihydropyridinonyl, Benzo[b]thiophenyl, 1,3-Benzodioxolyl oder Thiazolo[3,2-b][1,2,4]-triazolyl,
wobei die Substituenten ausgewählt sind aus der Gruppe bestehend aus: Halogen, -CH₂-R⁷, -CH₂-NH₂, -CH₂-NH(C₁-C₆-Alkyl), -CH₂-N(C₁-C₆-Alkyl)₂, -CH₂-OH, -CH₂-O-(C₁-C₆-Alkyl), -OR⁷, -C(O)R⁷, -C(O)OR⁷,-NR⁷H, -NR⁷(C₁-C₆-Alkyl-), -C(O)NR⁷H, -SR⁷, Aryl, Heteroaryl, Trifluormethyl und Trifluormethoxy,
und Aryl und Heteroaryl können wiederum mit C₁-C₆-Alkyl, C₁-C₆-Alkoxy, Halogen, Trifluormethyl, Trifluormethoxy oder OH zumindest monosubstituiert sein;
R² ist Wasserstoff;
R³, R⁴, R⁵ und R⁶ sind unabhängig voneinander ausgewählt aus der Gruppe bestehend aus:
Wasserstoff, Fluor, Chlor, Brom, -CN, -CH₂-R⁸, -CH₂-NH₂, -CH₂-NH(C₁-C₆-Alkyl), -CH₂-N(C₁-C₆-Alkyl)₂, -CH₂-OH, -CH₂-O-(C₁-C₆-Alkyl), -OR⁸, -C(O)R⁸, -C(O)OR⁸, -NR⁸H, -NR⁸(C₁-C₆-Alkyl), -C(O)NR⁸H, -SR⁸,-SO₂NR⁸H, SO₂-R⁸, Heterocyclyl, Trifluormethyl und Trifluormethoxy,
und Heterocyclyl kann wiederum mit C₁-C₆-Alkyl, C₁-C₆-Alkoxy, Halogen, Trifluormethyl, Trifluormethoxy oder OH zumindest monosubstituiert sein;
R⁷ und R⁸ sind unabhängig voneinander:
H;
unsubstituiertes oder zumindest monosubstituiertes C₁-C₆-Alkyl, Heterocyclyl, Phenyl oder Heteroaryl,
wobei die Substituenten ausgewählt sind aus der Gruppe bestehend aus: Heteroaryl, Heterocyclyl, Phenyl, Fluor, Chlor, Brom, OH, C₁-C₆-Alkoxy,-C(O)OH, -C(O)O-(C₁-C₆-alkyl), -C(O)NH₂, -C(O)NH(C₁-C₆-Alkyl),-C(O)N(C₁-Cₗₒ-Alkyl)₂, Trifluormethyl, Trifluormethoxy, -NH₂, -NH(C₁-C₆-Alkyl) und -N(C₁-C₆-alkyl)₂,
und Heterocyclyl, Phenyl und Heteroaryl können wiederum mit C₁-C₃-Alkyl, C₁-C₃-Alkoxy, Oxo, Trifluormethyl, Trifluormethoxy, Fluor, Chlor oder OH zumindest monosubstituiert sein;
Heteroaryl ist Imidazolyl, Thiophenyl, Furanyl, Thiazolyl, Imidazolyl, Oxazolyl, Isoxazolyl, Pyridinyl, Pyrimidinyl, Pyrazolyl, Benzo[b]thiophenyl, Thiazolo[3,2-b][1,2,4]-triazolyl, Pyrrolyl, Chinolinyl, Isochinolinyl, 1,2,3,4-Tetrahydrochinolinyl, Benzimidazolyl, Indolyl oder 1,3-Benzodioxolyl;
Aryl ist Naphthyl, Indanyl oder Phenyl;
Heterocyclyl ist 2-Oxo-azepanyl, Tetrahydrofuranyl, 1,4-Oxazepanyl, Dihydropyridinonyl, 1,3-Dioxolanyl, Morpholinyl, Pyrrolidinyl, Piperazinyl oder Piperidinyl;
oder ein physiologisch verträgliches Salz davon.

4. Verbindung nach einem der Ansprüch 1 bis 3, worin in der allgemeinen Formel (I) bedeuten:
A ist CR³;
B ist CR⁴ oder N;
D ist CR⁵;
E ist CR⁶;
R¹ ist unsubstituiertes oder zumindest monosubstituiertes Phenyl, Pyridinyl, Pyrimidinyl, Pyrazolyl, Thiophenyl, Oxazolyl, Isoxazolyl, Thiazolyl, Imidazolyl, Morpholinyl, Dihydropyridinonyl, Benzo[b]thiophenyl, Benzodioxolyl oder Thiazolo[3,2-b][1,2,4]-triazolyl,
wobei die Substituenten ausgewählt sind aus der Gruppe bestehend aus: Halogen, C₁-C₆-Alkyl, Phenyl-(C₁-C₆-alkyl)-, -OH, C₁-C₆-Alkoxy, (C₁-C₆-Alkyl)thio-, -O-Phenyl, -NH₂, -N(C₁-C₆-Alkyl)₂, -NH(C₁-C₆-Akyl), HO(O)C-(C₁-C₆-Alkyl)-NH-, (C₁-C₆-Alkyl)-O(O)C-(C₁-C₆-alkyl)-NH-, H₂N(O)C-(C₁-C₆-Alkyl)-NH-, (C₁-C₆-Alkyl)HN(O)C-(C₁-C₆-alkyl)-NH-H₂N-(C₁-C₆-Alkyl)-, (C₁-C₆-Alkyl)HN-(C₁-C₆-alkyl)-, (C₁-C₆-Alkyl)₂N-(C₁-C₆-alkyl)-, Heterocyclyl-(C₁-C₆-Alkyl)-O-, H₂N-(C₁-C₆-alkyl)-NH-; (C₁-C₆-Alkyl)HN-(C₁-C₆-alkyl)-NH-, (C₁-C₆-Alkyl)₂N-(C₁-C₆-alkyl)-NH-Heterocyclyl-(C₁-C₆-alkyl)-NH-, Heteroaryl-(C₁-C₆-alkyl)-NH-, Phenyl-(C₁-C₆-alkyl)-NH-, (C₁-C₆-Alkyl)-O-(C₁-C₆-alkyl)-NH-, HO-(C₁-C₆-Alkyl)-NH-, Heterocyclyl-(C₁-C₆-alkyl)-, Trifluormethyl, Trifluormethoxy, Phenyl und Heteroaryl,
und die Heterocyclyl-, Phenyl- und Heteroaryl-Fragmente dieser Substituenten können wiederum mit C₁-C₃-Alkyl, C₁-C₃-Alkoxy, Fluor, Chlor, Trifluormethyl, Trifluormethoxy oder OH zumindest monosubstituiert sein;
R² ist Wasserstoff;
R³, R⁴, R⁵ und R⁶ sind unabhängig voneinander ausgewählt aus der Gruppe bestehend aus:
Wasserstoff, Fluor, Chlor, Brom, -CN, C₁-C₆-Alkyl, -OH, C₁-C₆-Alkoxy,-C(O)OH, -C(O)O-(C₁-C₆-Alkyl), -NH₂, -N(C₁-C₆-Alkyl)₂, -NH(C₁-C₆-Alkyl), H₂N-(C₁-C₆-alkyl)-NH-, Hydroxy-(C₁-C₆-alkyl)-NH-, (C₁-C₆-Alkyl)HN-(C₁-C₆-alkyl)-NH-, (C₁-C₆-Akyl)₂N-(C₁-C₆-akyl)-NH-, Heterocyclyl-(C₁-C₆-alkyl)-NH-, Heteroaryl-(C₁-C₆-alkyl)-NH-, Phenyl-(C₁-C₆-alkyl)-NH-, -C(O)NH₂, -C(O)N(C₁-C₆-Alkyl)₂, -C(O)NH(C₁-C₆-Alkyl), H₂N-(C₁-C₆-alkyl)-NHC(O)-, Hydroxy-(C₁-C₆-alkyl)-NHC(O)-, (C₁-C₆-Alkyl)HN-(C₁-C₆-alkyl)-NHC(O)-, (C₁-C₆-alkyl)₂N-(C₁-C₆-alkyl)-NHC(O)-, Heterocyclyl-(C₁-C₆-alkyl)-NHC(O)-, Heteroaryl-(C₁-C₆-alkyl)-NHC(O)-, Phenyl-(C₁-C₆-alkyl)-NHC(O)-, Heterocyclyl, Heterocyclyl-(C₁-C₆-alkyl)-, Trifluormethyl und Trifluormethoxy,
und die Heteroaryl-, Heterocyclyl- und Phenyl-Fragmente dieser Gruppe können wiederum mit C₁-C₆-Alkyl, C₁-C₆-Alkoxy, Halogen, Trifluormethyl, Trifluormethoxy oder OH zumindest monosubstituiert sein;
Heteroaryl ist Imidazolyl, Thiophenyl, Furanyl, Thiazolyl, Imidazolyl, Oxazolyl, Isoxazolyl, Pyridinyl, Pyrimidinyl, Pyrazolyl, Benzo[b]thiophenyl, Thiazolo[3,2-b][1,2,4]-triazolyl, Pyrrolyl, Chinolinyl, Isochinolinyl, 1,2,3,4-Tetrahydrochinolinyl, Benzimidazolyl, Indolyl oder 1,3-Benzdioxolyl;
Heterocyclyl ist 2-Oxo-azepanyl, 1,4-Oxazepanyl, Dihydropyridinonyl, Tetrahydrofuranyl, 1,3-Dioxolanyl, Morpholinyl, Pyrrolidinyl, Piperazinyl oder Piperidinyl;
oder ein physiologisch verträgliches Salz davon.

5. Verbindung nach einem der Ansprüche 1 bis 4, worin in der allgemeinen Formel (I) bedeuten:
A ist CR³;
B ist CR⁴ oder N;
D ist CR⁵_{;}
E ist CR⁶;
R¹ ist Pyridin-4-yl, 4-Hydroxy-3-methoxy-5-(methylaminomethyl)-phenyl, 2-Ethylamino-pyrimidin-4-yl, 3,5-Dimethyl-4-hydroxyphenyl, 2-(1-Phenylethylamino)-pyrimidin-4-yl, 2-(2-Morpholin-4-ylethylamino)-pyrimidin-4-yl, 2-Methylamino-pyrimidin-4-yl, 6-Methyl-2-(2-morpholin-4-ylethylamino)-pyrimidin-4-yl, 3-Methoxy-4-hydroxy-phenyl, 2-Methylsulfanyl-pyrimidin-4-yl oder 4-Butylamino-pyrimidin-4-yl.
R² ist Wasserstoff;
R³, R⁴, R⁵ und R⁶ sind unabhängig voneinander ausgewählt aus der Gruppe bestehend aus:
Wasserstoff, Fluor, Chlor, Brom, -CN, -C(O)O-Methyl, (2-Diethylaminoethyl)-NHC(O)-, COOH, Methoxy, Ethoxy, (2-Cyclohexylamino-ethyl)-NHC(O)-, (3-(4-Methyl-piperazin-1-yl)-propyl)-NHC(O)-, (3-Hydroxypropyl)-NHC(O)-, (3-Cyclohexylamino-propyl)-NHC(O)-, (3-Imidazol-1-yl-propyl)-NHC(O)-, Methyl, Ethyl, 4-Methylpiperazin-1-yl, Trifluormethyl und Trifluormethoxy;
oder ein physiologisch verträgliches Salz davon.

6. Verbindung nach einem der Ansprüche 1 bis 5, ausgewählt aus der Gruppe bestehend aus:
4-(1H-Benzimidazol-2-yl)-6-(4-hydroxy-3-methoxy-5-methylaminomethylphenyl)-2H- pyridazin-3-on, 2-(3-Oxo-6-pyridin-4-yl-2,3-dihydro-pyridazin-4-yl)-1H-benzimidazol-5-carbonsäure-(2-diethylamino-ethyl)-amid, 4-(6-Trifluormethyl-1H-benzimidazol-2-yl)-6-pyridin-4-yl-2H-pyridazin-3-on, 4-(6-Methoxy-1H-benzimidazol-2-yl)-6-pyridin-4-yl-2H-pyridazin-3-on, 4-(5-Chlor-1H-benzimidazol-2-yl)-6-methyl-2H-pyridazin-3-on, 4-(6-Chlor-1H-benzimidazol-2-yl)-6-(4-hydroxi-3,5-dimethyl-phenyl)-2H-pyridazin-3-on, 4-(5-Fluor-1H-benzimidazol-2-yl)-6-pyridin-4-yl-2H-pyridazin-3-on, 6-(4-Hydroxy-3-methoxyphenyl)-4-(6-trifluormethyl-IH-benzimidazol-2-yl)-2H-pyridazin-3-on, 6-(2-Butylamino-pyrimidin-4-yl)-4-(6-chlor-1H-benzimidazol-2-yl)-2H-pyridazin-3-on, 4-(1H-Benzimidazol-2-yl)-6-pyridin-4-yl-2H-pyridazin-3-on, 6-[2-(2-Morpholin-4-yl-ethylamino)-pyrimidin-4-yl]-4-(6-trifluormethyl-1H-benzimidazol-2-yl)-2H-pyridazin-3-on und 4-(3H-Imidazol[4,5-c]pyridin-2-yl)-6-pyridin-4-yl-2H-pyridazin-3-on;
oder ein physiologisch verträgliches Salz davon.

7. Verbindung nach einem der Ansprüche 1 bis 6 sowie deren physiologisch verträglichen Salze zur Verwendung als Arzneimittel.

8. Verwendung einer Verbindung nach einem der Ansprüche 1 bis 6 oder einem physiologisch verträglichen Salz davon zur Herstellung von einem Medikament, das ein Inhibitor von GSK-3β ist.

9. Verwendung einer Verbindung nach einem der Ansprüche 1 bis 6 oder einem physiologisch verträglichen Salz davon zur Herstellung von einem Medikament zur Prophylaxe und/oder Behandlung von neurodegenerativen Erkrankungen, Schlaganfällen, Schädel- und Rückenmarksverletzungen und peripheren Neuropathien, Obesitas, metabolischen Erkrankungen, Typ-II-Diabetes, essentieller Hypertonie, atherosklerotischen Herzkreislauferkrankungen, Stein-Leventhal-Syndrom, Syndrom X, Immundefekten oder Krebs.

10. Verwendung gemäß Anspruch 9, **dadurch gekennzeichnet, dass** es sich bei den neurodegenerativen Erkrankungen um Alzheimer-Krankheit, Parkinson-Krankheit, frontoparietaler Demenz, kortikobasaler Degeneration oder Pick-Krankheit handelt.

11. Verwendung gemäß Anspruch 9 zur Prophylaxe und/oder Behandlung von Typ-II-Diabetes oder Alzheimer-Krankheit.

12. Pharmazeutische Zusammensetzung umfassend eine wirksame Menge von mindestens einer Verbindung oder einem physiologisch verträglichen Salz davon gemäß einem der Ansprüche 1 bis 6 und einen physiologisch verträglichen Träger.

13. Pharmazeutische Zusammensetzung gemäß Anspruch 12, wobei die pharmazeutische Zusammensetzung in Form einer Pille, Tablette, beschichteten Tablette, Lutschtablette, Granulat, Kapsel, harten oder weichen Gelatinkapsel, wässrigen Lösung, alkoholischen Lösung, ölartigen Lösung, Sirup, Emulsion, Suspension, Zäpfchen, Pastille, Lösung zur Injektion oder Infusion, Salbe, Tinktur, Creme, Lotion, Puder, Spray, transdermalen therapeutischen Systems, Nasenspray, Aerosol, Aerosol-Mischung, Mikrokapsel, Implantat, Stab oder Pflaster vorliegt.

14. Verfahren zur Herstellung einer Verbindung nach einem der Ansprüche 1 bis 6, **dadurch gekennzeichnet, dass** eine Verbindung der allgemeinen Formel (II) 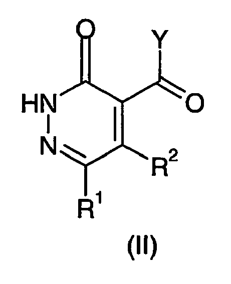 mit einer Verbindung gemäß der allgemeinen Formel (III) 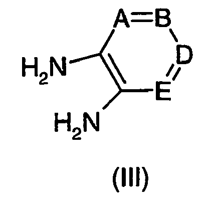 umgesetzt wird, wobei
a) Y eine Austrittsgruppe ist und die. Zyklisierung unter Verwendung einer Säure oder eines Wasser entziehenden Mittels erfolgt oder
b) Y gleich H ist und die Zyklisierung durch Oxidation, insbesondere durch Sauerstoff erfolgt,
und gegebenenfalls die Substituenten A, B, D, E, R¹ oder R² im Anschluss an die Zyklisierung modifiziert werden.

15. Verfahren zur Herstellung einer Verbindung nach einem der Ansprüche 1 bis 6, **dadurch gekennzeichnet, dass** eine Verbindung der allgemeinen Formel (VI) mit einer Verbindung gemäß der allgemeinen Formel (V) in Gegenwart eines Palladiumkomplexes zu einer Verbindung der Formal (Ia), gegebenenfalls mit anschließender Abspaltung der Schutzgruppe Y2, umgesetzt wird, 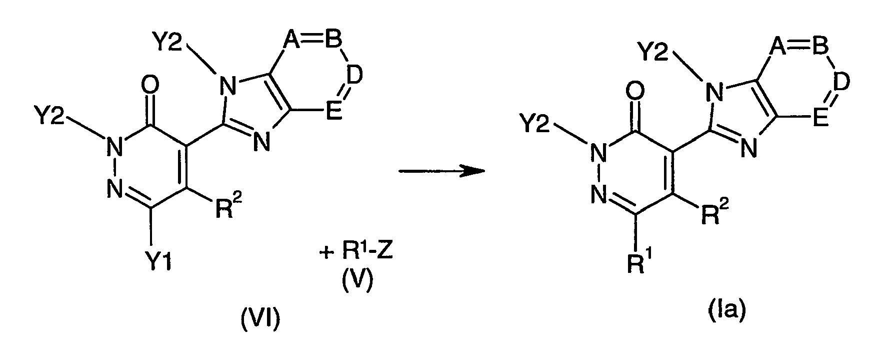 worin
Y1 gleich Halogen, B(OH)₂ oder Sn(C₁-C₁₀-alkyl) und Y2 gleich H oder eine Schutzgruppe sind,
und Z gleich B(OH)₂, B(C₁-C₁₀-alkoxy)₂, Sn(C₁-C₁₀-akyl)₃, Zn-(C₁-C₁₀-alkyl) oder Halogen und R¹ gleich unsubstituiertes oder zumindest monosubstituiertes Aryl oder Heteroaryl sind.

## Claims

1. A compound of the formula (I) 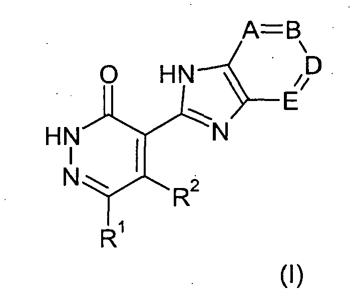
A is CR³ or N;
B is CR⁴ or N;
D is CR⁵ or N;
E is CR⁶ or N;
where a maximum of three of the substituents A, B, D and E can simultaneously be N;
R¹ is halogen;
unsubstituted or at least monosubstituted C₁-C₁₀-alkyl,
where the substituents are selected from the group consisting of: halogen, CN, NO₂, -OR⁷, -C(O)R⁷, -C(O)OR⁷, -O-C(O)R⁷, -NR⁷R⁸, -NHC(O)R⁷, -C(O)NR⁷R⁸, -NHC(S)R⁷, -C(S)NR⁷R⁸, -SR⁷, -S(O)R⁷, -SO₂R⁷, -NHSO₂R⁷, -SO₂N⁷R⁸, -O-SO₂R⁷, -SO₂-O-R⁷, aryl, heteroaryl, heterocyclyl, trifluoromethyl and trifluoromethoxy,
and heterocyclyl, aryl and heteroaryl may in turn be at least monosubstituted by C₁-C₆-alkyl, C₁-C₆-alkoxy, halogen, trifluoromethyl, trifluoromethoxy or OH;
or unsubstituted or at least monosubstituted heterocyclyl, aryl or heteroaryl,
where the substituents are selected from the group consisting of: halogen, -CN, NO₂, -CH₂-R⁷, -CH₂-NH₂, -CH₂-NH(C₁-C₆-alkyl), -CH₂-N(C₁-C₆-alkyl)₂, -CH₂-OH, -CH₂-O-(C₁-C₆-alkyl), -OR⁷, -C(O)R⁷, -C(O)OR⁷, -O-C(O)R⁷, -NR⁷R⁸, -NHC(O)R⁷, -C(O)NR⁷R⁸, -NHC(S)R⁷, -C(S)NR⁷R⁸, -SR⁷, -S(O)R⁷, -SO₂R⁷ , -NHSO₂R⁷, -SO₂NR⁷R⁸, -O-SO₂R⁷, -SO₂-O-R⁷, aryl, heteroaryl, trifluoromethyl and trifluoromethoxy,
and aryl and heteroaryl may in turn be at least monosubstituted by C₁-C₆-alkyl, C₁-C₆-alkoxy, halogen, trifluoromethyl, trifluoromethoxy or OH;
R² is hydrogen or C₁-C₁₀-alkyl;
R³, R⁴, R⁵ and R⁶ are independently of one another selected from the group consisting of:
hydrogen, halogen, -CN, NO₂, -CH₂-R⁸ , -CH₂-NH₂, -CH₂-NH(C₁-C₆-alkyl), -CH₂-N(C₁-C₆-alkyl)₂, -CH₂-OH, -CH₂-O-(C₁-C₆-alkyl), -OR⁸, -C(O)R⁸, -C(O)OR⁸, -O-C(O)R⁸, -NR⁷R⁸, -NHC(O)R⁸, -C(O)NR⁷R⁸, -NHC(S)R⁸, -C(S)NR⁷R⁸, -SR⁸, -S(O)R⁸, -SO₂R⁸, -NHSO₂R⁸, -SO₂NR⁷ R⁸ , -O-SO₂R⁸, -SO₂-O-R⁸, aryl, heteroaryl, heterocyclyl, trifluoromethyl and trifluoromethoxy,
and heterocyclyl, aryl and heteroaryl may in turn be at least monosubstituted by C₁-C₆-alkyl, C₁-C₆-alkoxy, oxo, halogen, trifluoromethyl, trifluoromethoxy or OH;
R⁷ and R ⁸ are independently of one another:
H;
unsubstituted or at least monosubstituted C₁-C₁₀-alkyl, C₂-C₁₀-alkenyl, C₂-C₁₀-alkynyl, heterocyclyl, aryl or heteroaryl,
where the substituents are selected from the group consisting of: heteroaryl, heterocyclyl, aryl, oxo, halogen, -OH, C₁-C₁₀-alkoxy, (C₁-C₁₀-alkyl)thio-, -C(O)OH, -C(O)O-(C₁-C₆-alkyl), -C(O)NH₂, -C(O)NH(C₁-C₆-alkyl), -C(O)N(C₁-C₁₀-alkyl)₂, trifluoromethyl, trifluoromethoxy, -CN, -NH₂, -NH(C₁-C₁₀-alkyl) and -N(C₁-C₁₀-alkyl)₂,
and heterocyclyl, aryl and heteroaryl may in turn be at least monosubstituted by C₁-C₆-alkyl, C₁-C₆-alkoxy, oxo, trifluoromethyl, trifluoromethoxy, fluorine, chlorine or OH;
heteroaryl is a 5 to 10-membered, aromatic, mono- or bicyclic heterocycle which comprises one or more heteroatoms selected from N, O and S;
aryl is a 5 to 10-membered, aromatic, mono- or bicyclic system;
heterocyclyl is a 5 to 10-membered, nonaromatic, mono- or bicyclic heterocycle which comprises one or more heteroatoms selected from N, O and S;
or a physiologically tolerated salt thereof;
with the proviso that R¹ is not unsubstituted or is at least monosubstituted pyrazolo[1,5-a]pyridinyl.

2. A compound as claimed in claim 1, in which the meanings in the formula (I) are:
A is CR³;
B is CR⁴ or N;
D is CR⁵ or N;
E is CR⁶;
R¹ is fluorine; chlorine; bromine;
unsubstituted or at least monosubstituted C₁-C₆-alkyl,
where the substituents are selected from the group consisting of: halogen, -OR⁷, -C(O)R⁷, -C(O)OR ⁷, -NR⁷H, -NR⁷(C₁-C₆-alkyl), -C(O)NR⁷H, -SR⁷, aryl, heteroaryl, heterocyclyl, trifluoromethyl and trifluoromethoxy,
and heterocyclyl, aryl and heteroaryl may in turn be at least monosubstituted by C₁-C₆-alkyl, C₁-C₆-alkoxy, halogen, trifluoromethyl, trifluoromethoxy or OH;
or unsubstituted or at least monosubstituted heterocyclyl, aryl or heteroaryl,
where the substituents are selected from the group consisting of: halogen, -CH₂-R⁷, -CH₂-NH₂, -CH₂-NH(C₁-C₆-alkyl), -CH₂-N(C₁-C₆-alkyl)₂, -CH₂-OH, -CH₂-O-(C₁-C₆-alkyl), -OR⁷, -C(O)R⁷, -C(O)OR⁷, -NR⁷H, -NR ⁷(C₁-C₆-alkyl-), -C(O)NR⁷H, -SR⁷, aryl, heteroaryl, trifluoromethyl and trifluoromethoxy,
and aryl and heteroaryl may in turn be at least monosubstituted by C₁-C₆-alkyl, C₁-C₆-alkoxy, halogen, trifluoromethyl, trifluoromethoxy or OH;
R² is hydrogen or C₁-C₆-alkyl;
R³, R⁴, R⁵ and R⁶ are independently of one another selected from the group consisting of:
hydrogen, halogen, -CN, -CH₂-R⁸, -CH₂-NH₂, -CH₂-NH(C₁-C₆-alkyl), -CH₂-N(C₁-C₆-alkyl)₂, -CH₂-OH, -CH2-O-(C₁-C₆-alkyl) -OR⁸, -C(O)R ⁸ -C(O)OR⁸, -NR⁸H, -NR⁸(C₁-C₆-alkyl), -C(O)NR⁸H, -SR ⁸ -SO₂NR⁸H, -SO₂-R⁸, aryl, heteroaryl, heterocyclyl, trifluoromethyl and trifluoromethoxy,
and heterocyclyl, aryl and heteroaryl may in turn be at least monosubstituted by C₁-C₆-alkyl, C₁-C₆-alkoxy, oxo, halogen, trifluoromethyl, trifluoromethoxy or OH;
R⁷ and R⁸ are independently of one another:
H;
unsubstituted or at least monosubstituted C₁-C₆-alkyl, heterocyclyl, phenyl or heteroaryl,
where the substituents are selected from the group consisting of: heteroaryl, heterocyclyl, phenyl, fluorine, chlorine, bromine, OH, C₁-C₆-alkoxy, -C(O)OH, -C(O)O-(C₁-C₆-alkyl), -C(O)NH₂, -C(O)NH(C₁-C₆-alkyl), -C(O)N(C₁-C₁₀-alkyl)₂, trifluoromethyl, trifluoromethoxy, -NH₂, -NH(C₁-C₆-alkyl) and -N(C₁-C₆-alkyl)₂,
and heterocyclyl, phenyl and heteroaryl may in turn be at least monosubstituted by C₁-C₃-alkyl, C₁-C₃-alkoxy, oxo, trifluoromethyl, trifluoromethoxy, fluorine, chlorine or OH;
heteroaryl is imidazolyl, thiophenyl, furanyl, thiazolyl, imidazolyl, oxazolyl, isoxazolyl, pyridinyl, pyrimidinyl, pyrazolyl, benzo[b]thiophenyl, thiazolo[3,2-b][1,2,4]-triazolyl, pyrrolyl, quinolinyl, isoquinolinyl, 1,2,3,4-tetrahydroquinolinyl, benzimidazolyl, indolyl or 1,3-benzodioxolyl;
aryl is naphthyl, indanyl or phenyl;
heterocyclyl is 2-oxoazepanyl, 1,4-oxazepanyl, dihydropyridinonyl, tetrahydrofuranyl, 1,3-dioxolanyl, morpholinyl, pyrrolidinyl, piperazinyl or piperidinyl;
or a physiologically tolerated salt thereof.

3. A compound as claimed in claim 1 or 2, in which the meanings in the formula (I) are:
A is CR³;
B is CR⁴ or N;
D is CR⁵;
E is CR⁶;
R¹ is chlorine;
unsubstituted or at least monosubstituted C₁-C₆-alkyl,
where the substituents are selected from the group consisting of: fluorine, chlorine, -OH, C₁-C₆-alkoxy, -NH₂, -NH(C₁-C₆-alkyl), -N(C₁-C₆-alkyl)₂, heterocyclyl-(C₁-C₆-alkyl)-NH-, aryl-(C₁-C₆-alkyl)-NH-, heterocyclyl, aryl and heteroaryl,
and the heterocyclyl, aryl and heteroaryl fragments of these substituents may in turn be at least monosubstituted by C₁-C₄-alkyl, C₁-C₄-alkoxy, fluorine, chlorine, trifluoromethyl, trifluoromethoxy or OH;
or unsubstituted or at least monosubstituted phenyl, pyridinyl, pyrimidinyl, pyrazolyl, thiophenyl, oxazolyl, isoxazolyl, thiazolyl, imidazolyl, morpholinyl, dihydropyridinonyl, benzo[b]thiophenyl, 1,3-benzodioxolyl or thiazolo[3,2-b][1,2,4]-triazolyl,
where the substituents are selected from the group consisting of: halogen, -CH₂-R⁷ , -CH₂-NH₂, -CH₂-NH(C₁-C₆-alkyl), -CH₂-N(C₁-C₆-alkyl)₂, -CH₂OH, -CH₂-O-(C₁-C₆-alkyl), -OR⁷, (O)R⁷, -C(O)OR⁷, -NR⁷H, -NR⁷(C₁-C₆-alkyl-), -C(O)NR⁷H, -SR⁷ , aryl, heteroaryl, trifluoromethyl and trifluoromethoxy,
and aryl and heteroaryl may in turn be at least monosubstituted by C₁-C₆-alkyl, C₁-C₆-alkoxy, halogen, trifluoromethyl, trifluoromethoxy or OH;
R² is hydrogen;
R³, R⁴, R⁵ and R⁶ are independently of one another selected from the group consisting of:
hydrogen, fluorine, chlorine, bromine, -CN, -CH₂-R⁸, -CH₂-NH₂, -CH₂-NH(C₁-C₆-alkyl), -CH₂-N(C₁-C₆-alkyl)₂, -CH₂-OH, -CH₂-O-(C₁-C₆-alkyl), -OR⁸, -C(O)R⁸, -C(O)OR⁸; -NR⁸H, -NR⁸(C₁-C₆-alkyl), -C(O)NR⁸H, -SR⁸, -SO₂NR⁸H, SO₂-R⁸, heterocyclyl, trifluoromethyl and trifluoromethoxy,
and heterocyclyl may in turn be at least monosubstituted by C₁-C₆-alkyl, C₁-C₆-alkoxy, halogen, trifluoromethyl, trifluoromethoxy or OH;
R⁷ and R⁸ are independently of one another:
H;
unsubstituted or at least monosubstituted C₁-C₆-alkyl, heterocyclyl, phenyl or heteroaryl,
where the substituents are selected from the group consisting of: heteroaryl, heterocyclyl, phenyl, fluorine, chlorine, bromine, OH, C₁-C₆-alkoxy, -C(O)OH; -C(O)O-(C₁-C₆-alkyl), -C(O)NH₂, -C(O)NH(C₁-C₆-alkyl), -C(O)N(C₁-C₁₀-alkyl)₂, trifluoromethyl, trifluoromethoxy, -NH₂, -NH(C₁-C₆-alkyl) and -N(C₁-C₆-alkyl)₂,
and heterocyclyl, phenyl and heteroaryl may in turn be at least monosubstituted by C₁-C₃-alkyl, C₁-C₃-alkoxy, oxo, trifluoromethyl, trifluoromethoxy, fluorine, chlorine or OH;
heteroaryl is imidazolyl, thiophenyl, furanyl, thiazolyl, imidazolyl, oxazolyl, isoxazolyl, pyridinyl, pyrimidinyl, pyrazolyl, benzo[b]thiophenyl, thiazolo[3,2-b)[1,2,4]-triazolyl, pyrrolyl, quinolinyl, isoquinolinyl, 1,2,3,4-tetrahydroquinolinyl, benzimidazolyl, indolyl or 1,3-benzodioxolyl;
aryl is naphthyl, indanyl or phenyl;
heterocyclyl is 2-oxoazepanyl, tetrahydrofuranyl, 1,4-oxazepanyl, dihydropyridinonyl, 1,3-dioxolanyl, morpholinyl, pyrrolidinyl, piperazinyl or piperidinyl;
or a physiologically tolerated salt thereof.

4. A compound as claimed in any of claims 1 to 3, in which the meanings in the formula (I) are:
A is CR³;
B is CR⁴ or N;
D is CR⁵;
E is CR⁶;
R¹ is unsubstituted or at least monosubstituted phenyl, pyridinyl, pyrimidinyl, pyrazolyl, thiophenyl, oxazolyl, isoxazolyl, thiazolyl, imidazolyl, morpholinyl, dihydropyridinonyl, benzo[b]thiophenyl, benzodioxolyl or thiazolo[3,2-b][1,2,4]-triazolyl, where the substituents are selected from the group consisting of: halogen, C₁-C₆-alkyl, phenyl-(C₁-C₆-alkyl)-, -OH, C₁-C₆-alkoxy, (C₁-C₆-alkyl)thio-, -O-phenyl, -NH₂, -N(C₁-C₆-alkyl)₂, -NH(C₁-C₆-alkyl), HO(O)C-(C₁-C₆-alkyl)-NH-, (C₁-C₆-alkyl)-O(O)C-(C₁-C₆-alkyl)-NH-, H₂N(O)C-(C₁-C₆-alkyl)-NH-, (C₁-C₆-alkyl)HN(O)C-(C₁-C₆-alkyl)-NH-, H₂N-(C₁-C₆-alkyl)-, (C₁-C₆-alkyl)HN-(C₁-C₆-alkyl)-, (C₁-C₆-alkyl)₂N-(C₁-C₆-alkyl)-, heterocyclyl-(C₁-C₆-alkyl)-O-, H₂N-(C₁-C₆-alkyl)-NH-, (C₁-C₆-alkyl)HN-(C₁-C₆-alkyl)-NH-, (C₁-C₆-alkyl)₂N-(C₁-C₆-alkyl)-NH-, heterocyclyl-(C₁-C₆-alkyl)-NH-, heteroaryl-(C₁-C₆-alkyl)-NH-, phenyl-(C₁-C₆-alkyl)-NH-, (C₁-C₆-alkyl)-O-(C₁-C₆-alkyl)-NH-, HO-(C₁-C₆-alkyl)-NH-, heterocyclyl-(C₁-C₆-alkyl)-, trifluoromethyl, trifluoromethoxy, phenyl and heteroaryl,
and the heterocyclyl, phenyl and heteroaryl fragments of these substituents may in turn be at least monosubstituted by C₁-C₃-alkyl, C₁-C₃-alkoxy, fluorine, chlorine, trifluoromethyl, trifluoromethoxy or OH;
R² is hydrogen;
R³, R⁴, R⁵ and R⁶ are independently of one another selected from the group consisting of:
hydrogen, fluorine, chlorine, bromine, -CN, C₁-C₆-alkyl, -OH, C₁-C₆-alkoxy, -C(O)OH, -C(O)O-(C₁-C₆-alkyl), -NH₂, -N(C₁-C₆-alkyl)₂, -NH(C₁-C₆-alkyl), H₂N-(C₁-C₆-alkyl)-NH-, hydroxy-(C₁-C₆-alkyl)-N H-, (C₁-C₆-alkyl)HN-(C₁-C₆-alkyl)-NH-, (C₁-C₆-alkyl)₂N-(C₁-C₆-alkyl)-NH-, (heterocyclyl-(C₁-C₆-alkyl)-NH-, (heteroaryl-(C₁-C₆-alkyl)-NH-, phenyl-(C₁-C₆-alkyl)-NH-, -C(O)NH₂, -C(O)N(C₁-C₆-alkyl)₂, -C(O)NH(C₁-C₆-alkyl), H₂N(C₁-C₆-alkyl)-NHC(O)-, hydroxy-(C₁-C₆-alkyl)-NHC(O)-, (C₁-C₆-alkyl)HN-(C₁-C₆-alkyl)-NHC(O)-, (C₁-C₆-alkyl)₂N-(C₁-C₆-alkyl)-NHC(O)-, heterocyclyl-(C₁-C₆-alkyl)-NHC(O)-, heteroaryl-(C₁-C₆-alkyl)-NHC(O)-, phenyl-(C₁-C₆-alkyl)-NHC(O)-, heterocyclyl, heterocyclyl-(C₁-C₆-alkyl), trifluoromethyl and trifluoromethoxy,
and the heteroaryl, heterocyclyl and phenyl fragments of this group may in turn be at least monosubstituted by C₁-C₆-alkyl, C₁-C₆-alkoxy, halogen, trifluoromethyl, trifluoromethoxy or OH;
heteroaryl is imidazolyl, thiophenyl, furanyl, thiazolyl, imidazolyl, oxazolyl, isoxazolyl, pyridinyl, pyrimidinyl, pyrazolyl, benzo[b]thiophenyl, thiazolo[3,2-b][1,2,4]-triazolyl, pyrrolyl, quinolinyl, isoquinolinyl, 1,2,3,4-tetrahydroquinolinyl, benzimidazolyl, indolyl or 1,3-benzodioxolyl;
heterocyclyl is 2-oxoazepanyl, 1,4-oxazepanyl, dihydropyridinonyl; tetrahydrofuranyl, 1,3-dioxolanyl, morpholinyl, pyrrolidinyl, piperazinyl or piperidinyl;
or a physiologically tolerated salt thereof.

5. A compound as claimed in any of claims 1 to 4, in which the meanings in the formula (I) are:
A is CR³;
B is CR⁴ or N;
D is CR⁵_{;}
E is CR⁶;
R¹ is pyridin-4-yl, 4-hydroxy-3-methoxy-5-(methylaminomethyl)phenyl, 2-ethylaminopyrimidin-4-yl, 3,5-dimethyl-4-hydroxyphenyl, 2-(1-phenylethylamino)pyrimidin-4-yl, 2-(2-morpholin-4-ylethylamino)pyrimidin-4-yl, 2-methylaminopyrimidin-4-yl, 6-methyl-2-(2-morpholin-4-ylethylamino)pyrimidin-4-yl, 3-methoxy-4-hydroxyphenyl, 2-methylsulfanylpyrimidin-4-yl or 4-butylaminopyrimidin-4-yl;
R² is hydrogen;
R³ R⁴, R⁵ and R⁶ are independently of one another selected from the group consisting of:
hydrogen, fluorine, chlorine, bromine, -CN, -C(O)O-methyl, (2-diethylaminoethyl)-NHC(O)-, COOH, methoxy, ethoxy, (2-cyclohexylaminoethyl)-NHC(O)-, (3-(4-methylpiperazin-1-yl)propyl)-NHC(O)-, (3-hydroxypropyl)-NHC(O)-, (3-cyclohexylaminopropyl)-NHC(O)-, (3-imidazbl-1-ylpropyl)-NHC(O)-, methyl, ethyl, 4-methylpiperazin-1-yl, trifluoromethyl and trifluoromethoxy;
or a physiologically tolerated salt thereof.

6. A compound as claimed in any of claims 1 to 5, selected from the group consisting of:
4-(1H-benzimidazol-2-yl)-6-(4-hydroxy-3-methoxy-5-methylaminomethylphenyl)-2H-pyridazin-3-one, 2-(3-oxo-6-pyridin-4-yl-2,3-dihydropyridazin-4-yl)-1H-benzimidazole-5-carboxylic acid (2-diethylaminoethyl)amide, 4-(6-trifluoromethyl-1H-benzimidazol-2-yl)-6-pyridin-4-yl-2H-pyridazin-3-one, 4-(6-methoxy-1H-benzimidazol-2-yl)-6-pyridin-4-yl-2H-pyridazin-3-one, 4-(5-chloro-1H-benzimidazol-2-yl)-6-methyl-2H-pyridazin-3-one, 4-(6-chloro-1H-benzimidazol-2-yl)-6-(4-hydroxy-3,5-dimethylphenyl)-2H-pyridazin-3-one, 4-(5-fluoro-1H-benzimidazol-2-yl)-6-pyridin-4-yl-2H-pyridazin-3-one, 6-(4-hydroxy-3-methoxyphenyl)-4-(6-trifluoromethyl-1H-benzimidazol-2-yl)-2H-pyridazin-3-one, 6-(2-butylaminopyrimidin-4-yl)-4-(6-chloro-1H-benzimidazol-2-yl)-2H-pyridazin-3-one, 4-(1H-benzimidazol-2-yl)-6-pyridin-4-yl-2H-pyridazin-3-one, 6-[2-(2-morpholin-4-ylethylamino)pyrimidin-4-yl]-4-(6-trifluoromethyl-1H-benzimidazol-2-yl)-2H-pyridazin-3-one and 4-(3H-imidazol[4,5-c]pyridin-2-yl)-6-pyridin-4-yl-2H-pyridazin-3-one;
or a physiologically tolerated salt thereof.

7. A compound as claimed in any of claims 1 to 6 and the physiologically tolerated salts thereof for use as pharmaceutical.

8. The use of a compound as claimed in any of claims 1 to 6 or of a physiologically tolerated salt thereof for producing a medicament which is an inhibitor of GSK-3β.

9. The use of a compound as claimed in any of claims 1 to 6 or of a physiologically tolerated salt thereof for producing a medicament for the prophylaxis and/or treatment of neurodegenerative diseases, strokes, cranial and spinal traumas and peripheral neuropathies, obesity, metabolic diseases, type II diabetes, essential hypertension, atherosclerotic cardiovascular diseases, polycystic ovary syndrome, syndrome X, immunodeficiency or cancer.

10. The use as claimed in claim 9, wherein the neurodegenerative diseases are Alzheimer's disease, Parkinson's disease, frontoparietal dementia, corticobasal degeneration or Pick's disease.

11. The use as claimed in claim 9 for the prophylaxis and/or treatment of type II diabetes or Alzheimer's disease.

12. A pharmaceutical composition comprising an effective amount of at least one compound or a physiologically tolerated salt thereof as claimed in any of claims 1 to 6 and a physiologically tolerated carrier.

13. A pharmaceutical composition as claimed in claim 12, where the pharmaceutical composition is in the form of a pill, tablet, coated tablet, lozenge, granule, capsule, hard or soft gelatin capsule, aqueous solution, alcoholic solution, oily solution, syrup, emulsion, suspension, suppository, pastille, solution for injection or infusion, ointment, tincture, cream, lotion, powder, spray, transdermal therapeutic systems, nasal spray, aerosol, aerosol mixture, microcapsule, implant, rod or patch.

14. A. process for preparing a compound as claimed in any of claims 1 to 6, which comprises reacting a compound of the formula (II) 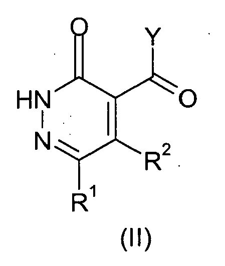 with a compound of the formula (III) 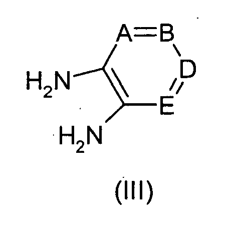 where
a) Y is a leaving group, and the cyclization takes place with use of an acid or of a dehydrating agent, or
b) Y equals H, and the cyclization takes place by oxidation, in particular by oxygen,
and where appropriate the substituents A, B, D, E, R¹ or R² are modified following the cyclization.

15. A process for preparing a compound as claimed in any of claims 1 to 6, which comprises reacting a compound of the formula (VI) with a compound of the formula (V) in the presence of a palladium complex to give a compound of the formula (Ia), where appropriate with subsequent elimination of the protective group Y2, 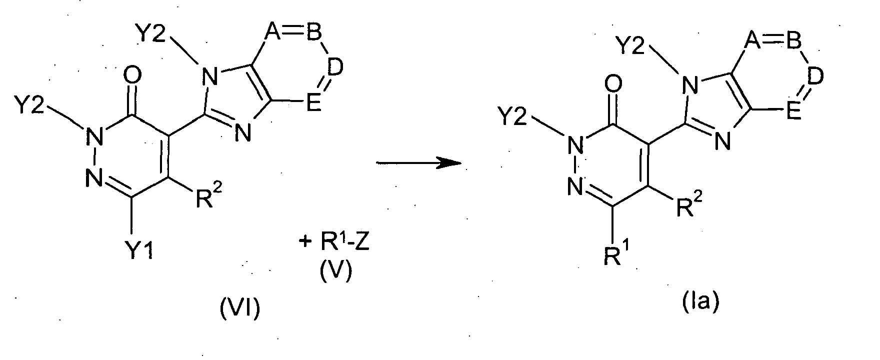 in which
Y1 equals halogen, B(OH)₂ or Sn(C₁-C₁₀-alkyl) and Y2 equals H or a protective group,
and Z equals B(OH)₂, B(C₁-C₁₀-alkoxy)₂, Sn(C₁-C₁₀-alkyl)₃, Zn-(C₁-C₁₀-alkyl) or halogen and R¹ equals unsubstituted or at least monosubstituted aryl or heteroaryl.

## Revendications

1. Composé de formule générale (I) 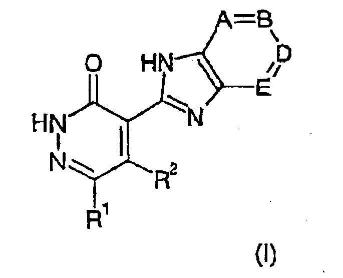 dans laquelle
A est CR³ ou N ;
B est CR⁴ ou N ;
D est CR⁵ ou N ;
E est CR⁶ OU N ;
trois des substituants A, B, D et E maximum pouvant être N simultanément ;
R¹ est un halogène;
un radical C₁-C₁₀-alkyle non substitué ou au moins monosubstitué,
les substituants étant sélectionnés dans le groupe composé d'un halogène, de CN, NO₂, -OR⁷, -C(O)R⁷, -C(O)OR⁷, -O-C(O)R⁷, -NR⁷R⁸, -NHC(O)R⁷, -C(O)NR⁷R⁸, -NHC(S)R⁷, -C (S)NR⁷R⁸, -SR⁷, -S(O)R⁷, -SO₂R⁷, -NHSO₂R⁷, -SO₂NR⁷R⁸, -O-SO₂R⁷, -SO₂-O-R⁷, aryle, hétéroaryle, hétérocyclyle, trifluorométhyle et trifluorométhoxy,
et les radicaux hétérocyclyle, aryle et hétéroaryle pouvant à leur tour être au moins monosubstitués avec un radical C₁-C₆-alkyle, C₁-C₆-alcoxy, un halogène, un radical trifluorométhyle, trifluorométhoxy ou OH ;
ou un radical hétérocyclyle, aryle ou hétéroaryle non substitué ou au moins monosubstitué,
les substituants étant sélectionnés dans le groupe composé d'un halogène, de -CN, NO₂, -CH₂-R⁷, -CH₂-NH₂, -CH₂-NH(C₁-C₆-alkyle), -CH₂-N (C₁-C₆-alkyle)₂, -CH₂-OH, -CH₂-O-(C₁-C₆-alkyle), -OR₇, -C(O)R⁷, -C(O)OR⁷, -O-C(O)R⁷, -NR⁷R⁸, -NHC(O)R⁷, -C(O)NR⁷R⁸, -NHC(S)R⁷, -C(S)NR⁷R⁸, -SR⁷, -S(O)R⁷, -SO₂R⁷, -NHSO₂R⁷, -SO₂NR⁷R⁸, -O-SO₂R⁷, -SO₂-O-R⁷, aryle, hétéroaryle, trifluorométhyle et trifluorométhoxy,
et les radicaux aryle et hétéroaryle pouvant à leur tour être au moins monosubstitués avec un radical C₁-C₆-alkyle, C₁-C₆-alcoxy, un halogène, un radical trifluorométhyle, trifluorométhoxy ou OH ;
R² est l'hydrogène ou un radical C₁-C₁₀-alkyle ;
R ³ R⁴, R⁵ et R⁶ sont sélectionnés, indépendamment les uns des autres, dans le groupe composé de :
l'hydrogène, un halogène, -CN, NO₂, -CH₂-R⁸, -CH₂-NH₂, -CH₂-NH(C₁-C₆-alkyle), -CH₂-N (C₁-C₆-alkyle) ₂, -CH₂-OH, -CH₂-O-(C₁-C₆-alkyle), -OR ₈ -C(O)R⁸, -C (O) OR⁸, -O-C(O)R⁸, -NR⁷R⁸ -NHC(O)R⁸, -C(O)NR⁷R⁸, -NHC(S)R⁸, -C(S)NR⁷R⁸, -SR⁸-S(O)R⁸, -SO₂R⁸, -NHSO₂R⁸, -SO₂NR⁷R⁸, -O-SO₂R⁸, -SO₂-O-R⁸, aryle, hétéroaryle, hétérocyclyle, trifluorométhyle et trifluorométhoxy,
les radicaux hétérocyclyle, aryle et hétéroaryle pouvant à leur tour être au moins monosubstitués avec un radical C₁-C₆-alkyle, C₁-C₆-alcoxy, oxo, un halogène, un radical trifluorométhyle, trifluorométhoxy ou OH ;
R⁷ et R⁸ sont, indépendamment l'un de l'autre :
H;
un radical C₁-C₁₀-alkyle, C₂-C₁₀-alcényle, C₂-C₁₀-alcynyle, hétérocyclyle, aryle ou hétéroaryle non substitué ou au moins monosubstitué,
les substituants étant sélectionnés dans le groupe composé d'un radical hétéroaryle, hétérocyclyle, aryle, oxo, d'un halogène, -OH, C₁-C₁₀-alcoxy, (C₁-C₁₀-alkyl) thio-, -C(O)OH, -C(O)O-(C₁-C₆-alkyle), -C(O)NH₂, -C(O)NH(C₁-C₆-alkyle), -C(O)N(C₁-C₁₀₋alkyle)₂, trifluorométhyle, trifluorométhoxy, -CN, -NH₂, -NH(C₁-C₁₀-alkyle) et -N(C₁-C₁₀-alkyle)₂,
les radicaux hétérocyclyle, aryle et hétéroaryle pouvant à leur tour être au moins monosubstitués avec un radical C₁-C₆-alkyle, C₁-C₆-alcoxy, oxo, trifluorométhyle, trifluorométhoxy, fluor, chlore ou OH ;
hétéroaryle est un hétérocycle aromatique, monocyclique ou bicyclique pentavalent à décavalent qui contient un ou plusieurs hétéroatomes sélectionnés parmi N, O et S ;
aryle est un monocycle ou bicycle aromatique pentavalent à décavalent ;
hétérocyclyle est un hétérocycle non aromatique, monocyclique ou bicyclique pentavalent à décavalent qui contient un ou plusieurs hétéroatomes sélectionnés parmi N, O et S;
ou un sel physiologiquement tolérable de celui-ci ;
à la condition que R¹ ne soit pas un pyrazolo[1,5,a]pyridinyle non substitué ou au moins monosubstitué.

2. Composé selon la revendication 1, dans lequel, dans la formule générale (I),
A est CR³ ;
B est CR⁴ ou N ;
D est CR⁵ ou N ;
E est CR⁶ ;
R' est le fluor ; le chlore ; le brome ; un C₁-C₆-alkyle non substitué ou au moins monosubstitué,
les substituants étant sélectionnés dans le groupe composé d'un halogène, de -OR⁷, -C(O)R⁷, -C (O) OR ₇ -NR⁷H, -NR⁷(C₁-C₆-alkyle) -C (O) NR⁷H, -SR⁷, aryle, hétéroaryle, hétérocyclyle, trifluorométhyle et trifluorométhoxy,
et les radicaux hétérocyclyle, aryle et hétéroaryle pouvant à leur tour être au moins monosubstitués avec un radical C₁-C₆-alkyle, C₁-C₆-alcoxy, un halogène, un radical trifluorométhyle, trifluorométhoxy ou OH ;
ou un radical hétérocyclyle, aryle ou hétéroaryle non substitué ou au moins monosubstitué,
les substituants étant sélectionnés dans le groupe composé d'un halogène, de -CH₂-R ₇ -CH₂-NH₂, -CH₂-NH(C₁-C₆-alkyle), -CH₂-N(C₁-C₆-alkyle)₂, -CH₂-OH, -CH₂-O-(C₁-C₆-alkyle), -OR⁷-C(O)R⁷, -C(O)OR⁷, -NR⁷H, -NR⁷(C₁-C₆-alkyl-), -C(O)NR⁷H, -SR⁷, aryle, hétéroaryle, trifluorométhyle et trifluorométhoxy,
les radicaux aryle et hétéroaryle pouvant à leur tour être au moins monosubstitués avec un radical C₁-C₆-alkyle, C₁-C₆-alcoxy, un halogène, un radical trifluorométhyle, trifluorométhoxy ou OH ;
R² est l'hydrogène ou un radical C₁-C₆-alkyle ;
R³, R⁴, R⁵ et R⁶ sont sélectionnés, indépendamment les uns des autres, dans le groupe composé de :
l'hydrogène, un halogène, -.CN, -CH₂-R⁸, -CH₂-NH₂, -CH₂-NH(C₁-C₆-alkyle), -CH₂-N(C₁-C₆-alkyle)₂, -CH₂-OH, -CH₂-O-(C₁-C₆-alkyle), -OR⁸, -C(O)R⁸, -C(O)OR⁸, -NR⁸H, -NR⁸(C₁-C₆-alkyle), -C(O)NR⁸H, -SR⁸, -SO₂NR⁸H, -SO₂-R⁸, aryle, hétéroaryle, hétérocyclyle, trifluorométhyle et trifluorométhoxy,
les radicaux hétérocyclyle, aryle et hétéroaryle pouvant à leur tour être au moins monosubstitués avec un radical C₁-C₆-alkyle, C₁-C₆-alcoxy, oxo, un halogène, un radical trifluorométhyle, trifluorométhoxy ou OH ;
R⁷ et R⁸ sont, indépendamment l'un de l'autre :
H ;
un radical C₁-C₆-alkyle, hétérocyclyle, phényle ou hétéroaryle non substitué ou au moins monosubstitué,
les substituants étant sélectionnés dans le groupe composé d'un radical hétéroaryle, hétérocyclyle, phényle, fluor, chlore, brome, OH, C₁-C₆-alcoxy, -C(O)OH, -C(O)O-(C₁-C₆-alkyle), -C(O)NH₂, -C(O)NH(C₁-C₆-alkyle), -C (O)N(C₁-C₁₀-alkyle)₂, trifluorométhyle, trifluorométhoxy, -NH₂, -NH(C₁-C₆-alkyle) et-N(C₁-C₆-alkyle)₂,
les radicaux hétérocyclyle, phényle et hétéroaryle pouvant à leur tour être au moins monosubstitués avec un radical C₁-C₃-alkyle, C₁-C₃-alcoxy, oxo, trifluorométhyle, trifluorométhoxy, fluor, chlore ou OH ;
hétéroaryle est l'imidazolyle, le thiophényle, le furanyle, le thiazolyle, l'imidazolyle, l'oxazolyle, l'isoxazolyle, le pyridinyle, le pyrimidinyle, le pyrazolyle, le benzo[b]thiophényle, le thiazolo[3,2-b][1,2,4]-triazolyle, le pyrrolyle, le quinoléinyle, l'isoquinoléinyle, le 1,2,3,4-tétrahydroquinoléinyle, le benzimidazolyle, l'indolyle ou le 1,3-benzodioxolyle ;
aryle est naphthyle, indanyle ou phényle ;
hétérocyclyle est le 2-oxo-azépanyle, le 1,4-oxazépanyle, le dihydropyridinonyle, le tétrahydrofuranyle, le 1,3-dioxolanyle, le morpholinyle, le pyrrolidinyle, le pipérazinyle ou le pipéridinyle ;
ou un sel physiologiquement tolérable de celui-ci.

3. Composé selon l'une des revendications 1 ou 2, dans lequel, dans la formule générale (I),
A est CR³ ;
B est CR⁴ ou N ;
D est CR⁵ ;
E est CR⁶ ;
R¹ est le chlore ;
un radical C₁-C₆-alkyle non substitué ou au moins monosubstitué,
les substituants étant sélectionnés dans le groupe composé du fluor, du chlore, de -OH, C₁-C₆-alcoxy, -NH₂, -NH(C₁-C₆-alkyle), -N(C₁-C₆-alkyle)₂, hétérocyclyl-(C₁-C₆-alkyl)-NH-, aryl-(C₁-C₆-alkyl)-NH-, hétérocyclyle, aryle, hétéroaryle,
les fragments hétérocyclyle, aryle et hétéroaryle de ces substituants pouvant à leur tour être au moins monosubstitués avec un radical C₁-C₄-alkyle, C₁-C₄-alcoxy, fluor, chlore, trifluorométhyle, trifluorométhoxy ou OH ;
ou un radical phényle, pyridinyle, pyrimidinyle, pyrazolyle, thiophényle, oxazolyle, isoxazolyle, thiazolyle, imidazolyle, morpholinyle, dihydropyridinonyle, benzo[b]thiophényle, 1,3-benzodioxolyle ou thiazolo[3,2-b][1,2,4]-triazolyle, non substitué ou au moins monosubstitué,
les substituants étant sélectionnés dans le groupe composé d'un halogène, de -CH₂-R⁷, -CH₂-NH₂, -CH₂-NH(C₁-C₆-alkyle) , -CH₂-N(C₁-C₆-alkyle)₂, -CH₂-OH, -CH₂-O-(C₁-C₆-alkyle), -OR⁷, -C(O)R⁷, -C(O)OR⁷, -NR⁷H, -NR⁷(C₁-C₆-alkyl-), -C(O)NR⁷H, -SR⁷, aryle, hétéroaryle, trifluorométhyle et trifluorométhoxy,
les radicaux aryle et hétéroaryle pouvant à leur tour être au moins monosubstitués avec un radical C₁-C₆-alkyle, C₁-C₆-alcoxy, un halogène, un radical trifluorométhyle, trifluorométhoxy ou OH ;
R² est l'hydrogène ;
R³, R⁴, R⁵, et R⁶ sont sélectionnés, indépendamment les uns des autres, dans le groupe composé de:
l'hydrogène, le fluor, le chlore, le brome, -CN, -CH₂-R⁸, -CH₂-NH₂, -CH₂-NH(C₁-C₆-alkyle), -CH₂-N(C₁-C₆-alkyle)₂, -CH₂-OH, -CH₂-O- (C₁-C₆-alkyle) , -OR⁸, -C(O)R⁸ -C (O) OR⁸ , -NR⁸H, -NR⁸(C₁-C₆-alkyle), -C (O) NR⁸H, -SR⁸, -SO₂NR⁸H, SO₂-R⁸, hétérocyclyle, trifluorométhyle et trifluorométhoxy,
le radical hétérocyclyle pouvant à son tour être au moins monosubstitué avec un radical C₁-C₆-alkyle, C₁-C₆-alcoxy, un halogène, un radical trifluorométhyle, trifluorométhoxy ou OH ;
R⁷ et R⁸v sont, indépendamment l'un de l'autre :
H ;
un radical C₁-C₆-alkyle, hétérocyclyle, phényle ou hétéroaryle non substitué ou au moins monosubstitué,
les substituants étant sélectionnés dans le groupe composé d'un radical hétéroaryle, hétérocyclyle, phényle, fluor, chlore, brome, OH, C₁-C₆-alcoxy, -C(O)OH, -C(O)O-(C₁-C₆-alkyle), -C(O)NH₂, -C(O)NH(C₁-C₆-alkyle), -C(O)N(C₁-C₁₀-alkyle)₂, trifluorométhyle, trifluorométhoxy, -NH₂, -NH(C₁-C₆-alkyle) et-N(C₁-C₆-alkyle)₂,
les radicaux hétérocyclyle, phényle et hétéroaryle pouvant à leur tour être au moins monosubstitués avec un radical C₁-C₃-alkyle, C₁-C₃-alcoxy, oxo, trifluorométhyle, trifluorométhoxy, fluor, chlore ou OH ;
hétéroaryle est l'imidazolyle, le thiophényle, le furanyle, le thiazolyle, l'imidazolyle, l'oxazolyle, l'isoxazolyle, le pyridinyle, le pyrimidinyle, le pyrazolyle, le benzo[b]thiophényle, le thiazolo[3,2-b][1,2,4]-triazolyle, le pyrrolyle, le quinoléinyle, l'isoquinoléinyle, le 1,2,3,4-tétrahydroquinoléinyle, le benzimidazolyle, l'indolyle ou le 1,3-benzodioxolyle ;
aryle est naphthyle, indanyle ou phényle ;
hétérocyclyle est le 2-oxo-azépanyle, le tétrahydrofuranyle, le 1,4-oxazépanyle, le dihydropyridinonyle, le 1,3-dioxolanyle, le morpholinyle, le pyrrolidinyle, le pipérazinyle ou le pipéridinyle ;
ou un sel physiologiquement tolérable de celui-ci.

4. Composé selon l'une des revendications 1 à 3, dans lequel, dans la formule générale (I),
A est CR³ ;
B est CR⁴ ou N ;
D est CR⁵ ;
E est CR⁶ ;
R¹ est un radical phényle, pyridinyle, pyrimidinyle, pyrazolyle, thiophényle, oxazolyle, isoxazolyle, thiazolyle, imidazolyle, morpholinyle, dihydropyridinonyle, benzo[b]thiophényle, benzodioxolyle ou thiazolo[3,2-b][1,2,4]-triazolyle, non substitué ou au moins monosubstitué,
les substituants étant sélectionnés dans le groupe composé d'un halogène, d'un radical C₁-C₆-alkyle, phényl-(C₁-C₆-alkyl)-, -OH, C₁-C₆-alcoxy, (C₁-C₆-alkyl) thio-, -O-phényle, -NH₂, -N(C₁-C₆-alkyle)₂, -NH(C₁-C₆-alkyle), HO(O)C-(C₁-C₆-alkyl)-NH-, (C₁-C₆-alkyl)-O(O)C-(C₁-C₆-alkyl)-NH-, H₂N(O)C-(C₁-C₆-alkyl)-NH-, (C₁-C₆-alkyl)HN(O)C-(C₁-C₆-alkyl)-NH-, H₂N-(C₁-C₆-alkyl)-, (C₁-C₆-alkyl)HN-(C₁-C₆-alkyl)-, (C₁-C₆-alkyl)₂N-(C₁-C₆-alkyl)-, hétérocyclyl-(C₁-C₆-alkyl)-O-, H₂N(C₁-C₆-alkyl)-NH-, (C₁-C₆-alkyl)HN-(C₁-C₆-alkyl)-NH-, (C₁-C₆-alkyl)₂N-(C₁-C₆-alkyl)-NH-, hétérocyclyl-(C₁-C₆-alkyl)-NH-, hétéroaryl-(C₁-C₆-alkyl)-NH-, phényl-(C₁-C₆-alkyl)-NH-, (C₁-C₆-alkyl)-O-(C₁-C₆-alkyl)-NH-, HO-(C₁-C₆-alkyl)-NH-, hétérocyclyl-(C₁-C₆-alkyl)-, trifluorométhyle, trifluorométhoxy, phényle et hétéroaryle,
les fragments hétérocyclyle, phényle et hétéroaryle de ces substituants pouvant à leur tour être au moins monosubstitués avec un radical C₁-C₃-alkyle, C₁-C₃-alcoxy, fluor, chlore, trifluorométhyle, trifluorométhoxy ou OH ;
R² est l'hydrogène ;
R³, R ⁴ R⁵ et R⁶ sont sélectionnés, indépendamment les uns des autres, dans le groupe composé de :
l'hydrogène, le fluor, le chlore, le brome, -CN, C₁-C₆-alkyle; -OH, C₁-C₆-alcoxy, -C(O)OH, -C(O)O-(C₁-C₆-alkyle), -NH₂, -N(C₁-C₆-alkyle)₂, -NH(C₁-C₆-alkyle), H₂N-(C₁-C₆-alkyl)-NH-, hydroxy-(C₁-C₆-alkyl)-NH-, (C₁-C₆-alkyl)HN-(C₁-C₆-alkyl)-NH-, (C₁-C₆-alkyl)₂N-(C₁-C₆-alkyl)-NH-, hétérocyclyl-(C₁-C₆-alkyl)-NH-, hétéroaryl-(C₁-C₆-alkyl)-NH-, phényl-(C₁-C₆-alkyl)-NH-, -C(O)NH₂, -C(O)N(C₁-C₆-alkyle)₂, -C(O)NH(C₁-C₆-alkyle), H₂N-(C₁-C₆-alkyl)-NHC(O)-, hydroxy-(C₁-C₆-alkyl)-NHC(O)-, (C₁-C₆-alkyl)HN-(C₁-C₆-alkyl)-NHC(O)-, (C₁-C₆-alkyl)₂N-(C₁-C₆-alkyl)-NHC(O)-, hétérocyclyl-(C₁-C₆-alkyl)-NHC(O), hétéroaryl-(C₁-C₆-alkyl)-NHC(O)-, phényl-(C₁-C₆-alkyl)-NHC(O)-, hétérocyclyle, hétérocyclyl-(C₁-C₆-alkyl)-, trifluorométhyle et trifluorométhoxy,
les fragments hétéroaryle, hétérocyclyle et phényle de ce groupe pouvant à leur tour être au moins monosubstitués avec un radical C₁-C₆-alkyle, C₁-C₆-alcoxy, un halogène, un radical trifluorométhyle, trifluorométhoxy ou OH ;
hétéroaryle est l'imidazolyle, le thiophényle, le furanyle, le thiazolyle, l'imidazolyle, l'oxazolyle, l'isoxazolyle, le pyridinyle, le pyrimidinyle, le pyrazolyle, le benzo[b]thiophényle, le thiazolo[3,2-b][1,2,4]-triazolyle, le pyrrolyle, le quinoléinyle, l'isoquinoléinyle, le 1,2,3,4-tétrahydroquinoléinyle, le benzimidazolyle, l'indolyle ou le 1,3-benzodioxolyle ;
hétérocyclyle est le 2-oxo-azépanyle, le 1,4-oxazépanyle, le dihydropyridinonyle, le tétrahydrofuranyle, le 1,3-dioxolanyle, le morpholinyle, le pyrrolidinyle, le pipérazinyle ou le pipéridinyle ;
ou un sel physiologiquement tolérable de celui-ci.

5. Composé selon l'une des revendications 1 à 4, dans lequel, dans la formule générale (I),
A est CR³ ;
B est CR⁴ ou N ;
D est CR⁵ ;
E est CR⁶;
R¹ est un radical pyridin-4-yle, 4-hydroxy-3-méthoxy-5-(méthylaminométhyl)-phényle, 2-éthylaminopyrimidin-4-yle, 3,5-diméthyl-4-hydroxyphényle, 2-(1-phényléthylamino)-pyrimidin-4-yle, 2-(2-morpholin-4-yléthylamino)-pyrimidin-4-yle, 2-méthylaminopyrimidin-4-yle, 6-méthyl-2(2-morpholin-4-yléthylamino)-pyrimidin-4-yle, 3-méthoxy-4-hydroxyphényle, 2-méthylsulfanylpyrimidin-4-yle ou 4-butylaminopyrimidin-4-yle ;
R² est l'hydrogène;
R³, R ⁴, R⁵ et R⁶ sont sélectionnés, indépendamment les uns des autres, dans le groupe composé de :
l'hydrogène, le fluor, le chlore, le brome, -CN, -C(O)O-méthyle, (2-diéthylaminoéthyl)-NHC(O)-, COOH, méthoxy, éthoxy, (2-cyclohexylaminoéthyl)-NHC(O)-, (3-(4-méthylpipérazin-1-yl)-propyl)-NHC(O)-, (3-hydroxypropyl)-NHC(O)-, (3-cyclohexylaminopropyl) -NHC(O)-, (3-imidazol-1-yl-propyl)-NHC(O)-, méthyle, éthyle, 4-méthylpipérazin-1-yle, trifluorométhyle et trifluorométhoxy ;
ou un sel physiologiquement tolérable de celui-ci.

6. Composé selon l'une des revendications 1 à 5 sélectionné dans le groupe composé de :
4-(1H-benzimidazol-2-yl)-6-(4-hydroxy-3-méthoxy-5-méthylaminométhylphényl)-2H-pyridazin-3-one,
(2-diéthylaminoéthyl)-amide d'acide 2-(3-oxo-6-pyridin-4-yl-2,3-dihydropyridazin-4-yl)-1H-benzimidazole-5-carboxylique,
4-(6-trifluorométhyl-1H-benzimidazol-2-yl)-6-pyridin-4-yl-2H-pyridazin-3-one,
4-(6-méthoxy- 1H-benzimidazol-2,-yl)-6-pyridin-4-yl-2H-pyridazin-3-one,
4-(5-chloro-1H-benzimidazol-2-yl)-6-méthyl-2H-pyridazin-3-one,
4-(6-chloro-1H-benzimidazol-2-yl)-6-(4-hydroxy-3,5-diméthylphényl)-2H-pyridazin-3-one,
4-(5-fluoro-1H-benzimidazol-2-yl)-6-pyridin-4-yl-2H-pyridazin-3-one,
6-(4-hydroxy-3-méthoxyphényl)-4-(6-trifluorométhyl-1H-benzimidazol-2-yl)-2H-pyridazin-3-one,
6-(2-butylaminopyrimidin-4-yl)-4-(6-chloro-1H-benzimidazol-2-yl)-2H-pyridazin-3-one,
4-(1H-benzimidazol-2-yl)-6-pyridin-4-yl-2H-pyridazin-3-one,
6-[2-(2-morpholino-4-yl-éthylamino)-pyrimidin-4-yl] -4-(6-trifluorométhyl-1H-benzimidazol-2-yl)-2H-pyridazin-3-one et
4-(3H-imidazol[4,5-c]pyridin-2-yl)-6-pyridin-4-yl-2H-pyridazin-3-one ;
ou un sel physiologiquement tolérable de celui-ci.

7. Composé selon l'une des revendications 1 à 6 ainsi que leurs sels physiologiquement tolérables à utiliser comme médicament.

8. Utilisation d'un composé selon l'une des revendications 1 à 6 ou d'un sel physiologiquement tolérable de celui-ci pour la préparation d'un médicament qui est un inhibiteur de GSK-3β.

9. Utilisation d'un composé selon l'une des revendications 1 à 6 ou d'un sel physiologiquement tolérable de celui-ci pour la préparation d'un médicament en vue de la prophylaxie et/ou du traitement de maladies neurodégénératives, d'attaques d'apoplexie, de lésions du crâne et de la moelle osseuse et de neuropathies périphériques, de l'obésité, de maladies métaboliques, du diabète de type II, de l'hypertonie essentielle, de maladies cardiovasculaires athérosclérotiques, du syndrome de Stein-Leventhal, du syndrome X, d'immunodéficiences ou du cancer.

10. Utilisation selon la revendication 9, **caractérisée en ce que** les maladies neurodégénératives sont la maladie d'Alzheimer, la maladie de Parkinson, la démence frontopariétale, la dégénération corticobasale ou la maladie de Pick.

11. Utilisation selon la revendication 9 pour la prophylaxie et/ou le traitement du diabète de type II ou de la maladie d'Alzheimer.

12. Composition pharmaceutique comprenant une quantité active d'au moins un composé ou d'un sel physiologiquement tolérable de celui-ci conformément à l'une des revendications 1 à 6 et un excipient physiologiquement tolérable.

13. Composition pharmaceutique selon la revendication 12, la composition pharmaceutique étant présente sous la forme d'une pilule, d'un comprimé, d'un comprimé enrobé, d'une pastille à sucer, d'un granulé, d'une gélule, d'une gélule de gélatine dure ou molle, d'une solution aqueuse, d'une solution alcoolique, d'une solution huileuse, d'un sirop, d'une émulsion, d'une suspension, d'un suppositoire, d'une pastille, d'une solution pour injection ou perfusion, d'une pommade, d'une teinture, d'une crème, d'une lotion, d'une poudre, d'un spray, d'un système thérapeutique transdermique, d'un spray nasal, d'un aérosol, d'un mélange en aérosol, d'une microgélule, d'un implant, d'un bâtonnet ou d'un patch.

14. Procédé de préparation d'un composé selon l'une des revendications 1 à 6, **caractérisé en ce qu'**un composé de formule générale (II) 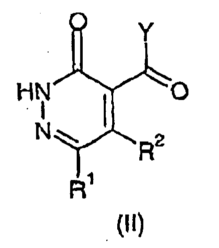 est mis en réaction avec un composé de formule générale (III) 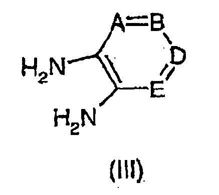
a) Y étant un groupe sortant et la cyclisation étant effectuée en utilisant un acide ou un agent déshydratant ou
b) Y étant H et la cyclisation étant effectuée par oxydation, en particulier par oxygène,
et, éventuellement, les substituants A, B, D, E, R¹ ou R² sont modifiés à la suite de la cyclisation.

15. Procédé de préparation d'un composé selon l'une des revendications 1 à 6, **caractérisé en ce qu'**un composé de formule générale (VI) est mis en réaction avec un composé de formule générale (V) en présence d'un complexe de palladium en un composé de formule (Ia), éventuellement avec séparation consécutive du groupe protecteur Y2, 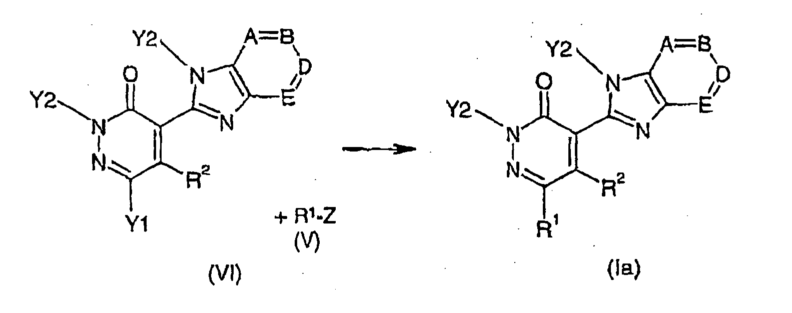
Y1 étant un halogène, B(OH)₂ ou Sn(C₁-C₁₀-alkyle) et Y2 étant H ou un groupe protecteur
et Z étant B(OH)₂, B(C₁-C₁₀-alcoxy)₂, Sn(C₁-C₁₀-alkyle)₃, Zn-(C₁-C₁₀-alkyle) ou un halogène et R¹ étant un groupe aryle ou hétéroaryle non substitué ou au moins monosubstitué.
